# EUROPEAN PATENT SPECIFICATION

(11) **EP 4 168 578 B1**
(45) Date of publication and mention of the grant of the patent: **07.08.2024**
(21) Application number: 21740355.9
(22) Date of filing: 17.06.2021
(51) Int. Cl.: C12Q 1/6841, C12Q 1/6806

(54) **COMPOSITIONS AND METHODS FOR IN SITU SINGLE CELL ANALYSIS USING ENZYMATIC NUCLEIC ACID EXTENSION**
ZUSAMMENSETZUNGEN UND VERFAHREN ZUR IN-SITU-EINZELZELLENANALYSE UNTER VERWENDUNG VON ENZYMATISCHER NUKLEINSÄUREVERLÄNGERUNG
COMPOSITIONS ET PROCÉDÉS POUR UNE ANALYSE DE CELLULE UNIQUE IN SITU À L'AIDE D'UNE EXTENSION D'ACIDE NUCLÉIQUE ENZYMATIQUE

(30) Priority: 18.06.2020 US 202063040651 P
(43) Date of publication of application: 26.04.2023
(73) Proprietor: Bruker Spatial Biology, Inc., Billerica, MA 01821 (US)
(72) Inventor: CHENG, Jonathan, Dublin, California 94568 (US); DUNAWAY, Dwayne, Seattle, Washington 98115 (US); GREGORY, Mark, Boise, Idaho 83709 (US); JUNG, Jaemyeong, Bellevue, Washington 98008 (US); KHAFIZOV, Rustem, Seattle, Washington 98125 (US); KIM, Dae, Bellevue, Washington 98006 (US); PERILLO, Evan, Woodinville, Washington 98072 (US); RANE, Tushar, Seattle, Washington 98119 (US); WU, Lidan, Seattle, Washington 98105 (US)
(74) Representative: Cooley (UK) LLP
(86) International application number: PCT/US2021/037772
(87) International publication number: WO 2021/257795

(56) References cited:
- WO-A1-2019/224544
- MERRITT CHRISTOPHER R ET AL: "Multiplex digital spatial profiling of proteins and RNA in fixed tissue", NATURE BIOTECHNOLOGY, GALE GROUP INC, NEW YORK, vol. 38, no. 5, 1 May 2020 (2020-05-01), pages 586 - 599, XP037113520, ISSN: 1087-0156, [retrieved on 20200511], DOI: 10.1038/S41587-020-0472-9

## Description

### BACKGROUND OF THE INVENTION

Standard immunohistochemical and *in situ* hybridization methods allow for simultaneous detection of, at most, six to ten protein or nucleic acid targets, with three to four targets being typical. There exists a need for probes, compositions, methods, and kits for simultaneous, multiplexed detection and quantification of protein and/or nucleic acid expression in a user-defined region of a tissue, user-defined cell, and/or user-defined subcellular structure within a cell at a high enough resolution and with minimal background noise to do so. Furthermore, there is a need for such systems to be adaptable for use with existing sequencing technologies that are already available to a large number of end users. Merritt Christopher et al. Nature Biotechnology, 38:5, pp. 586-599 (2020) discusses multiplex spatial profiling of proteins or RNA. WO 2019/224544 describes methods for synthesizing polynucleotide molecules according to a predefined nucleotide sequence.

### SUMMARY OF THE INVENTION

The invention is defined by the appended claim set. Any other configurations or instances as set forth herein not falling within the scope of the claims are for information only.

In a related instance provided for illustrative purposes the present disclosure provides a method for *in situ* synthesis of a nucleic acid sequence in a tissue sample, the method comprising: a) contacting the tissue sample with at least one probe, wherein the probe comprises a target-binding domain and a target identification domain, wherein the probe comprises a free 3'-OH moiety, and wherein the target-binding domain binds to at least one target molecule at a first location of the tissue sample; b) ligating a nucleotide to the free 3'-OH moiety of at least one bound probe by contacting the free 3'-OH moiety with at least one nucleotide-polymerase complex, thereby extending at least one bound probe, wherein the at least one nucleotide-polymerase complex comprises the nucleotide operably linked to a polymerase via a photocleavable linker, c) illuminating the first location of the tissue sample with light sufficient to cleave the photocleavable linker of the at least one nucleotide-polymerase complex, thereby releasing the polymerase and exposing a free 3'-OH moiety on the extended at least one bound probe; and d) repeating steps (b) and (c) until the nucleic acid sequence has been synthesized.

In a related instance provided for illustrative purposes the present disclosure provide a method for *in situ* synthesis of a nucleic acid sequence in a tissue sample, the method comprising: a) contacting the tissue sample with at least one probe, wherein the probe comprises a target-binding domain and a target identification domain, wherein the probe comprises a free 3'-OH moiety, and wherein the target-binding domain binds to at least one target molecule located at a first location of the tissue sample; b) ligating a nucleotide to the free 3'-OH moiety of at least one bound probe by contacting the tissue sample with at least one reversible terminator nucleotide and at least one polymerase, thereby extending the at least one bound probe, wherein the at least one reversible terminator nucleotide comprises the nucleotide operably linked to a 3' terminator moiety via a photocleavable linker; c) illuminating the first location of the tissue sample with light sufficient to cleave the photocleavable linker of the at least one reversible terminator nucleotide, thereby releasing the 3' terminator moiety and exposing a free 3'-OH moiety on the extended at least one bound probe; and d) repeating steps (b) and (c) until the nucleic acid sequence has been synthesized.

In some aspects of the preceding methods, a target-binding domain binds to at least one target molecule at an at least second location of the tissue sample, and wherein the method further comprises repeating steps (b) - (d) at the at least second location.

In some aspects of the preceding methods, a nucleic acid sequence synthesized at the first location of the tissue sample is different than the nucleic acid sequence synthesized at the at least second location of the tissue sample.

The present invention provides a method of producing a spatially-resolved profile of the abundance of at least two target analytes in a first and an at least second location of a tissue sample comprising: a) contacting the tissue sample with a solution comprising at least two species of probes, the probes comprising a target-binding domain and a target identification domain, wherein each species of probe comprises a unique target-binding domain that binds to one of the at least two target analytes and a unique target identification domain specific for the target analyte, and a free 3'-OH moiety; b) ligating a nucleotide to the free 3'-OH moiety of at least one bound probe by contacting the tissue sample with a first plurality of nucleotide-polymerase complexes, thereby extending the barcode domain of the at least one bound probe and forming a spatial barcode domain, wherein at least one nucleotide-polymerase complex in the first plurality comprises the nucleotide operably linked to a polymerase via a photocleavable linker; c) illuminating at least one location of the tissue sample with light sufficient to cleave the photocleavable linker of the nucleotide-polymerase complexes, thereby releasing the polymerases and exposing a free 3'-OH moiety on the spatial barcode domain at the at least one location of the tissue sample; d) ligating a nucleotide to the free 3'-OH moiety of the spatial barcode domain of at least one bound probe at the at least one location illuminated in step (c) by contacting the tissue sample with an additional plurality of nucleotide-polymerase complexes, thereby extending the spatial barcode domain of the at least one bound probe, wherein at least one nucleotide-polymerase complex in the additional plurality comprises the nucleotide operably linked to a polymerase via a photocleavable linker; e) repeating steps (c) and (d) until the spatial barcode domain of at least one probe bound to a target analyte in the first location of the tissue sample has been extended such that the spatial barcode domain comprises a unique nucleic acid sequence specific to the first location of the tissue sample, and the spatial barcode domain of at least one probe bound to a target analyte in the at least second location of the tissue sample has been extended such that the spatial barcode domain comprises a unique nucleic acid sequence specific to the at least second location of the tissue sample; f) collecting the probes bound to target analytes in the tissue sample; and g) quantifying via sequencing the probes collected in step (f), thereby determining the abundance of the at least two target analytes in the first and the at least second location of the tissue sample, thereby producing a spatially-resolved profile of the abundance of the at least two target analytes.

The present invention provides a method of producing a spatially-resolved profile of the abundance of at least two target analytes in a first and an at least second location of a tissue sample comprising: a) contacting the tissue sample with a solution comprising at least two species of probes, the probes comprising a target-binding domain and a target identification domain, wherein each species of probe comprises a unique target-binding domain that binds to one of the at least two target analytes and a unique target identification domain specific for the target analyte, and a free 3'-OH moiety; b) ligating a nucleotide to the free 3'-OH moiety of the barcode domain of at least one bound probe by contacting the tissue sample with a first plurality of reversible terminator nucleotides and a first plurality of polymerases, thereby extending the at least one bound probe and forming a spatial barcode domain, wherein at least one reversible terminator nucleotide in the first plurality comprises the nucleotide operably linked to a 3' terminator moiety via a photocleavable linker; c) illuminating at least one location of the tissue sample with light sufficient to cleave the photocleavable linker of the reversible terminator nucleotides, thereby releasing the reversible 3' terminator moieties and exposing a free 3'-OH moiety on the spatial barcode domain at the at least one location of the tissue sample; d) ligating a nucleotide to the free 3'-OH moiety of the spatial barcode domain of at least one bound probe at the at least one location illuminated in step (c) by contacting the tissue sample with an additional plurality of reversible terminator nucleotides and an additional plurality of polymerases, thereby extending the spatial barcode domain of the at least one bound probe, wherein at least one reversible terminator nucleotide in the additional plurality comprises the nucleotide operably linked to a reversible 3' terminator moiety via a photocleavable linker; e) repeating steps (c) and (d) until the spatial barcode domain of at least one probe bound to a target analyte in the first location of the tissue sample has been extended such that the spatial barcode domain comprises a unique nucleic acid sequence specific to the first location of the tissue sample, the spatial barcode domain of at least one probe bound to a target analyte in the at least second location of the tissue sample has been extended such that the spatial barcode domain comprises a unique nucleic acid sequence specific to the at least second location of the tissue sample; f) collecting the probes bound to target analytes in the tissue sample; and g) quantifying via sequencing the probes collected in step (f), thereby determining the abundance of the at least two target analytes in the first and the at least second location of the tissue sample, thereby producing a spatially-resolved profile of the abundance of the at least two target analytes.

In some aspects of the invention, the preceding methods can further comprise comparing the abundance of the at least two target analytes in the first location of the tissue sample and the at least two target analytes in the at least second location of the tissue sample.

A polymerase can be a terminal deoxynucleotidyl transferase or a biologically-active portion thereof.

An at least one reversible terminator nucleotide can comprise 3'-O-(2-nitrobenzyl)-dATP, 3'-O-(2-nitrobenzyl)-dCTP, 3'-O-(2-nitrobenzyl)-dGTP or 3'-O-(2-nitrobenzyl)-dTTP.

A 3' terminator moiety can comprise 2-nitrobenzyl.

A probe can further comprise a unique molecular identifier. A unique molecular identifier can be at least about 14 nucleotides in length.

A probe can further comprise an amplification primer binding site. An amplification primer binding site can be at least about 24 nucleotides in length.

A probe can further comprise a constant region. A constant region can be at least about 12 to at least about 20 nucleotides in length.

A probe can comprise, from 5' to 3', a target binding domain, followed by a amplification primer binding site, followed by a unique molecular identifier, followed by a target identification domain, followed by a constant region.

In some aspects of the preceding methods, a spatial barcode domain of at least one probe bound to a target analyte in the first location of the tissue sample comprises a unique spatial identifier sequence specific to the first location of the tissue sample. In some aspects of the preceding methods, a spatial barcode domain of at least one probe bound to a target analyte in the at least second location of the tissue sample comprises a unique spatial identifier sequence specific to the at least second location of the tissue sample.

A spatial identifier sequence can comprise at least about 20 nucleotides.

A spatial identifier sequence can comprise at least four spatial identification domains. Each of the at least four spatial identification domains can comprise the same number of nucleotides. At least one of the at least four spatial identifications domains can comprise a different number of nucleotides as compared to another spatial identification domain within the same spatial barcode. Each spatial identification domain can comprise about 1 to about 4 nucleotides. Each spatial identification domain can comprise about 4 nucleotides. Each of the at least four spatial identification domains can comprise the same nucleotide at the 3' terminus

In some aspects of the invention , the method can further comprise, after step (e) and prior to step (f), repeating steps (c) and (d) to extend the spatial barcode domain in each location of the tissue sample such that the spatial barcode domain comprises, at the 3' end, a delimiting domain.

In some aspects of the invention, the method can further comprise, after step (e) and prior to step (f), extending the spatial barcode domain in each location of the tissue sample such that the spatial barcode domain comprises a polyT domain.

In some aspects of the invention, the method can further comprise, after step (e) and prior to step (f): (i) repeating steps (c) and (d) to extend the spatial barcode domain in each location of the tissue sample such that the spatial barcode domain comprises, a delimiting domain; and (ii) extending the spatial barcode domain in each location of the tissue sample such that the spatial barcode domain comprises a polyT domain.

A delimiting domain can be at least about 4 to at least about 6 nucleotides in length. The sequence of the delimiting domain is the same for every spatial barcode in the sample.

A polyT domain can comprise at least about 14 nucleotides.

In some aspects of the invention, the illumination in step (c) can provided by a light source selected from the group consisting of an arc-lamp, a laser, a focused UV light source, and light emitting diode. A laser can be an infrared laser.

In some aspects of the invention, the illumination in step (c) can be provided by a two-photon excitation microscope.

In some aspects of the invention, the first location of the tissue sample and the second location of the tissue sample can be no more than about 500 nm in the x and/or y direction and no more than about 1500 nm in the z direction.

In some aspects of the invention, the first location of the tissue sample and the at least second location of the tissue sample can be subcellular.

In some aspects of the invention, the first location of the tissue sample and the at least second location of the tissue sample each can comprise no more than one cell.

In some aspects of the invention, the first location of the tissue sample and the at least second location of the tissue sample each can comprise no more than ten cells.

In some aspects of the invention, each cell within the first location of the tissue sample and the at least second location of the tissue sample can be individually automatically identified and encoded.

In some aspects of the invention, the method can further comprise prior to step (a), subjecting the tissue sample to ddTTP (dideoxthymidine-triphosphate) termination. Subjecting the tissue sample to ddTTP termination can comprise contacting the tissue sample with ddTTP and TdT.

In some aspects of the invention, the method can further comprise after step (f) and prior to step (g), amplifying the collected probes. Amplifying the collected probes can comprise the use of a first amplification primer and a second amplification primer, wherein the first amplification primer comprises a first flow cell adapter sequence, a first NGS index sequence and a first sequencing primer binding site, and the second amplification primer comprises a second flow cell adapter sequence, a second NGS index sequence and second sequencing primer binding site. At least one of the first and the second amplification primers can comprise a nucleic acid sequence that is complementary to the delimiting sequence and/or the polyT domain.

Any of the above aspects can be combined with any other aspect.

Unless otherwise defined, all technical and scientific terms used herein have the same meaning as commonly understood by one of ordinary skill in the art to which this disclosure belongs. In the Specification, the singular forms also include the plural unless the context clearly dictates otherwise; as examples, the terms "a," "an," and "the" are understood to be singular or plural and the term "or" is understood to be inclusive. By way of example, "an element" means one or more element. Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

### BRIEF DESCRIPTION OF THE DRAWINGS

The above and further features will be more clearly appreciated from the following detailed description when taken in conjunction with the accompanying drawings.
FIG. 1 is an exemplary schematic of an *in situ* nucleic acid synthesis method of the present disclosure (provided for illustrative purposes).
FIG. 2 is an exemplary schematic of an *in situ* nucleic acid synthesis method of the present disclosure (provided for illustrative purposes).
FIG. 3A-3N is an exemplary schematic of the steps of a method of producing a spatially-resolved profile of the abundance of at least one target analytes in at least two location of a tissue sample.
FIG. 4A-4N is an exemplary schematic of the steps of a method of producing a spatially-resolved profile of the abundance of at least one target analytes in at least two location of a tissue sample.
FIG. 5A is an exemplary optical schematic of a two-photon excitation method that can be used in the methods of the present disclosure.
FIG. 5B is an exemplary optical schematic of a two-photon excitation method that can be used in the methods of the present disclosure.
FIG. 6 shows the structures of various proparglyamino dNTPs.
FIG. 7 shows the structure of BP-23354.
FIG. 8 is a schematic of an exemplary nucleotide-polymerase complex preparation method.
FIG. 9 shows the structures of various reversible terminator nucleotides for use in the methods of the present disclosure.
FIG. 10 is an exemplary schematic of a probe of the present disclosure.
FIG. 11 is an exemplary schematic of the probe of FIG. 10 that has been extended using TdT extension in the methods of the present disclosure to form a spatial barcode domain, wherein the spatial barcode domain comprises four spatial identification (ID) domains.
FIG. 12 is an exemplary schematic of the probe of FIG. 10 that has been extended using TdT extension in the methods of the present disclosure to form a spatial barcode domain, wherein the spatial barcode domain comprises four spatial identification (ID) domains and a delimiting domain.
FIG. 13 is exemplary schematic of the probe of FIG. 10 that has been extended using TdT extension in the methods of the present disclosure to form a spatial barcode domain, wherein the spatial barcode domain comprises four spatial identification (ID) domains, a delimiting domain and a polyT domain.
FIG. 14A is an exemplary schematic of an extended probe of the present disclosure, and two amplification primers of the present disclosure.
FIG. 14B is an exemplary schematic of an extended probe of the present disclosure and two amplification primers of the present disclosure. The second amplification primer comprises two degenerate bases ("BN") at the 3' end.
FIG. 15 is an exemplary schematic of a primer design for use in an amplification experiment to test for the presence and amplification of the probes of the present disclosure.
FIG. 16 is an image of gel-electrophoresis analysis of the amplification of probes of the present disclosure using amplification primers of the present disclosure.
FIG. 17 is an exemplary schematic of a primer design for use in an amplification experiment to test for the presence and amplification of the probes of the present disclosure.
FIG. 18 is an image of gel-electrophoresis analysis of the amplification of probes of the present disclosure using amplification primers of the present disclosure.
FIG. 19 is a chart showing the recovery of DNA, including probes, from tissue samples as part of the methods of the present disclosure.
FIG. 20 is an exemplary schematic of a probe of the present disclosure.
FIG. 21 is exemplary schematic of the probe of FIG. 20 that has been extended using TdT extension in the methods of the present disclosure to form a spatial barcode domain, wherein the spatial barcode domain comprises four identification (ID) domains, a delimiting domain and a polyT domain.
FIG. 22 is a series of images showing the improved photocleaving results obtained using two-photon illumination in the methods of the present disclosure.
FIG. 23 is an exemplary schematic of a method of purifying the probes collected from a tissue sample as part of quantifying the probes via sequencing.

### DETAILED DESCRIPTION OF THE INVENTION

The invention is defined by the appended claims.

The present disclosure is based in part on probes, compositions, methods, and kits for simultaneous, multiplexed spatial detection and quantification of protein and/or nucleic acid expression in a user-defined region of a tissue, user-defined cell, and/or user-defined subcellular structure within a cell using existing sequencing methods.

The present disclosure provides a comparison of the identity and abundance of target proteins and/or target nucleic acids present in a first region of interest (*e.g*., tissue type, a cell (including normal and abnormal cells), and a subcellular structure within a cell) and the identity and abundance of target proteins and/or target nucleic acids present in a second region of interest. There is no pre-defined upper limit to the number of regions of interest and comparisons that can be made; the upper limit relates to the size of the region of interest relative the size of the sample. As examples, when a single cell represents a region of interest, then a section may have hundreds to thousands of regions of interest; however, if a tissue section includes only two cell types, then the section may have only two regions of interest (each including only one cell type).

The present disclosure provides a higher degree of multiplexing than is possible with standard immunohistochemical or *in situ* hybridization methods. Standard immunohistochemical methods allow for maximal simultaneous detection of six to ten protein targets, with three to four protein targets being more typical. Similarly, *in situ* hybridization methods are limited to simultaneous detection of fewer than ten nucleic acid targets. The present disclosure provides detection of large combinations of nucleic acid targets and/or protein targets from a defined region of a sample. The present disclosure provides an increase in objective measurements by digital quantification and increased reliability and consistency, thereby enabling comparison of results among multiple centers.

As opposed to other methods known in the art, the methods of the invention do not require sequential fluidic steps to extract probes/barcodes from specific regions of a tissue sample.

Moreover, as opposed to other methods known in the art that rely on digital micromirror-directed illumination systems, the methods of the invention are compatible with two-photon illumination systems, including point scanning two-photon systems. The two-photon illumination systems exhibit increased spatial resolution, allowing the illumination of a single region of interest (ROI) that is as small as a single cell or even a subcellular structure within a single cell.

Various compositions (not claimed), methods of the invention, and related methods provided for illustrative purposes are described in full detail herein.

### Methods

In some aspects, the methods described herein can be referred to as a "DNA writing microscope," that can use a combination of two-photon microscopy and photo-activatable chemistry to create single-cell spatial barcodes *in situ,* thereby allowing for the quantification of gene-expression at single-cell resolution in a spatially resolved manner.

In an instance provided for illustrative purposes and which is not claimed, the present disclosure provides a method for *in situ* synthesis of a nucleic acid sequence in a tissue sample, the method comprising: a) contacting the tissue sample with at least one probe, wherein the probe comprises a target-binding domain and a target identification domain, wherein the probe comprises a free 3'-OH moiety, and wherein the target-binding domain binds to at least one target molecule at a first location of the tissue sample; b) ligating a nucleotide to the free 3'-OH moiety of at least one bound probe by contacting the free 3'-OH moiety with at least one nucleotide-polymerase complex, thereby extending the at least one bound probe, wherein the at least one nucleotide-polymerase complex comprises the nucleotide operably linked to a polymerase via a photocleavable linker, c) illuminating the first location of the tissue sample with light sufficient to cleave the photocleavable linker of the at least one nucleotide-polymerase complex, thereby releasing the polymerase and exposing a free 3'-OH moiety on the extended at least one bound probe; and d) repeating steps (b) and (c) until the nucleic acid sequence has been synthesized.

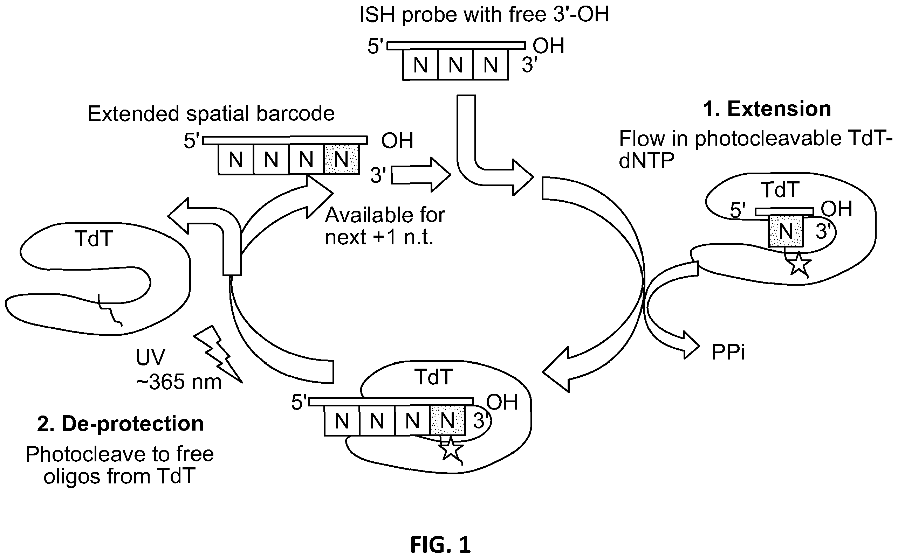

An exemplary schematic of the preceding method is shown in FIG. 1. In FIG. 1, a probe with a free 3'-OH is provided (top, "ISH probe with free 3'-OH"). This probe is then extended by ligating a nucleotide to the free 3'-OH moiety by contacting the free 3'-OH moiety with a nucleotide polymerase complex comprising a nucleotide operably linked to a polymerase via a photocleavable linker. In this non-limiting example, the polymerase is terminal deoxynucleotidyl transferase (TdT). The nucleotide (boxed "N") is operably linked to the TdT molecule via a photocleavable linker (represented by the star shape in FIG. 1). In the next step, de-protection, the photocleavable linker is cleaved by illuminating the probe and nucleotide-polymerase complex with light of a wavelength sufficient to cleave the photocleavable linker (in this non-limiting example, the light is UV light with a wavelength of about 365 nm). This releases the polymerase from the extended probe, exposing a free 3'-OH moiety on the extended probe. The process can then be repeated until the desired nucleic acid sequence has been synthesized.

In an instance provided for illustrative purposes and which is not claimed, the present disclosure provides a method for *in situ* synthesis of a nucleic acid sequence in a tissue sample, the method comprising: a) contacting the tissue sample with at least one probe, wherein the probe comprises a target-binding domain and a target identification domain, wherein the probe comprises a free 3'-OH moiety, and wherein the target-binding domain binds to at least one target molecule located at a first location of the tissue sample; b) ligating a nucleotide to the free 3'-OH moiety of at least one bound probe by contacting the tissue sample with at least one reversible terminator nucleotide and at least one polymerase, thereby extending the at least one bound probe, wherein the at least one reversible terminator nucleotide comprises the nucleotide operably linked to a 3' terminator moiety via a photocleavable linker; c) illuminating the first location of the tissue sample with light sufficient to cleave the photocleavable linker of the at least one reversible terminator nucleotide, thereby releasing the 3' terminator moiety and exposing a free 3'-OH moiety on the extended at least one bound probe; and d) repeating steps (b) and (c) until the nucleic acid sequence has been synthesized.

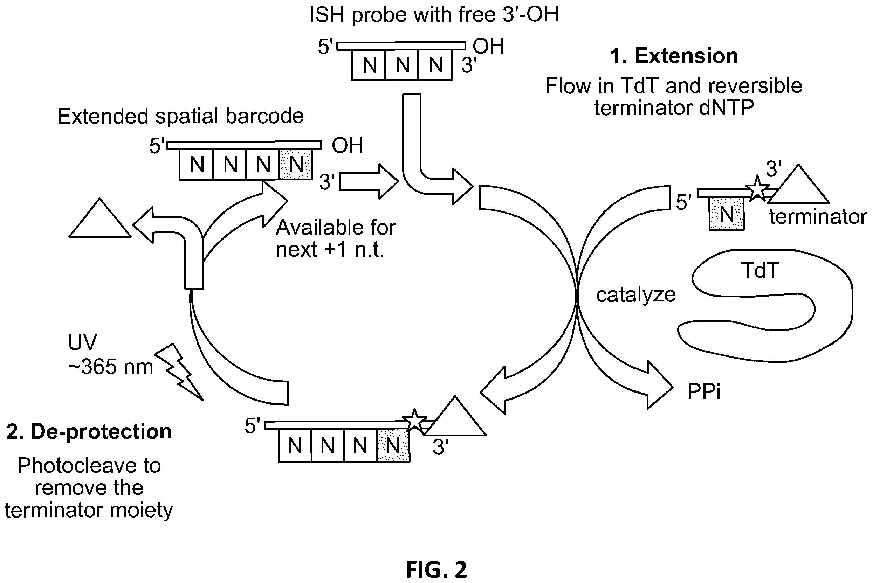

A schematic of the preceding method is shown in FIG. 2. In FIG. 2, a probe with a free 3'-OH moiety is provided (top, "ISH probe with free 3'-OH). This probe is then extended by ligating a nucleotide to the free 3'-OH moiety by contacting the probe with at least one reversible terminator nucleotide and at least one polymerase. In this non-limiting example, the polymerase is terminal deoxynucleotidyl transferase (TdT). The nucleotide (boxed "N") is operably linked to a terminator moiety (triangle) by a photocleavable linker (represented by the star shape in FIG. 2). In the next step, de-protection, the photocleavable linker is cleaved by illuminating the probe with light of a wavelength sufficient to cleave the photocleavable linker (in this non-limiting example, the light is UV light with a wavelength of about 365 nm). This releases the terminator moiety (triangle), thereby exposing a free 3'-OH moiety on the extended probe. The process can then be repeated until the desired nucleic acid sequence has been synthesized.

The present invention provides a method of producing a spatially-resolved profile of the abundance of at least two target analytes in a first and an at least second location of a tissue sample comprising: a) contacting the tissue sample with a solution comprising at least two species of probes, the probes comprising a target-binding domain and a target identification domain, wherein each species of probe comprises a unique target-binding domain that binds to one of the at least two target analytes and a unique target identification domain specific for the target analyte, and a free 3'-OH moiety; b) ligating a nucleotide to the free 3'-OH moiety of at least one bound probe by contacting the tissue sample with a first plurality of nucleotide-polymerase complexes, thereby extending the barcode domain of the at least one bound probe and forming a spatial barcode domain, wherein at least one nucleotide-polymerase complex in the first plurality comprises the nucleotide operably linked to a polymerase via a photocleavable linker; c) illuminating at least one location of the tissue sample with light sufficient to cleave the photocleavable linker of the nucleotide-polymerase complexes, thereby releasing the polymerases and exposing a free 3'-OH moiety on the spatial barcode domain at the at least one location of the tissue sample; d) ligating a nucleotide to the free 3'-OH moiety of the spatial barcode domain of at least one bound probe at the at least one location illuminated in step (c) by contacting the tissue sample with an additional plurality of nucleotide-polymerase complexes, thereby extending the spatial barcode domain of the at least one bound probe, wherein at least one nucleotide-polymerase complex in the additional plurality comprises the nucleotide operably linked to a polymerase via a photocleavable linker; e) repeating steps (c) and (d) until the spatial barcode domain of at least one probe bound to a target analyte in the first location of the tissue sample has been extended such that the spatial barcode domain comprises a unique nucleic acid sequence specific to the first location of the tissue sample, and the spatial barcode domain of at least one probe bound to a target analyte in the at least second location of the tissue sample has been extended such that the spatial barcode domain comprises a unique nucleic acid sequence specific to the at least second location of the tissue sample; f) collecting the probes bound to target analytes in the tissue sample; and g) quantifying via sequencing the probes collected in step (f), thereby determining the abundance of the at least two target analytes in the first and the at least second location of the tissue sample, thereby producing a spatially-resolved profile of the abundance of the at least two target analytes.

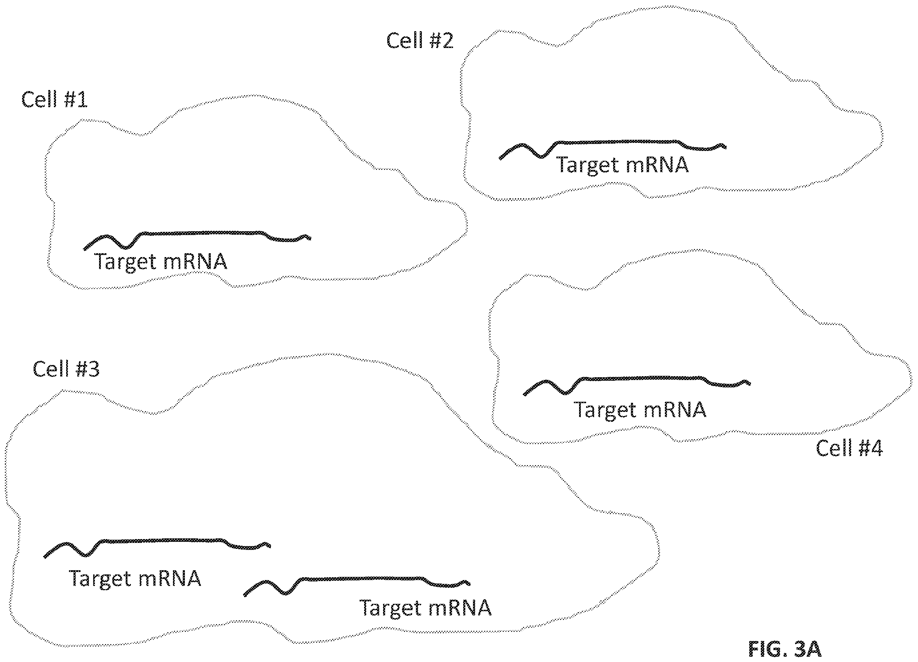

A schematic of a non-limiting example of the preceding method is shown in FIG. 3A-3N. The method begins in FIG. 3A with a sample. In this non-limiting example, the sample comprises four cells, each cell comprising at least one copy of the specific target mRNA to be measured. Cell #1, Cell #2 and Cell #3 comprise one copy of the target mRNA and Cell #3 comprises two copies of the target mRNA.

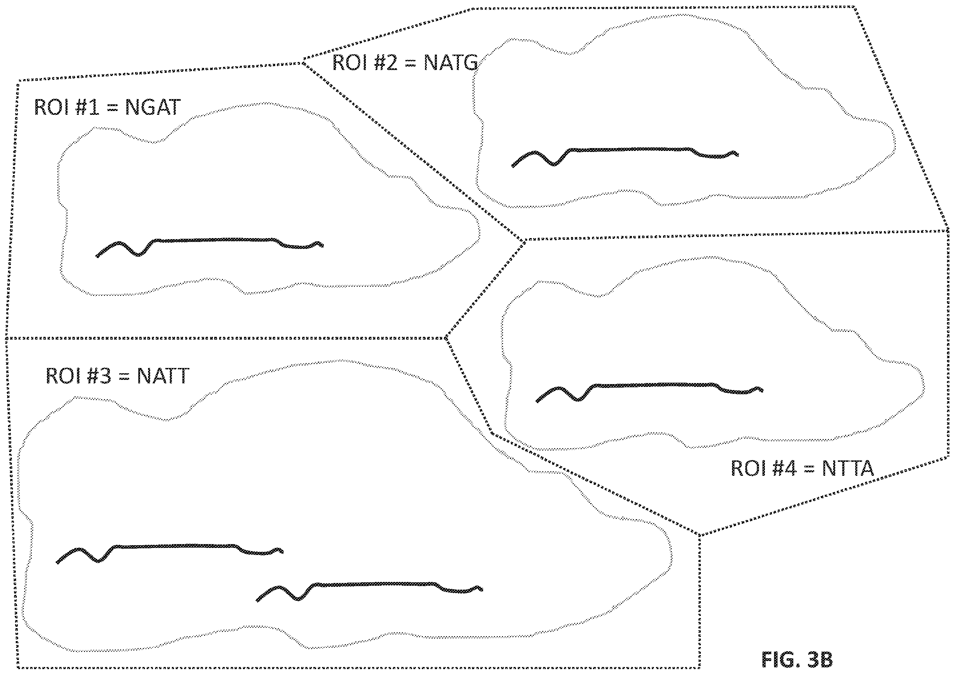

The sample can be subdivided into regions of interest (ROI) as shown in FIG. 3B. In this non-limiting example, each ROI comprises one of the cells. Moreover, each ROI is pre-assigned a specific spatial barcode that is unique to that ROI. In this example, each spatial barcode comprises 4 nucleotides, wherein the first nucleotide is any nucleotide (as represented by "N"). The spatial barcode for ROI #1 is NGAT, the spatial barcode for ROI #2 NATG, the spatial barcode for ROI #3 is NATT and the spatial barcode for ROI #4 is NTTA. As described further herein, the spatial barcode can comprise any number of nucleotides, and the four nucleotide spatial barcodes depicted in FIGs 3A-3N are for exemplary purposes only.

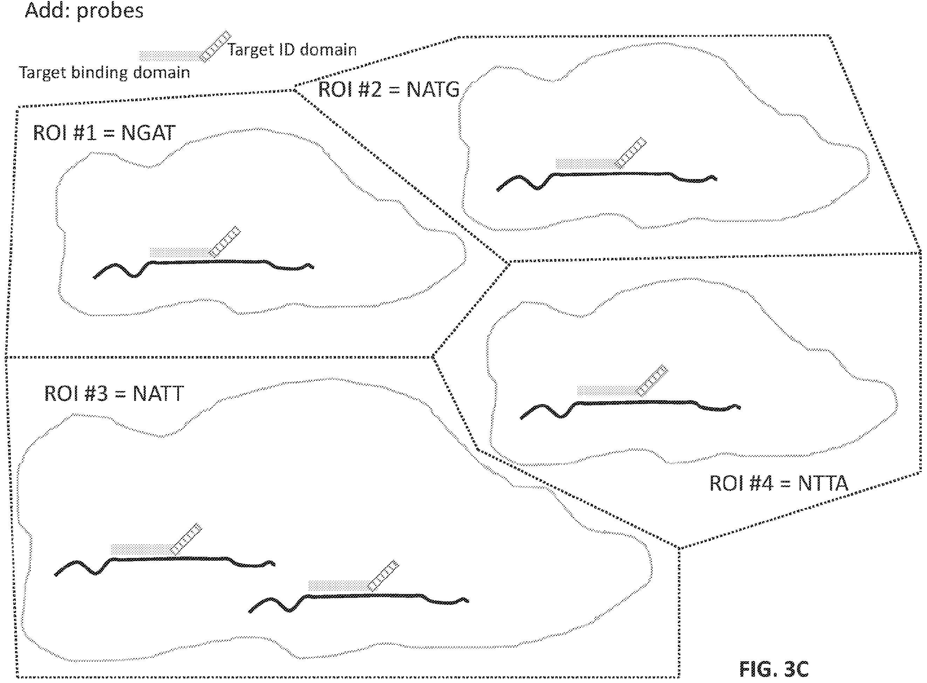

In a first step of the method, as shown in FIG. 3C, the sample is contacted with a plurality of probes, wherein the probes comprise a unique target-binding domain that binds to the target mRNA, a unique target identification domain specific for the target analyte, and a free 3'-OH moiety, as shown in the top left hand corner of FIG. 3C. The target binding domain is specific for the target mRNA and hybridizes to each target mRNA within each cell. As described further herein, the sample may be contacted with a plurality of probes comprising any number of different species of probes wherein each species of probe comprises a unique target-binding domain that binds to one of the at least two target analytes and a unique target identification domain specific for the target analyte, and a free 3'-OH moiety. FIGs. 3A-3N have a single species for probe for exemplary purposes only.

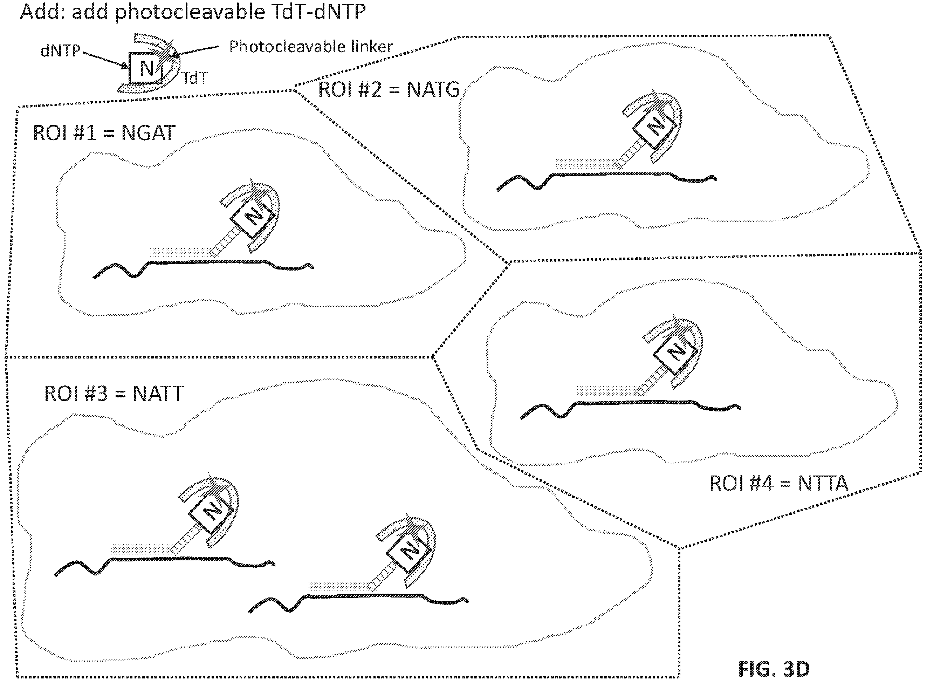

In the second step of the method, shown in FIG. 3D, the first nucleotide of the spatial barcode domains is ligated onto the free 3'-OH of each probe bound to a target mRNA by contacting the sample with a plurality of nucleotide-polymerase complexes. In this non-limiting example, the nucleotide polymerase complexes comprise a polymerase, TdT, operably linked to a nucleotide ("N") via a photocleavable linker (depicted as a four-cornered star-shape).

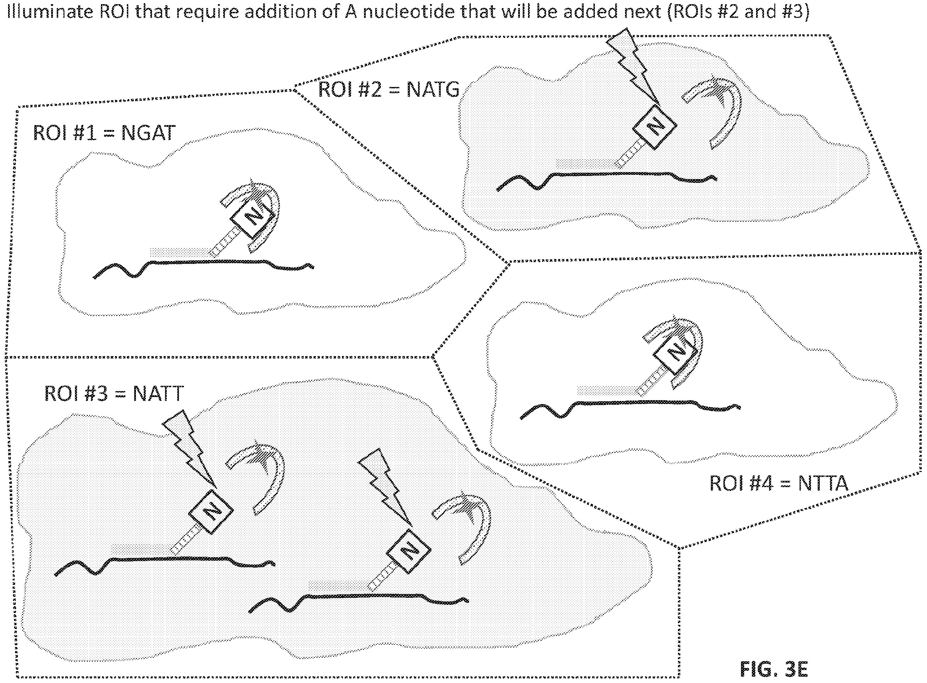

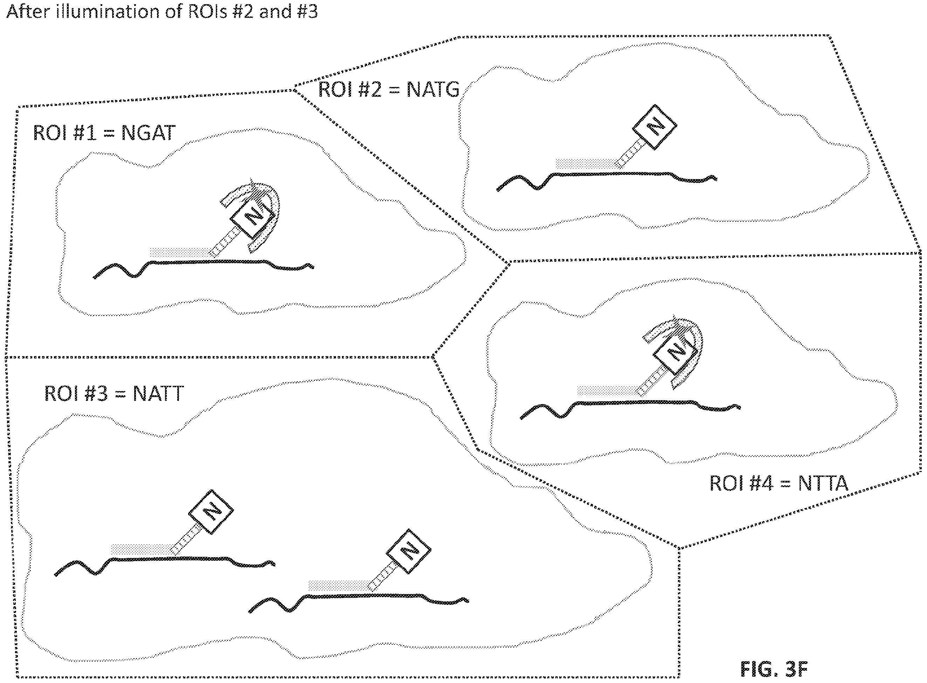

In the third step of the method, shown in FIG. 3E, the ROI's that require the addition of an "A" nucleotide to construct the appropriate spatial barcode are illuminated with light of sufficient wavelength to cleave the photocleavable linker of the nucleotide-polymerase complexes. In this non-limiting example, ROI #2 and ROI #3 require the addition of an "A" nucleotide, so these two ROI's are illuminated in this step. The illumination is represented by the lighting bolt shape in FIG. 3E and the shading of the illuminated cell. The result of this illumination is that, within the illuminated ROIs, the TdT molecules are liberated from the previously ligated "N" nucleotide, exposing a free 3'-OH moiety that can be ligated to in a subsequent step. FIG. 3F shows the sample after illumination of ROI #2 and ROI #3. The bound probes in ROI #2 and ROI #3 have free 3'-OH moieties as a result of the photocleavage. Conversely, the bound probes in ROI #1 and ROI #4, which do not require the addition of an A nucleotide, do not have free 3'-OH moieties as the TdT molecules are still bound by the intact photocleavable linker to the previously ligated "N" nucleotide.

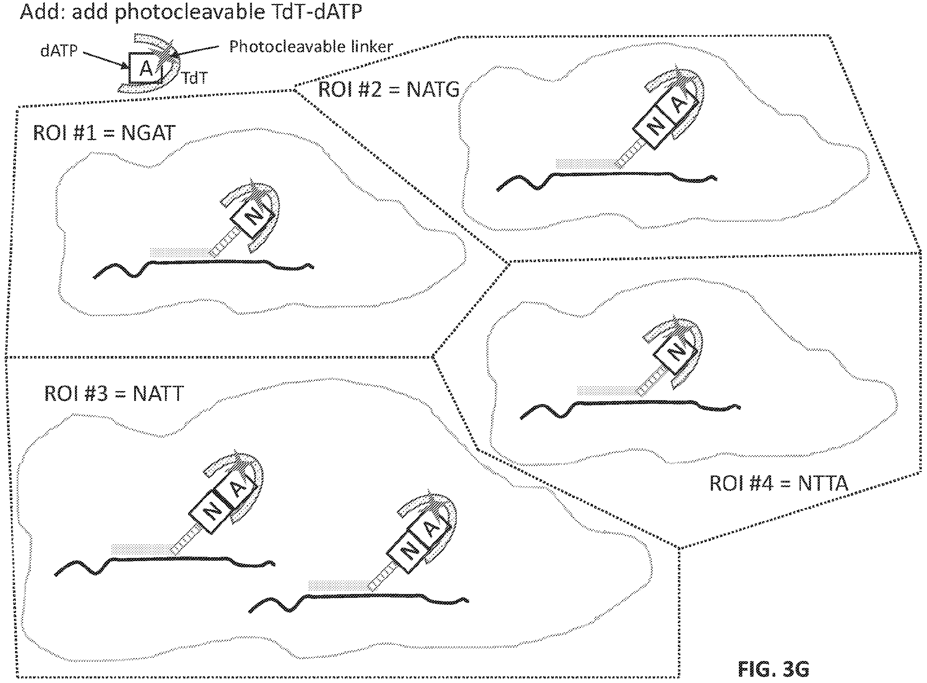

The method continues in FIG. 3G, where the sample is contacted with a plurality of nucleotide-polymerase complexes, wherein the nucleotide polymerase complexes comprise a dATP ("A") operably linked to a TdT molecule via a photocleavable linker (depicted as a four-cornered star shape). These A nucleotides are only ligated to the bound probes in ROI #2 and ROI #3 as these are the only probes that comprise a free 3'-OH moiety that are able to facilitate a ligation reaction. The 3'-termini of the bound probes in ROI #1 and ROI #4 are protected by the still-bound TdT molecules. Thus, an "A" nucleotide is added to the spatial barcodes in ROI #2 and ROI #3, further synthesizing the spatial barcodes in those ROI.

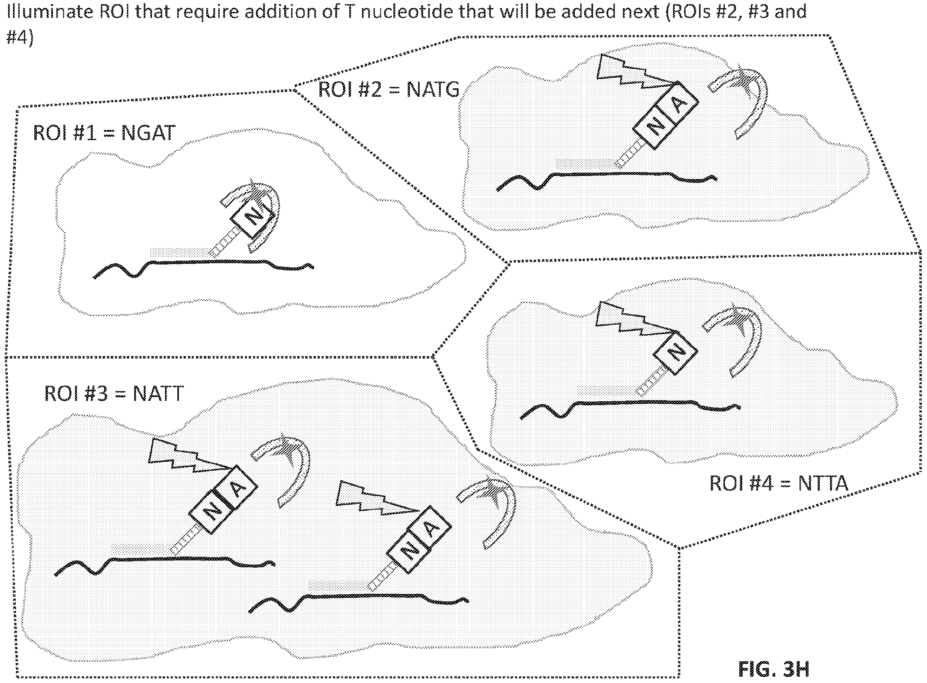

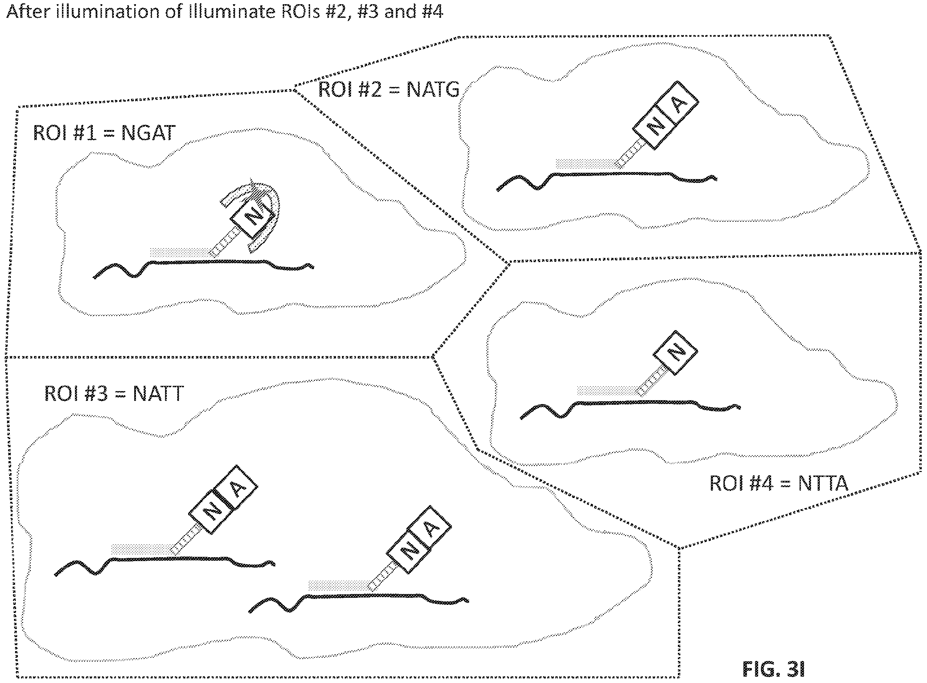

The method continues in FIG. 3H, where the ROI's that require the addition of a "T" nucleotide to construct the appropriate spatial barcode are illuminated with light of sufficient wavelength to cleave the photocleavable linker of the nucleotide-polymerase complexes. In this non-limiting example, ROI #2, ROI #3 and ROI #4 require the addition of a "T" nucleotide in the next step, so these three ROI's are illuminated. The result of this illumination is that, within the illuminated ROIs, the TdT molecules are liberated from the previously ligated nucleotide, exposing a free 3'-OH moiety that can be ligated to in a subsequent step. FIG. 3I shows the sample after illumination of ROI #2, ROI #3 and ROI #4. The bound probes in ROI #2, ROI #3 and ROI #4 have free 3'-OH moieties as a result of the photocleavage. Conversely, the bound probes in ROI #1, which do not require the addition of an T nucleotide, do not have free 3'-OH moieties as the TdT molecules are still bound by the intact photocleavable linker to the previously ligated nucleotide.

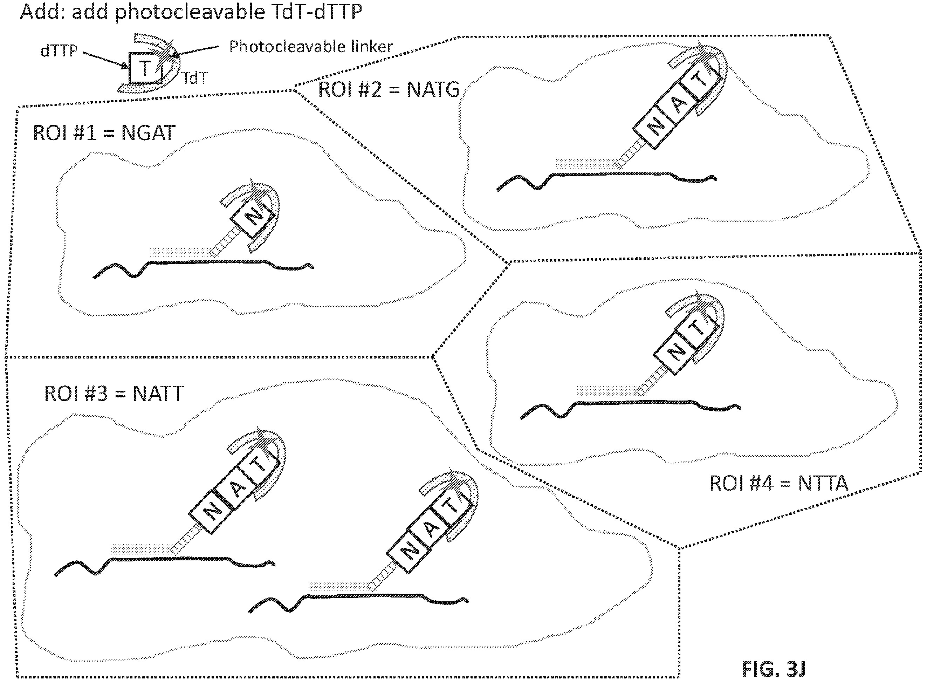

The method continues in FIG. 3J, where the sample is contacted with a plurality of nucleotide-polymerase complexes, wherein the nucleotide polymerase complexes comprise a dTTP ("T") operably linked to a TdT molecule via a photocleavable linker (depicted as a four-cornered star shape). These T nucleotides are only ligated to the bound probes in ROI #2, ROI #3 and ROI #4 as these are the only probes that comprise a free 3'-OH moiety that are able to facilitate a ligation reaction. The 3'-termini of the bound probes in ROI #1 are protected by the still-bound TdT molecules. Thus, an "T" nucleotide is added to the spatial barcodes in ROI #2, ROI #3 and ROI #4, further synthesizing the spatial barcodes in those ROI.

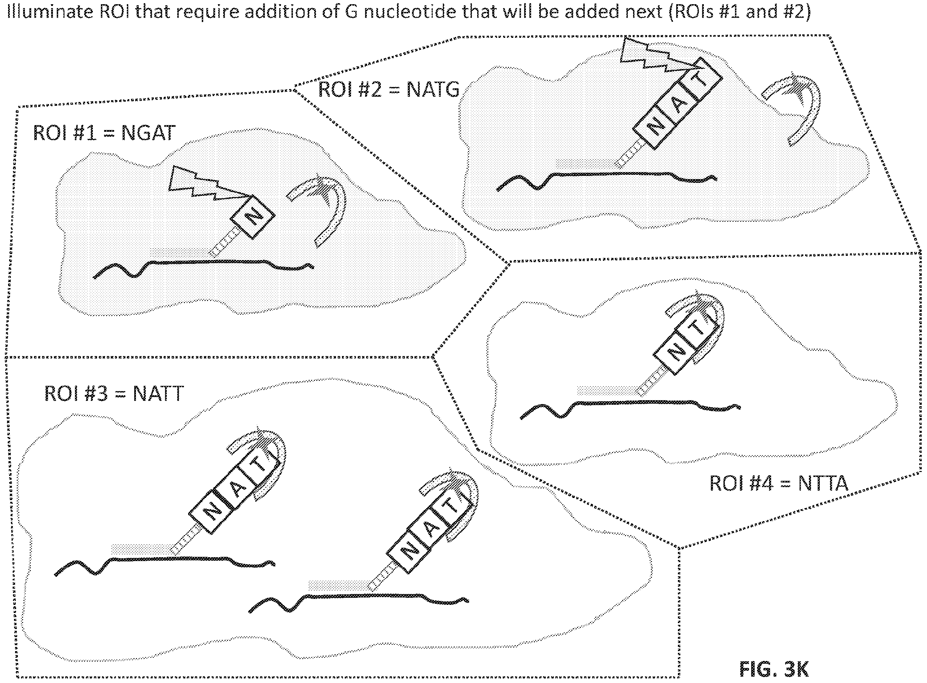

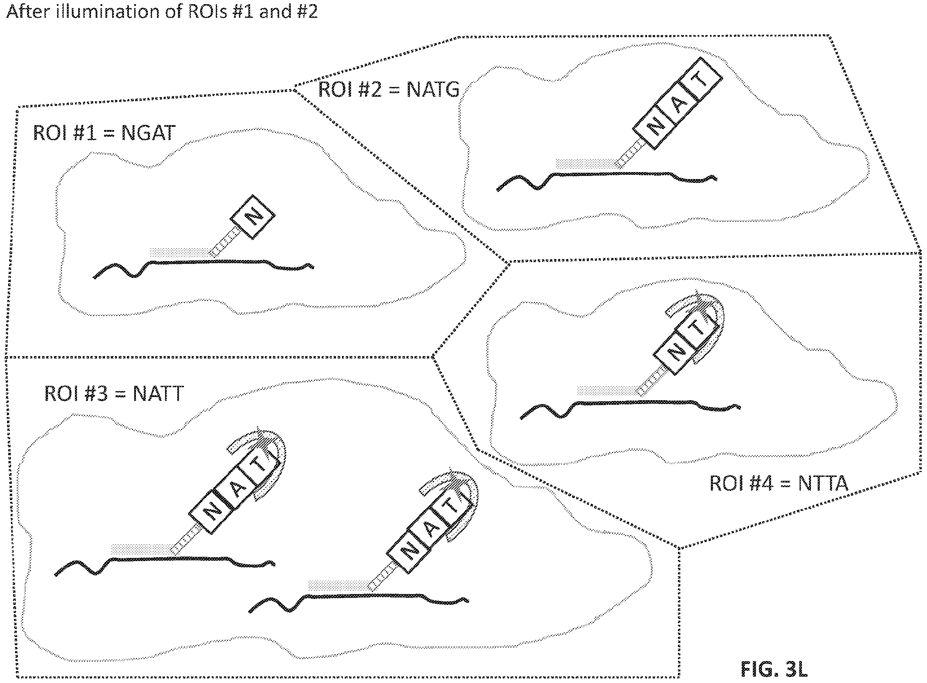

The method continues in FIG. 3K, where the ROI's that require the addition of a "G" nucleotide to construct the appropriate spatial barcode are illuminated with light of sufficient wavelength to cleave the photocleavable linker of the nucleotide-polymerase complexes. In this non-limiting example, ROI #1 and ROI #2 require the addition of a "G" nucleotide in the next step, so these two ROI's are illuminated. The result of this illumination is that, within the illuminated ROIs, the TdT molecules are liberated from the previously ligated nucleotide, exposing a free 3'-OH moiety that can be ligated to in a subsequent step. FIG. 3L shows the sample after illumination of ROI #1 and ROI #2. The bound probes in ROI #1 and ROI #2 have free 3'-OH moieties as a result of the photocleavage. Conversely, the bound probes in ROI #3 and ROI #4, which do not require the addition of an T nucleotide, do not have free 3'-OH moieties as the TdT molecules are still bound by the intact photocleavable linker to the previously ligated nucleotide.

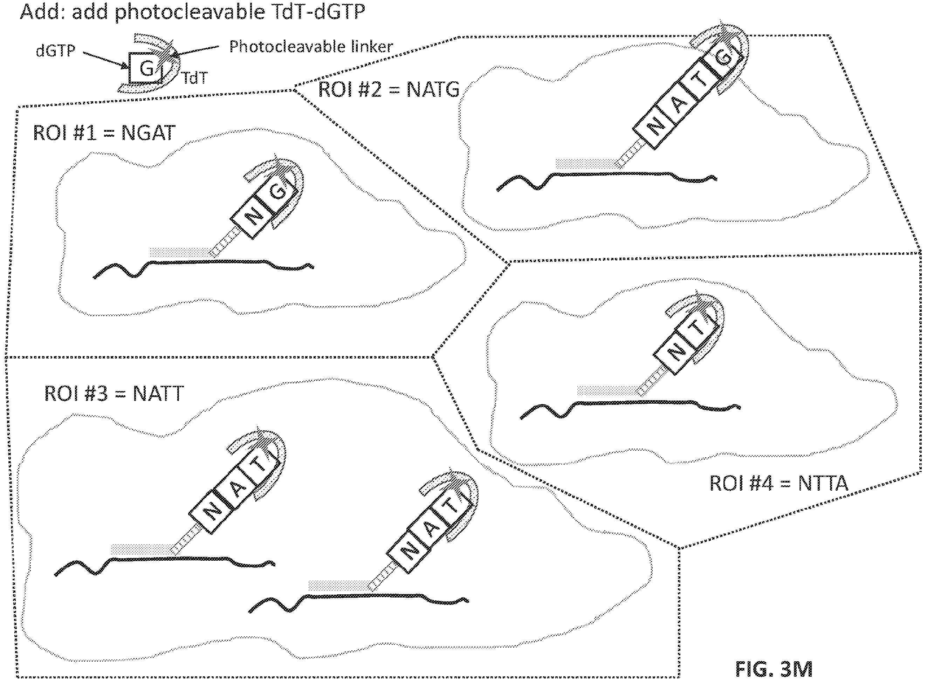

The method continues in FIG. 3M, where the sample is contacted with a plurality of nucleotide-polymerase complexes, wherein the nucleotide polymerase complexes comprise a dGTP ("G") operably linked to a TdT molecule via a photocleavable linker (depicted as a four-cornered star shape). These G nucleotides are only ligated to the bound probes in ROI #1 and ROI #2 as these are the only probes that comprise a free 3'-OH moiety that are able to facilitate a ligation reaction. The 3'-termini of the bound probes in ROI #3 and ROI #4 are protected by the still-bound TdT molecules. Thus, an "G" nucleotide is added to the spatial barcodes in ROI #2, ROI #3 and ROI #4, further synthesizing the spatial barcodes in those ROI. After this ligation, the bound probe in ROI #2 has a completely synthesized spatial barcode of "NATG".

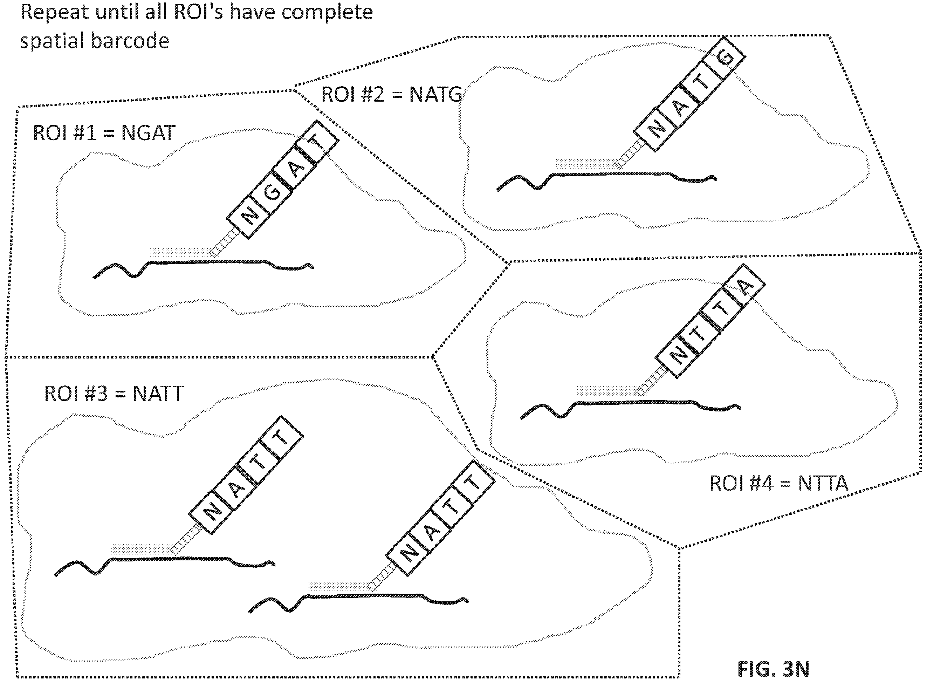

The above-described steps can be repeated any number of times with any of the four nucleotides (dATP, dGTP, dTTP and dCTP) until all of the bound probes in each ROI have complete spatial barcodes, as shown in FIG. 3N. After the completion of all of the spatial barcodes in each ROI, the bound probes can be washed from the sample, collected, and then sequenced. By sequencing the spatial barcode for each collected probe, the ROI from which that specific probe originated can be identified, allowing for the quantification of the abundance of each target mRNA within each ROI, thereby producing a spatially-resolved profile of the abundance of the target mRNA.

The order of nucleotide addition (*i.e.* A, then T then G) in FIGs 3A-3N are for exemplary purposes only. Nucleotides can be added to the spatial barcodes in any order and/or combination to synthesize the appropriate spatial barcodes for each ROI.

In some aspects of the methods of the invention and related methods provided for illustrative purposes, a nucleotide-polymerase complex comprises a nucleotide operably linked to a polymerase via a photocleavable linker.

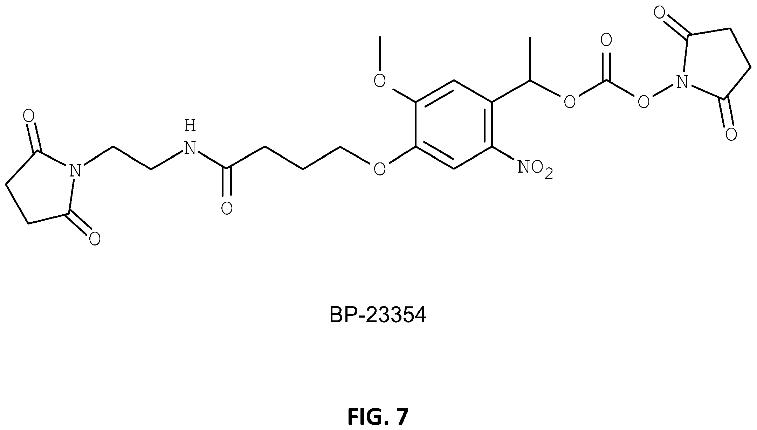

In some aspects, a photocleavable linker can comprise an amine-to-thiol crosslinker. In some aspects, an amine-to-thiol crosslinker can comprise PEG₄-SPDP (2-pyridyldithiol-tetraoxatetradecase-*N-*hydroxysuccinimide). In some aspects, a photocleavable linker can comprise a maleimide-NHS carbonate crosslinker such as BP-23354. FIG. 7 shows the structure of BP-23354.

In some aspects, a nucleotide-polymerase complex can comprise a nucleotide-polymerase complex described in Palluk et al. Nature Biotechnology, 2018, Vol. 36, No. 7, pgs. 645-650.

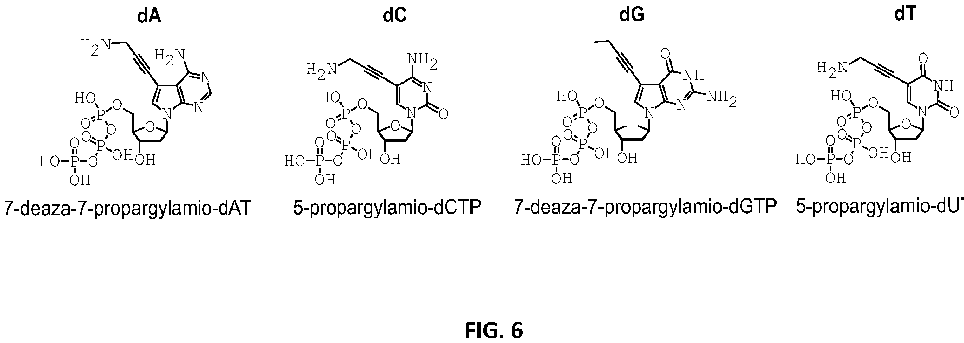

In some aspects, the nucleotide in a nucleotide-polymerase complex can comprise a nucleotide analogue. In some aspects, a nucleotide analogue can be a proparglyamino dNTP. In some aspects, a nucleotide analogue can be 7-deaza-7-proparglyamino-dATP, 5-proparglyamino-dCTP, 7-deaza-7-proparglyamino-dGRP or 5-proparglyamino-dUTP. FIG. 6 shows the structures of these various proparglyamino dNTPs.

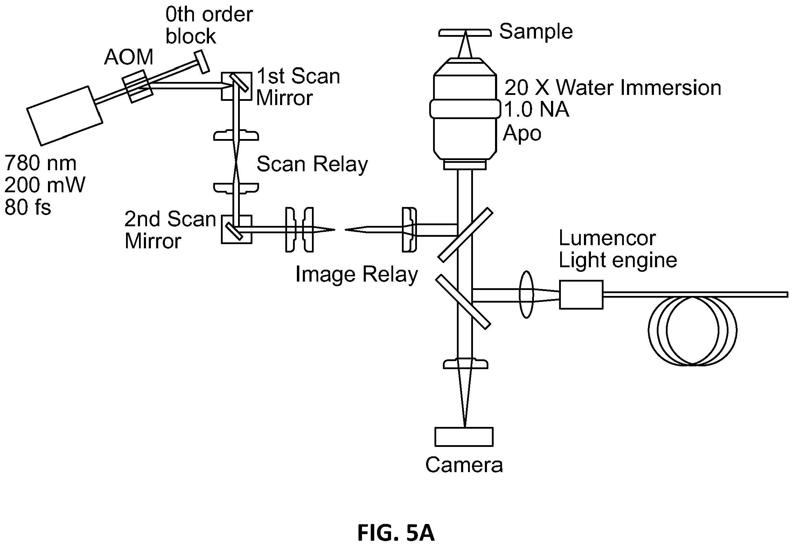

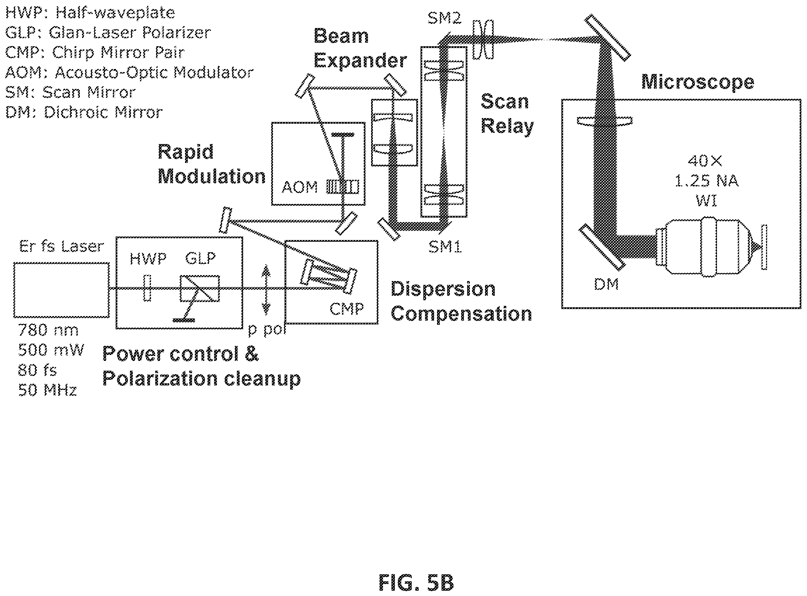

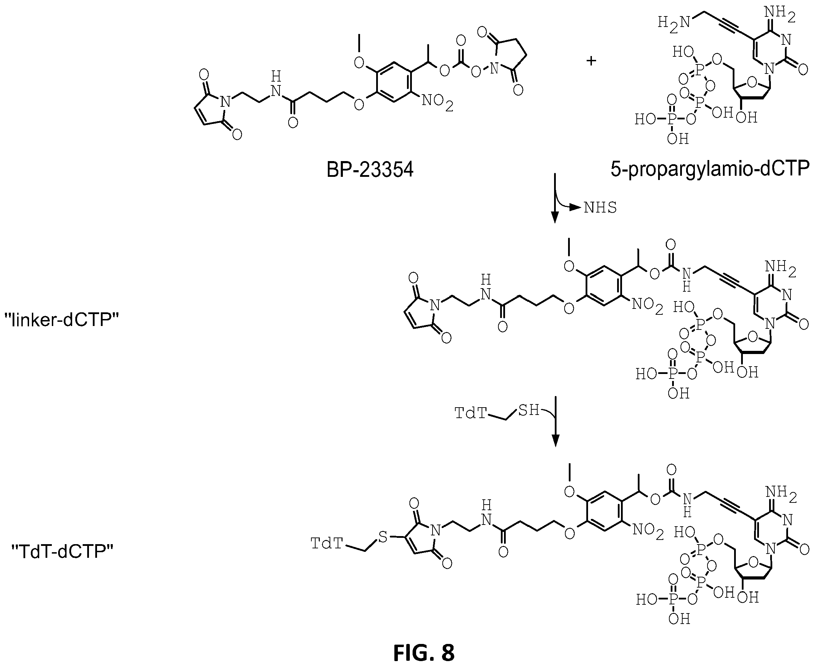

In some aspects, a nucleotide-polymerase complex can be prepared by reacting a proparglyamino dNTP with an amine-to-thiol crosslinker, such as, but not limited to, BP-23354, to form a photocleavable linker-nucleotide "linker-dNTP". The maleimide moiety of the linker-dNTP can then be reacted with a polymerase, such as, but not limited to, TdT, containing a single exposed cysteine residue to form the nucleotide-polymerase complex. FIG. 8 is a schematic of an exemplary nucleotide-polymerase complex preparation method. In FIG. 8, 5-proparglyamino-dCTP is first reacted with BP-23354 to form "linker-dCTP". The linker-dCTP is then reacted with a TdT polymerase that comprises a single exposed cysteine to form "TdT-dCTP". In this example, dCTP can be substituted with any other proparglyamino dNTP, including, but not limited to 7-deaza-7-proparglyamino-dATP, 7-deaza-7-proparglyamino-dGRP or 5-proparglyamino-dUTP.

In some aspects, the polymerase in a nucleotide polymerase complex can be terminal deoxynucleotidyl transferase (TdT) or a biologically-active portion thereof. In some aspects, the TdT can be *Mus musculus* TdT. In some aspects, the TdT can be the short isoform of *Mus musculus* TdT, which has the amino acid sequence:

MDPLQAVHLGPRKKRPRQLGTPVASTPYDIRFRDLVLFILEKKMGTTRRAFLMELARRK
GFRVENELSDSVTHIVAENNSGSDVLEWLQLQNIKASSELELLDISWLIECMGAGKPVE
MMGRHQLVVNRNSSPSPVPGSQNVPAPAVKKISQYACQRRTTLNNYNQLFTDALDILA
ENDELRENEGSCLAFMRASSVLKSLPFPITSMKDTEGIPCLGDKVKSIIEGIIEDGESSEAK
AVLNDERYKSFKLFTSVFGVGLKTAEKWFRMGFRTLSKIQSDKSLRFTQMQKAGFLYY
EDLVSCVNRPEAEAVSMLVKEAVVTFLPDALVTMTGGFRRGKMTGHDVDFLITSPEAT
EDEEQQLLHKVTDFWKQQGLLLYCDILESTFEKFKQPSRKVDALDHFQKCFLILKLDHG
RVHSEKSGQQEGKGWKAIRVDLVMCPYDRRAFALLGWTGSRQFERDLRRYATHERKM
MLDNHALYDRTKRVFLEAESEEEIFAHLGLDYIEPWERNA (SEQ ID NO: 1)

In some aspects, the polymerase in a nucleotide polymerase complex can comprise residues 132-510 of the short isoform of *Mus musculus* TdT. The amino acid sequence of residues 132-510 of the short isoform *Mus musculus* TdT is:

SPSPVPGSQNVPAPAVKKISQYACQRRTTLNNYNQLFTDALDILAENDELRENEGSCLAF
MRASSVLKSLPFPITSMKDTEGIPCLGDKVKSIIEGIIEDGESSEAKAVLNDERYKSFKLFT
SVFGVGLKTAEKWFRMGFRTLSKIQSDKSLRFTQMQKAGFLYYEDLVSCVNRPEAEAV
SMLVKEAVVTFLPDALVTMTGGFRRGKMTGHDVDFLITSPEATEDEEQQLLHKVTDFW
KQQGLLLYCDILESTFEKFKQPSRKVDALDHFQKCFLILKLDHGRVHSEKSGQQEGKGW
KAIRVDLVMCPYDRRAFALLGWTGSRQFERDLRRYATHERKMMLDNHALYDRTKRVF
LEAESEEEIFAHLGLDYIEPWERNA (SEQ ID NO: 2)

In some aspects, the polymerase in a nucleotide polymerase complex can comprise residues 132-510 of the short isoform of *Mus musculus* TdT, wherein all exposed native cysteine residues have been removed, such that the cysteine at position 188 is substituted by Alanine, the cysteine at position 216 is substituted by Serine, the cysteine at position 302 is substituted by Alanine, the cysteine at position 378 is substituted by Alanine and the cysteine at position 438 is substituted by Serine. This sequence, herein referred to as "exposed Cys-less 132-510 *Mus musculus* TdT" has the amino acid sequence:

SPSPVPGSQNVPAPAVKKISQYACQRRTTLNNYNQLFTDALDILAENDELRENEGSALAF
MRASSVLKSLPFPITSMKDTEGIPSLGDKVKSIIEGIIEDGESSEAKAVLNDERYKSFKLFT
SVFGVGLKTAEKWFRMGFRTLSKIQSDKSLRFTQMQKAGFLYYEDLVSAVNRPEAEAV
SMLVKEAVVTFLPDALVTMTGGFRRGKMTGHDVDFLITSPEATEDEEQQLLHKVTDFW
KQQGLLLYADILESTFEKFKQPSRKVDALDHFQKCFLILKLDHGRVHSEKSGQQEGKGW
KAIRVDLVMSPYDRRAFALLGWTGSRQFERDLRRYATHERKMMLDNHALYDRTKRVF
LEAESEEEIFAHLGLDYIEPWERNA (SEQ ID NO: 3)

In some aspects, the polymerase in a nucleotide polymerase complex can comprise the sequence of exposed Cys-less 132-510 *Mus musculus* TdT, wherein the Glutamic Acid at position 180 is replaced with a cysteine residue. This sequence, herein referred to as MmTdT180 has the amino acid sequence:

SPSPVPGSQNVPAPAVKKISQYACQRRTTLNNYNQLFTDALDILAENDCLRENEGSALAF
MRASSVLKSLPFPITSMKDTEGIPSLGDKVKSIIEGIIEDGESSEAKAVLNDERYKSFKLFT
SVFGVGLKTAEKWFRMGFRTLSKIQSDKSLRFTQMQKAGFLYYEDLVSAVNRPEAEAV

SMLVKEAVVTFLPDALVTMTGGFRRGKMTGHDVDFLITSPEATEDEEQQLLHKVTDFW
KQQGLLLYADILESTFEKFKQPSRKVDALDHFQKCFLILKLDHGRVHSEKSGQQEGKGW
KAIRVDLVMSPYDRRAFALLGWTGSRQFERDLRRYATHERKMMLDNHALYDRTKRVF
LEAESEEEIFAHLGLDYIEPWERNA (SEQ ID NO: 4)

In some aspects, the polymerase in a nucleotide polymerase complex can comprise the sequence of exposed Cys-less 132-510 *Mus musculus* TdT, wherein the Alanine at position 188 is replaced with a cysteine residue. This sequence, herein referred to as MmTdT188 has the amino acid sequence:

SPSPVPGSQNVPAPAVKKISQYACQRRTTLNNYNQLFTDALDILAENDELRENEGSCLAF
MRASSVLKSLPFPITSMKDTEGIPSLGDKVKSIIEGIIEDGESSEAKAVLNDERYKSFKLFT
SVFGVGLKTAEKWFRMGFRTLSKIQSDKSLRFTQMQKAGFLYYEDLVSAVNRPEAEAV
SMLVKEAVVTFLPDALVTMTGGFRRGKMTGHDVDFLITSPEATEDEEQQLLHKVTDFW
KQQGLLLYADILESTFEKFKQPSRKVDALDHFQKCFLILKLDHGRVHSEKSGQQEGKGW
KAIRVDLVMSPYDRRAFALLGWTGSRQFERDLRRYATHERKMMLDNHALYDRTKRVF
LEAESEEEIFAHLGLDYIEPWERNA (SEQ ID NO: 5)

In some aspects, the polymerase in a nucleotide polymerase complex can comprise the sequence of exposed Cys-less 132-510 *Mus musculus* TdT, wherein the Threonine at position 253 is replaced with a cysteine residue. This sequence, herein referred to as MmTdT253 has the amino acid sequence:

SPSPVPGSQNVPAPAVKKISQYACQRRTTLNNYNQLFTDALDILAENDELRENEGSALAF
MRASSVLKSLPFPITSMKDTEGIPSLGDKVKSIIEGIIEDGESSEAKAVLNDERYKSFKLFC
SVFGVGLKTAEKWFRMGFRTLSKIQSDKSLRFTQMQKAGFLYYEDLVSAVNRPEAEAV
SMLVKEAVVTFLPDALVTMTGGFRRGKMTGHDVDFLITSPEATEDEEQQLLHKVTDFW
KQQGLLLYADILESTFEKFKQPSRKVDALDHFQKCFLILKLDHGRVHSEKSGQQEGKGW
KAIRVDLVMSPYDRRAFALLGWTGSRQFERDLRRYATHERKMMLDNHALYDRTKRVF
LEAESEEEIFAHLGLDYIEPWERNA (SEQ ID NO: 6)

In some aspects, the polymerase in a nucleotide polymerase complex can comprise the sequence of exposed Cys-less 132-510 *Mus musculus* TdT, wherein the Alanine at position 302 is replaced with a cysteine residue. This sequence, herein referred to as MmTdT253 has the amino acid sequence:

SPSPVPGSQNVPAPAVKKISQYACQRRTTLNNYNQLFTDALDILAENDELRENEGSALAF
MRASSVLKSLPFPITSMKDTEGIPSLGDKVKSIIEGIIEDGESSEAKAVLNDERYKSFKLFT
SVFGVGLKTAEKWFRMGFRTLSKIQSDKSLRFTQMQKAGFLYYEDLVSCVNRPEAEAV
SMLVKEAVVTFLPDALVTMTGGFRRGKMTGHDVDFLITSPEATEDEEQQLLHKVTDFW
KQQGLLLYADILESTFEKFKQPSRKVDALDHFQKCFLILKLDHGRVHSEKSGQQEGKGW
KAIRVDLVMSPYDRRAFALLGWTGSRQFERDLRRYATHERKMMLDNHALYDRTKRVF
LEAESEEEIFAHLGLDYIEPWERNA (SEQ ID NO: 7)

In some aspects, the polymerase in a nucleotide polymerase complex can be a Maltose Binding Protein (MBP)-fusion protein. In some aspects, MBP can be fused to the N-terminus of the polymerase with an amino acid linker of any length between the MBP and the start of the polymerase.

The present invention provides a method of producing a spatially-resolved profile of the abundance of at least two target analytes in a first and an at least second location of a tissue sample comprising: a) contacting the tissue sample with a solution comprising at least two species of probes, the probes comprising a target-binding domain and a target identification domain, wherein each species of probe comprises a unique target-binding domain that binds to one of the at least two target analytes and a unique target identification domain specific for the target analyte, and a free 3'-OH moiety; b) ligating a nucleotide to the free 3'-OH moiety of the barcode domain of at least one bound probe by contacting the tissue sample with a first plurality of reversible terminator nucleotides and a first plurality of polymerases, thereby extending the at least one bound probe and forming a spatial barcode domain, wherein at least one reversible terminator nucleotide in the first plurality comprises the nucleotide operably linked to a 3' terminator moiety via a photocleavable linker; c) illuminating at least one location of the tissue sample with light sufficient to cleave the photocleavable linker of the reversible terminator nucleotides, thereby releasing the reversible 3' terminator moieties and exposing a free 3'-OH moiety on the spatial barcode domain at the at least one location of the tissue sample; d) ligating a nucleotide to the free 3'-OH moiety of the spatial barcode domain of at least one bound probe at the at least one location illuminated in step (c) by contacting the tissue sample with an additional plurality of reversible terminator nucleotides and an additional plurality of polymerases, thereby extending the spatial barcode domain of the at least one bound probe, wherein at least one reversible terminator nucleotide in the additional plurality comprises the nucleotide operably linked to a reversible 3' terminator moiety via a photocleavable linker; e) repeating steps (c) and (d) until the spatial barcode domain of at least one probe bound to a target analyte in the first location of the tissue sample has been extended such that the spatial barcode domain comprises a unique nucleic acid sequence specific to the first location of the tissue sample, the spatial barcode domain of at least one probe bound to a target analyte in the at least second location of the tissue sample has been extended such that the spatial barcode domain comprises a unique nucleic acid sequence specific to the at least second location of the tissue sample; f) collecting the probes bound to target analytes in the tissue sample; and g) quantifying via sequencing the probes collected in step (f), thereby determining the abundance of the at least two target analytes in the first and the at least second location of the tissue sample, thereby producing a spatially-resolved profile of the abundance of the at least two target analytes.

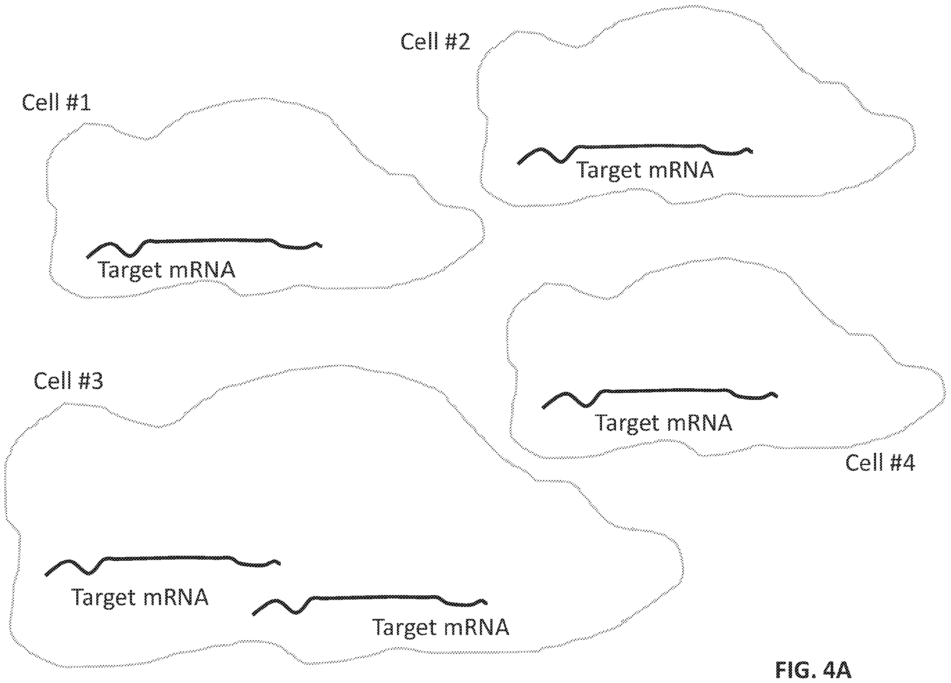

A schematic of a non-limiting example of the preceding method is shown in FIG. 4A-4N. The method begins in FIG. 4A with a sample. In this non-limiting example, the sample comprises four cells, each cell comprising at least one copy of the specific target mRNA to be measured. Cell #1, Cell #2 and Cell #3 comprise one copy of the target mRNA and Cell #3 comprises two copies of the target mRNA.

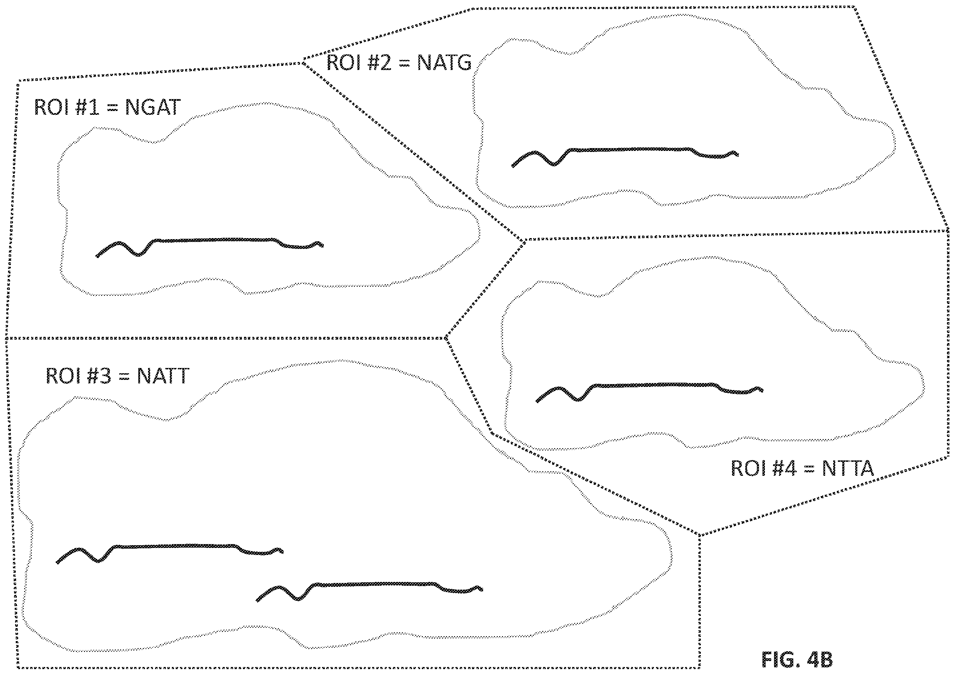

The sample can be subdivided into regions of interest (ROI) as shown in FIG. 4B. In this non-limiting example, each ROI comprises one of the cells. Moreover, each ROI is pre-assigned a specific spatial barcode that is unique to that ROI. In this example, each spatial barcode comprises 4 nucleotides, wherein the first nucleotide is any nucleotide (as represented by "N"). The spatial barcode for ROI #1 is NGAT, the spatial barcode for ROI #2 NATG, the spatial barcode for ROI #3 is NATT and the spatial barcode for ROI #4 is NTTA. As described further herein, the spatial barcode can comprise any number of nucleotides, and the four nucleotide spatial barcodes depicted in FIGs 4A-4N are for exemplary purposes only.

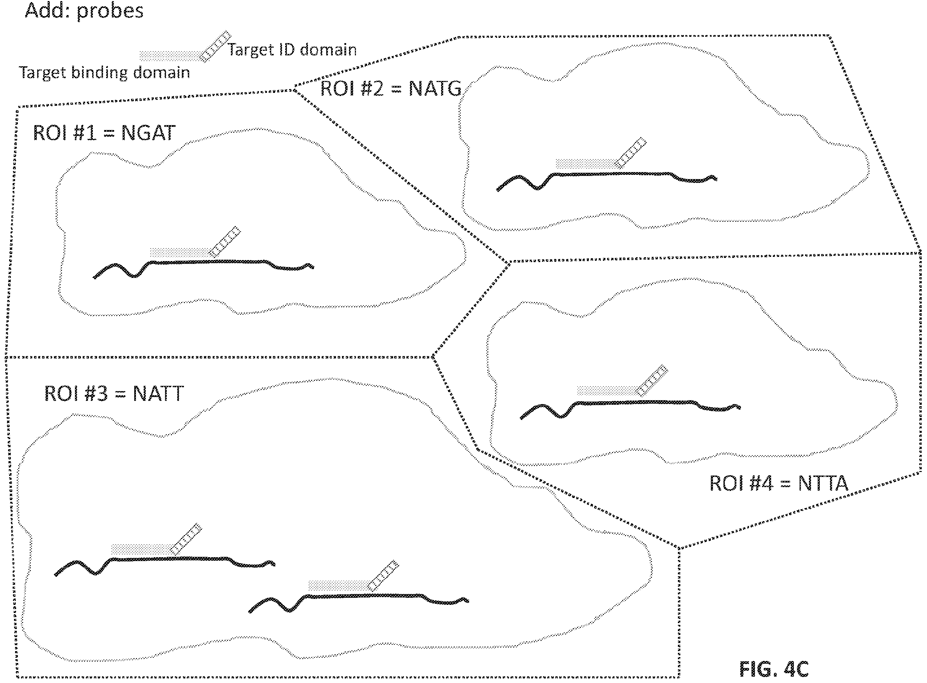

In a first step of the method, as shown in FIG. 4C, the sample is contacted with a plurality of probes, wherein the probes comprise a unique target-binding domain that binds to the target mRNA, a unique target identification domain specific for the target analyte, and a free 3'-OH moiety, as shown in the top left hand corner of FIG. 4C. The target binding domain is specific for the target mRNA and hybridizes to each target mRNA within each cell. As described further herein, the sample may be contacted with a plurality of probes comprising any number of different species of probes wherein each species of probe comprises a unique target-binding domain that binds to one of the at least two target analytes and a unique target identification domain specific for the target analyte, and a free 3'-OH moiety. FIGs. 4A-4N have a single species for probe for exemplary purposes only.

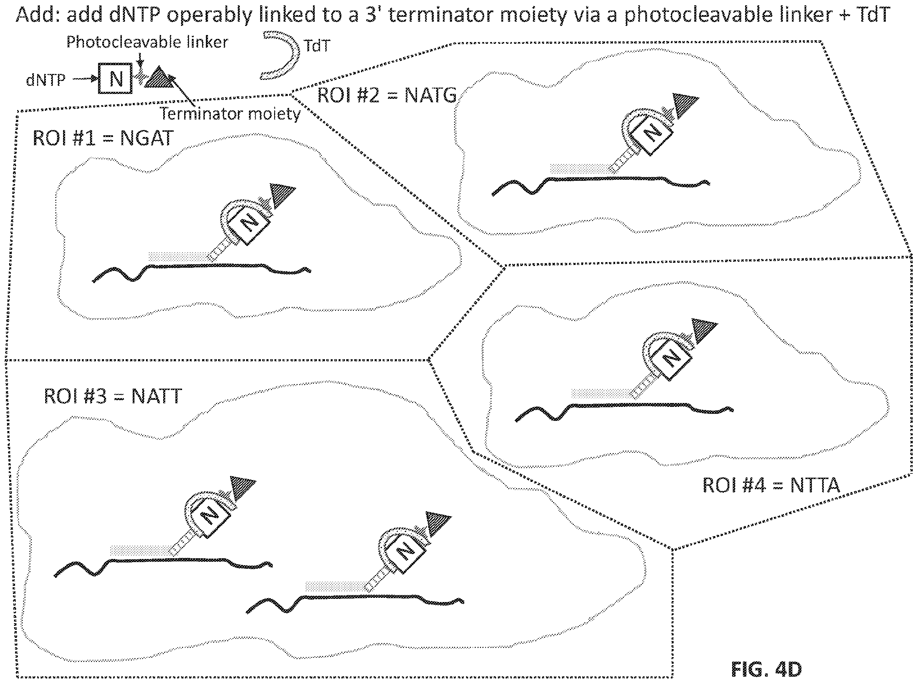

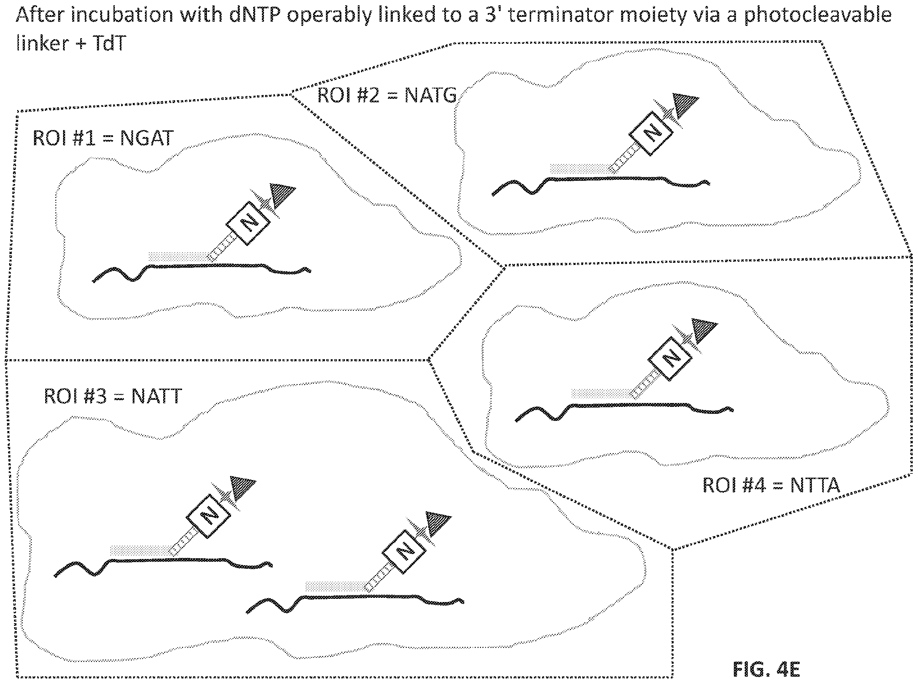

In the second step of the method, shown in FIG. 4D, the first nucleotide of the spatial barcode domains is ligated onto the free 3'-OH of each probe bound to a target mRNA by contacting the sample with a plurality of reversible terminator nucleotides and a plurality of polymerases. In this non-limiting example, the reversible terminator nucleotides comprise a nucleotide (dNTP, "N") operably linked to a terminator moiety (depicted as a triangle) via a photocleavable linker (depicted as a four-cornered star-shape). In this non-limiting example, the polymerase is TdT. After incubation with the reversible terminator nucleotides and polymerases, each bound probe has been extended by one nucleotide, "N", as shown in FIG. 4E.

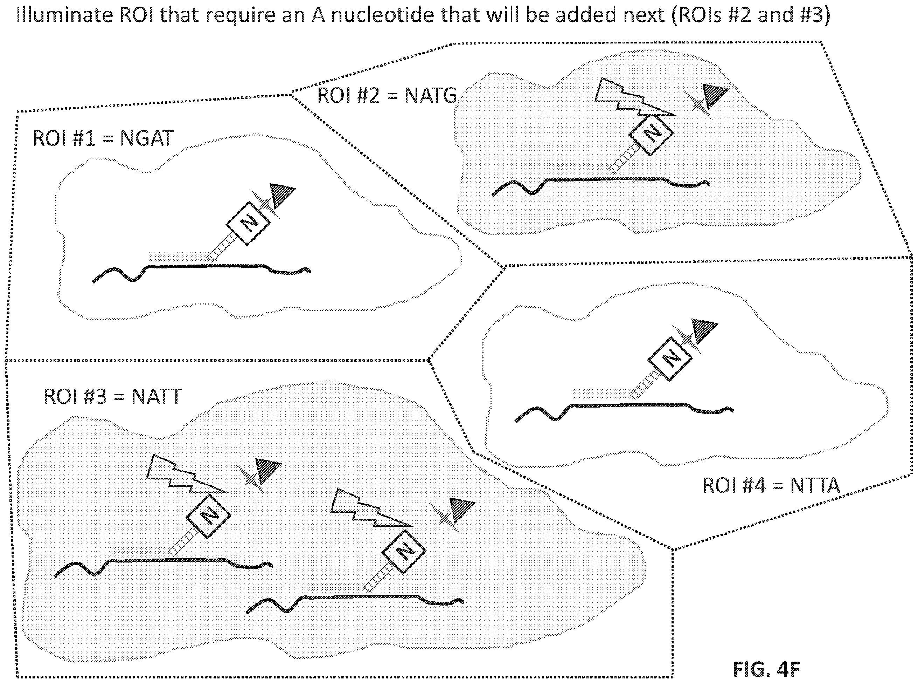

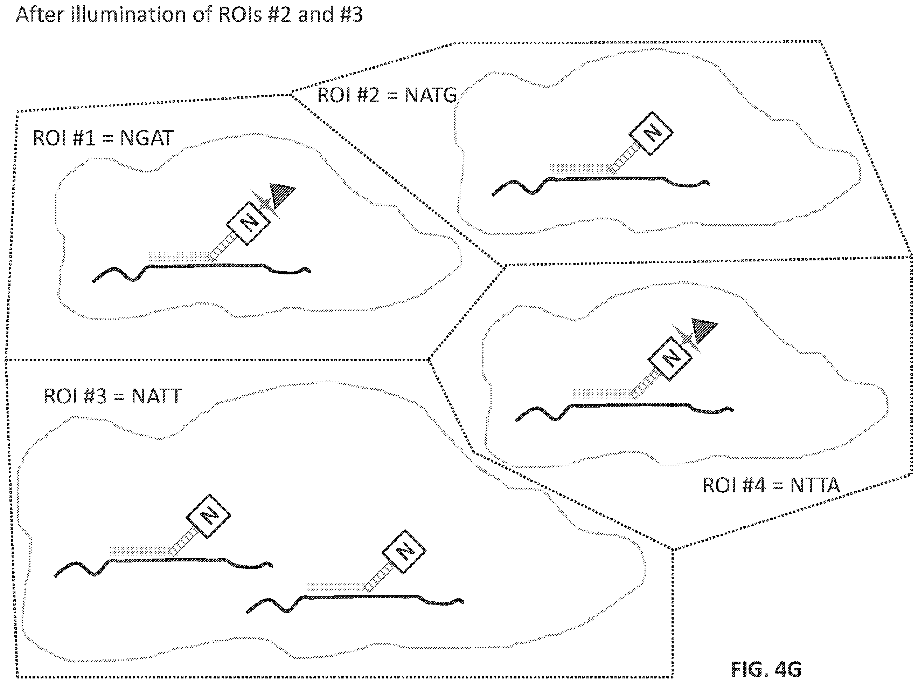

In the next step of the method, shown in FIG. 4F, the ROI's that require the addition of an "A" nucleotide to construct the appropriate spatial barcode are illuminated with light of sufficient wavelength to cleave the photocleavable linker of the reversible terminator nucleotides complexes. In this non-limiting example, ROI #2 and ROI #3 require the addition of an "A" nucleotide, so these two ROI's are illuminated in this step. The illumination is represented by the lightning bolt shape in FIG. 4F and the shading of the illuminated cell. The result of this illumination is that, within the illuminated ROIs, the terminator moieties are liberated from the previously ligated "N" nucleotide, exposing a free 3'-OH moiety that can be ligated to in a subsequent step. FIG. 4G shows the sample after illumination of ROI #2 and ROI #3. The bound probes in ROI #2 and ROI #3 have free 3'-OH moieties as a result of the photocleavage. Conversely, the bound probes in ROI #1 and ROI #4, which do not require the addition of an A nucleotide, do not have free 3'-OH moieties as the terminator moieties are still bound by the intact photocleavable linker to the previously ligated "N" nucleotide.

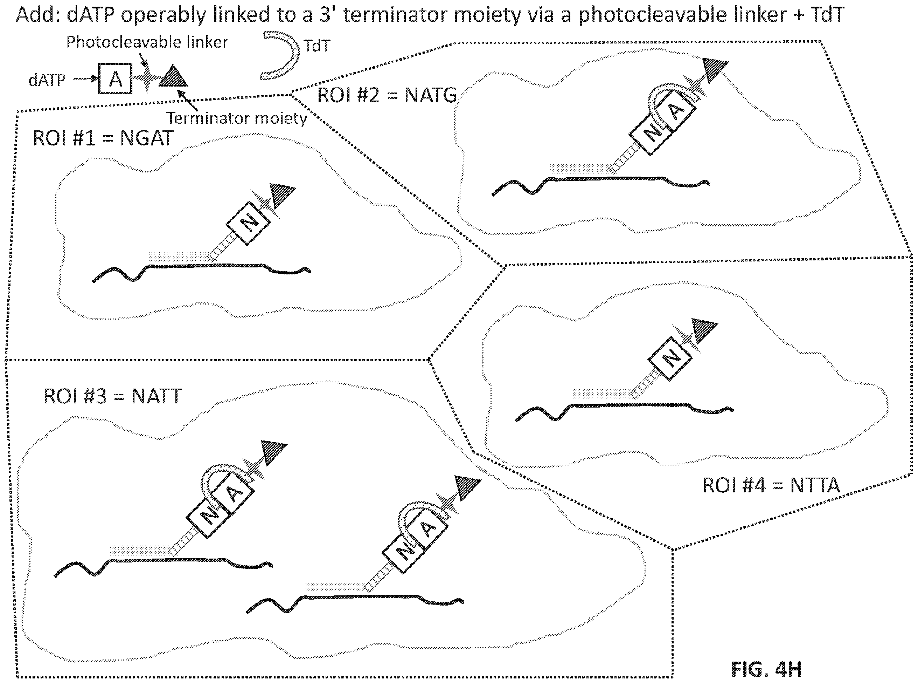

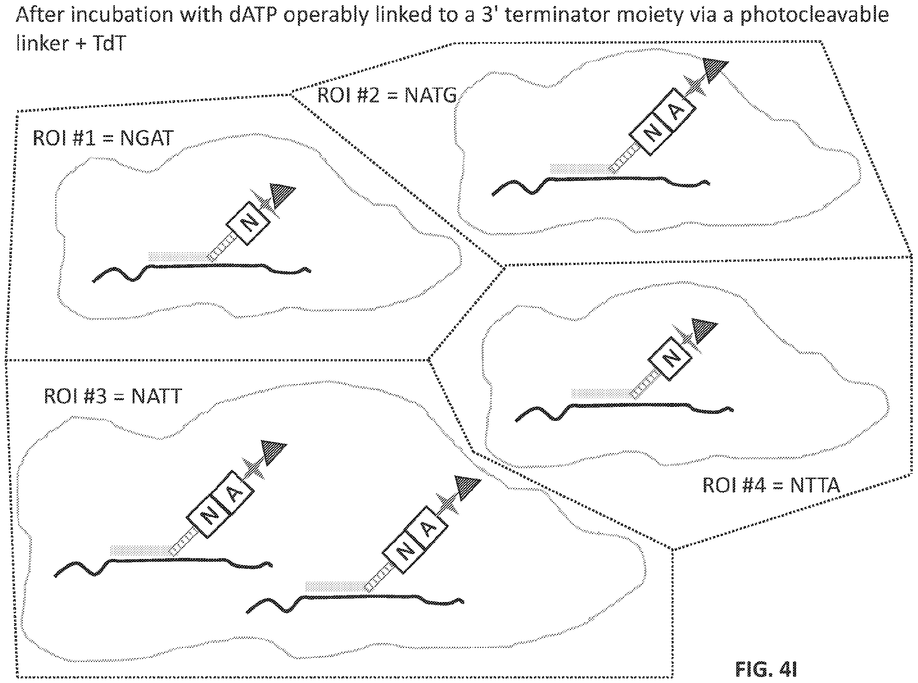

The method continues in FIG. 4H, where the sample is contacted with plurality of reversible terminator nucleotides and a plurality of polymerases. The reversible terminator nucleotides comprise a dATP ("A") operably linked to a terminator moiety (depicted as a triangle) via a photocleavable linker (depicted as a four-cornered star shape). These A nucleotides are only ligated to the bound probes in ROI #2 and ROI #3 as these are the only probes that comprise a free 3'-OH moiety that are able to facilitate a ligation reaction. The 3'-termini of the bound probes in ROI #1 and ROI #4 are protected by the still-bound terminator moieties. As shown in FIG. 4I, an "A" nucleotide is added to the spatial barcodes in ROI #2 and ROI #3, further synthesizing the spatial barcodes in those ROI.

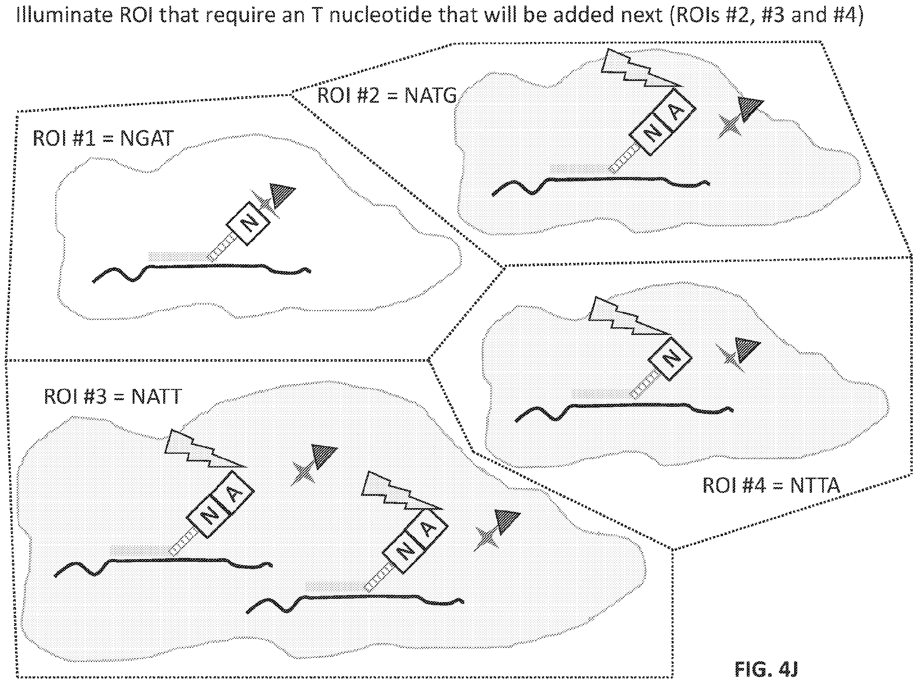

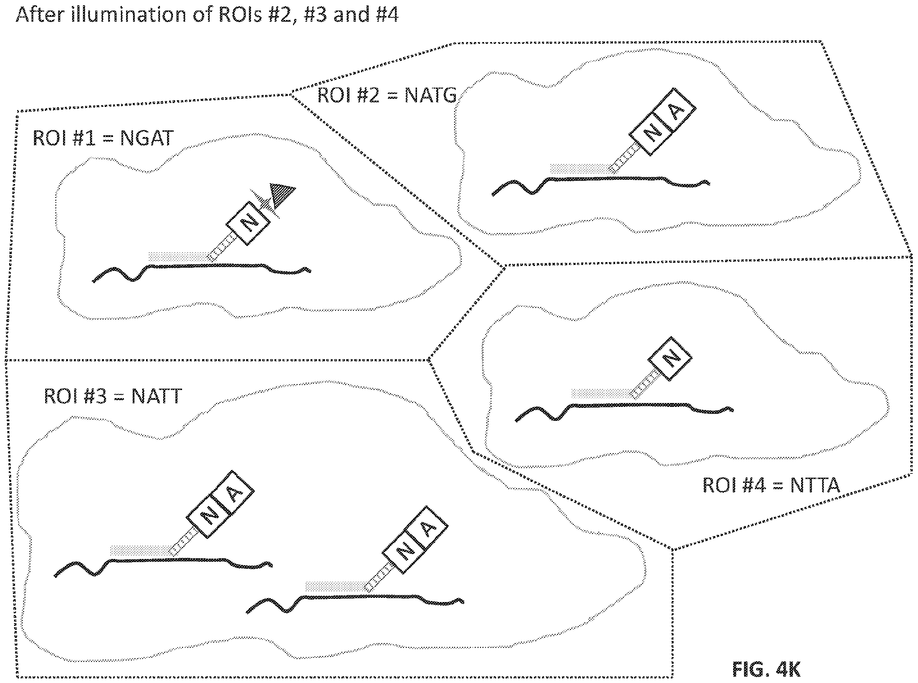

The method continues in FIG. 4J, where the ROI's that require the addition of a "T" nucleotide to construct the appropriate spatial barcode are illuminated with light of sufficient wavelength to cleave the photocleavable linker of the reversible terminator nucleotides. In this non-limiting example, ROI #2, ROI #3 and ROI #4 require the addition of a "T" nucleotide in the next step, so these three ROI's are illuminated. The result of this illumination is that, within the illuminated ROIs, the terminator moieties are liberated from the previously ligated nucleotide, exposing a free 3' -OH moiety that can be ligated to in a subsequent step. FIG. 4K shows the sample after illumination of ROI #2, ROI #3 and ROI #4. The bound probes in ROI #2, ROI #3 and ROI #4 have free 3'-OH moieties as a result of the photocleavage. Conversely, the bound probes in ROI #1, which do not require the addition of an T nucleotide, do not have free 3'-OH moieties as the terminator moieties are still bound by the intact photocleavable linker to the previously ligated nucleotide.

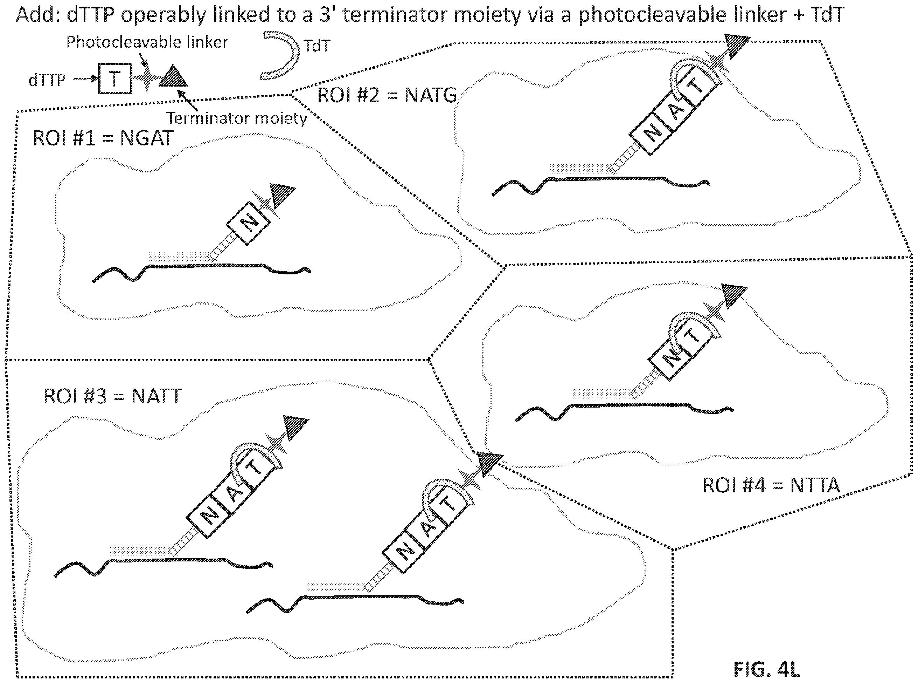

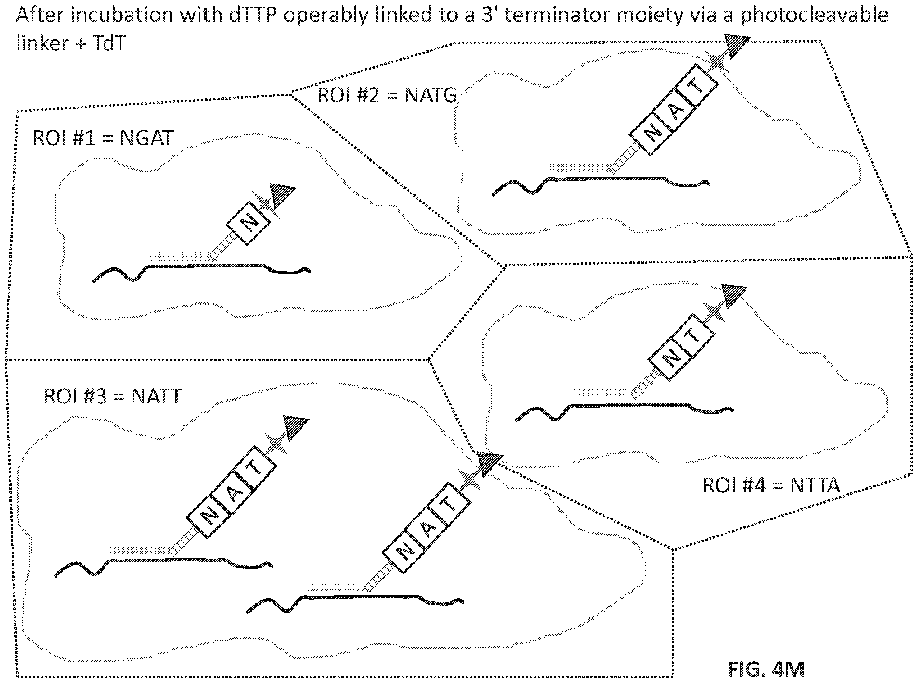

The method continues in FIG. 4L, where the sample is contacted with plurality of reversible terminator nucleotides and a plurality of polymerases. The reversible terminator nucleotides comprise a dTTP ("T") operably linked to a terminator moiety (depicted as a triangle) via a photocleavable linker (depicted as a four-cornered star shape). These T nucleotides are only ligated to the bound probes in ROI #2, ROI #3 and ROI #4 as these are the only probes that comprise a free 3'-OH moiety that are able to facilitate a ligation reaction. The 3'-termini of the bound probes in ROI #1 are protected by the still-bound terminator moieties. As shown in FIG. 4M, a "T" nucleotide is added to the spatial barcodes in ROI #2, ROI #3 and ROI #4, further synthesizing the spatial barcodes in those ROI.

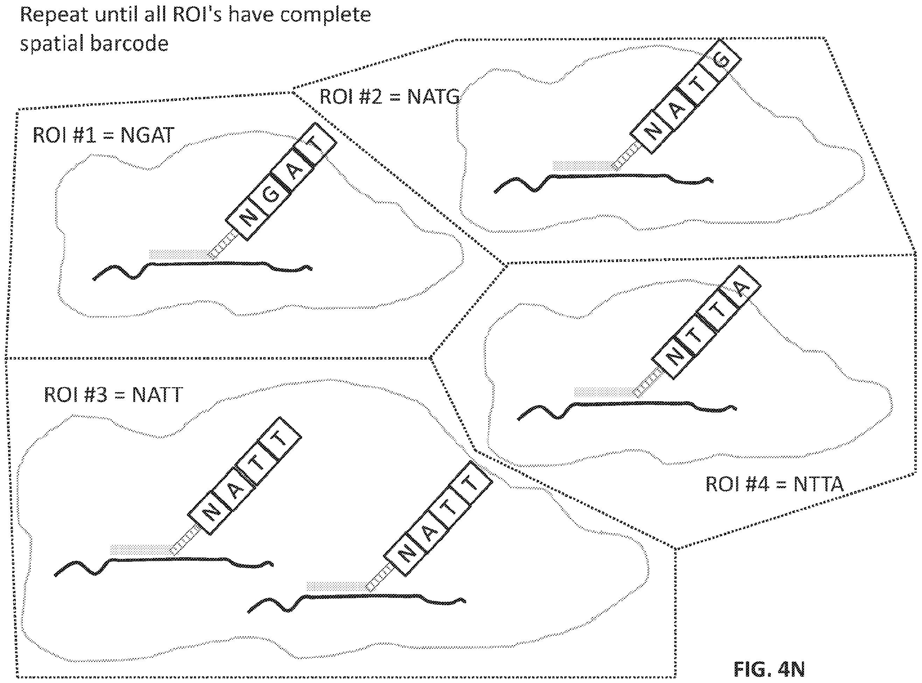

The above-described steps can be repeated any number of times with any of the four nucleotides (dATP, dGTP, dTTP and dCTP) until all of the bound probes in each ROI have complete spatial barcodes, as shown in FIG. 4N. After the completion of all of the spatial barcodes in each ROI, the bound probes can be washed from the sample, collected, and then sequenced. By sequencing the spatial barcode for each collected probe, the ROI from which that specific probe originated can be identified, allowing for the quantification of the abundance of each target mRNA within each ROI, thereby producing a spatially-resolved profile of the abundance of the target mRNA.

The order of nucleotide addition (*i*.*e*. A, then T then G) in FIGs 4A-4N are for exemplary purposes only. Nucleotides can be added to the spatial barcodes in any order and/or combination to synthesize the appropriate spatial barcodes for each ROI.

In some aspects of the methods of the invention and related methods provided for illustrative purposes, a 3' terminator moiety can comprise 2-nitrobenzyl.

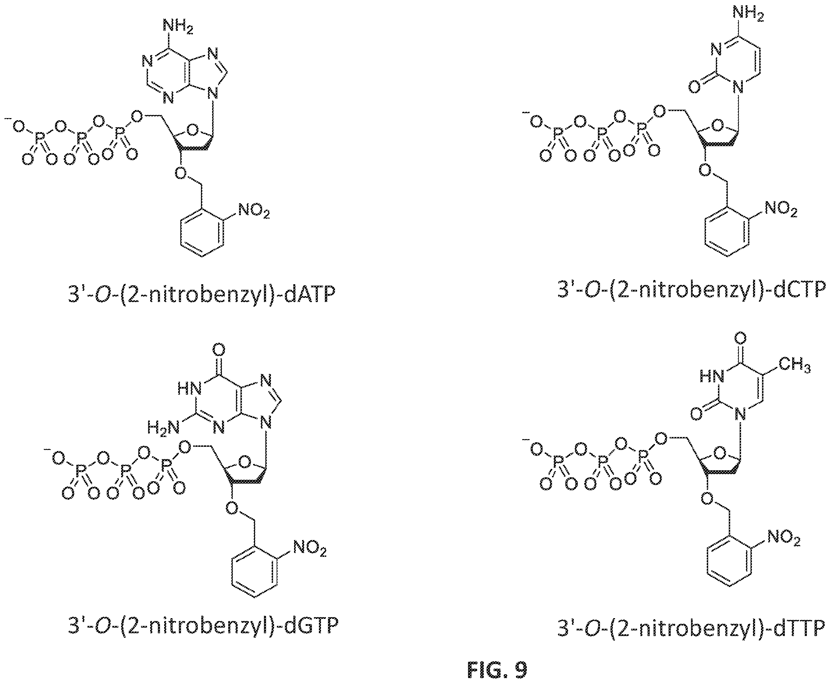

In some aspects , a reversible terminator nucleotide can comprise 3'-*O*-(2-nitrobenzyl)-dATP, 3'-*O*-(2-nitrobenzyl)-dCTP, 3'-*O*-(2-nitrobenzyl)-dGTP or 3'-*O*-(2-nitrobenzyl)-dTTP. The structures of these reversible terminator nucleotide are shown in FIG. 9.

In some aspects, a reversible terminator nucleotide can comprise any of the modified nucleotides described in Wu et al. PNAS, 2007, Vol. 104, No. 42, pgs. 16462-16467.

In some aspects, the methods of the invention can be used to produce a spatially-resolved profile of the abundance of any number of target analytes in at least about 10, or at least about 100, or at least about 1,000, or at least about 10,000, or at least about 100,000, or at least about 1,000,000, or at least about 10⁷, or at least about 10⁸, or at least about 10⁹, or at least about 10¹⁰, or at least about 10¹¹, or at least about 10¹², or at least about 10¹³, or at least about 10¹⁴, or at least about 10¹⁵, or more locations in a tissue sample.

In some aspects, the methods of the invention can be used to produce a spatially-resolved profile of the abundance of at least about three, or at least about four, or at least about five, or at least about six, or at least about seven, or at least about eight, or at least about nine, or at least about ten, or at least about 20, or at least about 30, or at least about 40, or at least about 50, or at least about 60, or at least about 70, or at least about 80, or at least about 90, or at least about 100, or at least about 250, or at least about 500, or at least about 750, or at least about 1000, or at least about 5000, or at least about 10,000, or at least about 100,000 target analytes in any number of locations in a tissue sample.

In some aspects of the methods of the invention and related methods provided for illustrative purposes, contacting a tissue sample with a solution comprising at least two species of probes can comprise contacting a tissue sample with at least about three, or at least about four, or at least about five, or at least about six, or at least about seven, or at least about eight, or at least about nine, or at least about ten, or at least about 20, or at least about 30, or at least about 40, or at least about 50, or at least about 60, or at least about 70, or at least about 80, or at least about 90, or at least about 100, or at least about 250, or at least about 500, or at least about 750, or at least about 1000, or at least about 5000, or at least about 10,000, or at least about 100,000 species of probes, the probes comprising a target-binding domain and a target identification domain, wherein each species of probe comprises a unique target-binding domain that binds to one of the at least two target analytes and a unique target identification domain specific for the target analyte, and a free 3'-OH moiety.

As used herein, the term "spatially detecting" is used in its broadest sense to refer to the identification of the presence of a specific target analyte within a specific region of interest in a sample. Spatially detecting can comprise quantifying the amount of a specific target analyte present within a specific region of interest in a sample. Spatially detecting can further comprise quantifying the relative amount of a first target analyte within a specific region of interest in a sample as compared to the amount of at least a second target analyte within a specific region of interest in a sample. Spatially detecting can also comprise quantifying the relative amount of a specific target analyte within a first region of interest in a sample compared to the amount of the same target analyte in at least a second region of interest in the same sample or different sample.

### Target Analyte

In some aspects of the methods of the invention and the related methods provided for illustrative purposes, a target analyte can be any molecule within a sample that is to be spatially detected. Target analytes include, but are not limited to, nucleic acid molecules and protein molecules. When the target analyte is a protein, the protein can be referred to as a target protein. When the target analyte is a nucleic acid, the nucleic acid can be referred to as a target nucleic acid. Target nucleic acids can include, but are not limited to, mRNA molecules, micro RNA (miRNA) molecules, tRNA molecules, rRNA molecules, gDNA or any other nucleic acid present within a sample.

In some aspects, a target analyte is a target nucleic acid. A target nucleic acid can be DNA or RNA. A target nucleic acid can be a messenger RNA (mRNA). A target nucleic acid can be a microRNA (miRNA).

### Probes

In some aspects of the methods of the invention and the related methods provided for illustrative purposes, a probe can be a polynucleotide. In some aspects, a probe can comprise DNA, RNA or a combination of DNA and RNA. In some aspects, a probe can comprise DNA. In some aspects, a probe can be a single-stranded polynucleotide. In some aspects, a probe can be a double-stranded polynucleotide. In some aspects, a probe can be a partially single-stranded polynucleotide. In some aspects, a probe can be a partially double-stranded polynucleotide.

In some aspects, a probe can comprise a combination of nucleic acids and amino acids. In a non-limiting example, a probe can comprise an antibody operably linked to a polynucleotide, wherein the polynucleotide comprises DNA, RNA or a combination of DNA and RNA.

In some aspects , a probe can comprise a target-binding domain.

The target binding domain can comprise a series of nucleotides (e.g. is a polynucleotide). The target binding domain can comprise DNA, RNA, or a combination thereof. In some aspects, the target binding domain comprises DNA. In some aspects, the target-binding domain is a single-stranded polynucleotide.

In aspects wherein the target binding domain is a polynucleotide, the target binding domain can bind directly or indirectly to a target nucleic acid. A target binding domain can bind directly to a target nucleic acid by hybridizing to a portion of the target nucleic acid that is complementary to the target binding domain of the sequencing probe.

In some aspects, that target-binding domain of a probe can indirectly hybridize to a target nucleic acid present in a sample (via an intermediary oligonucleotide).

The target binding domain of the sequencing probe can be designed to control the likelihood of sequencing probe hybridization and/or de-hybridization and the rates at which these occur. Generally, the lower a probe's Tm, the faster and more likely that the probe will de-hybridize to/from a target nucleic acid. Thus, use of lower Tm probes will decrease the number of probes bound to a target nucleic acid.

The length of a target binding domain, in part, affects the likelihood of a probe hybridizing and remaining hybridized to a target nucleic acid. Generally, the longer (greater number of nucleotides) a target binding domain is, the less likely that a complementary sequence will be present in the target nucleotide. Conversely, the shorter a target binding domain is, the more likely that a complementary sequence will be present in the target nucleotide.

A target binding domain can be any number of nucleotides in length. In some aspects, a target binding domain can be at least about two, or at least about three, or at least about four, or at least about five, or at least about six, or at least about seven, or at least about eight, or at least about nine, or at least about ten, or at least about 11, or at least about 12, or at least about 13, or at least about 14, or at least about 15, or at least about 16, or at least about 17, or at least about 18, or at least about 19, or at least about 20, or at least about 21, or at least about 22, or at least about 23, or at least about 24, or at least about 25, or at least about 26, or at least about 27, or at least about 28, or at least about 29, or at least about 30, or at least about 31, or at least about 32, or at least about 33, or at least about 34, or at least about 35, or at least about 36, or at least about 37, or at least about 38, or at least about 39, or at least about 40, or at least about 41, or at least about 42, or at least about 43, or at least about 44, or at least about 45, or at least about 46, or at least about 47, or at least about 48, or at least about 49, or at least about 50 or more nucleotides in length.

Probes of the present disclosure can be used for spatially detecting a target nucleic acid. In this aspect, the target binding domain can be a target nucleic acid-binding region. The target nucleic acid-binding region is preferably at least 15 nucleotides in length, and more preferably is at least 20 nucleotides in length. In specific aspects, the target nucleic acid-binding region is approximately 10 to 500, 20 to 400, 25, 30 to 300, 35, 40 to 200, or 50 to 100 nucleotides in length. Probes and methods for binding and identifying a target nucleic acid have been described in, *e.g.,* US2003/0013091, US2007/0166708, US2010/0015607, US2010/0261026, US2010/0262374, US2010/0112710, US2010/0047924, and US2014/0371088.

The target binding domain can comprise at least one natural base. The target binding domain can comprise no natural bases. The target binding domain can comprise at least one modified nucleotide or nucleic acid analog. The target binding domain can comprise no modified nucleotides or nucleic acid analogs. The target binding domain can comprise at least one universal base. The target binding domain can comprise no universal bases. The target binding domain can comprise at least one degenerate base. The target binding domain can comprise no degenerate bases.

The target binding domain can comprise any combination natural bases (*e.g.* 0, 1, 2, 3, 4, 5, 6, 7, 8, 9, 10, or more natural bases), modified nucleotides or nucleic acid analogs (*e.g.* 0, 1, 2, 3, 4, 5, 6, 7, 8, 9, 10, or more modified nucleotides or nucleic acid analogs), universal bases (*e.g.* 0, 1, 2, 3, 4, 5, 6, 7, 8, 9, 10, or more universal bases), or degenerate bases (*e.g.* 0, 1, 2, 3, 4, 5, 6, 7, 8, 9, 10, or more degenerative bases). When present in a combination, the natural bases, modified nucleotides or nucleic acid analogs, universal bases and degenerate bases of a particular target binding domain can be arranged in any order.

The terms "modified nucleotides" or "nucleic acid analogues" include, but are not limited to, locked nucleic acids (LNA), bridged nucleic acids (BNA), propyne-modified nucleic acids, zip nucleic acids (ZNA^{®}), isoguanine, isocytosine 6-amino-1-(4-hydroxy-5-hydroxy methyl-tetrahydro-furan-2-yl)-1,5-dihydro-pyrazolo[3,4-d]pyrimidin-4-one (PPG) and 2'-modified nucleic acids such as 2'-O-methyl nucleic acids. The target binding domain can include zero to six (*e.g.* 0, 1, 2, 3, 4, 5 or 6) modified nucleotides or nucleic acid analogues. Preferably, the modified nucleotides or nucleic acid analogues are locked nucleic acids (LNAs).

The term "locked nucleic acids (LNA)" as used herein includes, but is not limited to, a modified RNA nucleotide in which the ribose moiety comprises a methylene bridge connecting the 2' oxygen and the 4' carbon. This methylene bridge locks the ribose in the 3'-endo confirmation, also known as the north confirmation, that is found in A-form RNA duplexes. The term inaccessible RNA can be used interchangeably with LNA. The term "bridged nucleic acids (BNA)" as used herein includes, but is not limited to, modified RNA molecules that comprise a five-membered or six-membered bridged structure with a fixed 3'-endo confirmation, also known as the north confirmation. The bridged structure connects the 2' oxygen of the ribose to the 4' carbon of the ribose. Various different bridge structures are possible containing carbon, nitrogen, and hydrogen atoms. The term "propyne-modified nucleic acids" as used herein includes, but is not limited to, pyrimidines, namely cytosine and thymine/uracil, that comprise a propyne modification at the C5 position of the nucleic acid base. The term "zip nucleic acids (ZNA^{®})" as used herein includes, but is not limited to, oligonucleotides that are conjugated with cationic spermine moieties.

The term "universal base" as used herein includes, but is not limited to, a nucleotide base does not follow Watson-Crick base pair rules but rather can bind to any of the four canonical bases (A, T/U, C, G) located on the target nucleic acid. The term "degenerate base" as used herein includes, but is not limited to, a nucleotide base that does not follow Watson-Crick base pair rules but rather can bind to at least two of the four canonical bases A, T/U, C, G), but not all four. A degenerate base can also be termed a Wobble base; these terms are used interchangeably herein.

In some aspects, the term "degenerate base" can also be used to refer to a specific position on a nucleic acid molecule (*e.g.* an amplification primer, a probe, etc.) that, in the context of a plurality of the nucleic acid molecules, can independently be one or more different nucleotides on different nucleic acid molecules within said plurality. In a non-limiting example, a plurality of 4-nucleotide long nucleic acid molecules may have a degenerate base at the fourth nucleotide position, wherein the degenerate base can be either A, C or G, and wherein the nucleotides at positions 1, 2 and 3 are the same between different nucleic acid molecules. That is, within said plurality, there may be nucleic acid molecules with A at the fourth position, nucleic acid molecules with C at the fourth position, and nucleic acid molecules with G at the fourth position, while the all of the nucleic acid molecules are identical at positions 1, 2 and 3.

In some aspect wherein the target analyte is a target protein, a target binding domain can be a protein-target binding domain. In some aspects, a protein-target binding domain can comprise an antibody or an antibody fragment that binds to the target protein. In some aspects, an antibody or an antigen-binding fragment thereof comprises a Fv, a Fab, a Fab', a Fab'-SH, a F(ab')2, a nanobody, a diabody, a linear antibody, a single-chain antibody molecule, an scFV or a multispecific antibody. In some aspects, a protein-target binding domain can comprise an aptamer that binds to the target protein. In some aspects, a protein-target binding domain can comprise any molecule that is able to specifically bind to a target protein in a biological sample.

In some aspects, a probe can comprise a target identification domain.

A target identification domain is a nucleic acid molecule that identifies the target analyte bound to the target binding domain of a probe. The target identification domain comprises a unique nucleic acid sequence that identifies the target analyte bound to the target binding domain of the probe. In a non-limiting example, a probe with a target binding domain that binds to the protein P53 comprises a target identification domain with a unique nucleic acid sequence that corresponds to P53, while a probe with a target binding domain that binds to the protein P97 comprises a target identification domain with a unique nucleic acid sequence that corresponds to P97.

A target identification domain can be any number of nucleotides in length. In some aspects, a target identification domain can be at least about 12 nucleotides in length. In some aspects, a target identification domain be at least about 7 to at least about 17 nucleotides in length. In some aspects, a target identification domain be at least about 2 to at least about 22 nucleotides in length. In some aspects, a target identification domain can be at least about 2, or at least about 3, or at least about 4, or at least about 5, or at least about 6, or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20, or at least about 21, or at least about 22, or at least about 23, or at least about 24, or at least about 25, or at least about 26, or at least about 27, or at least about 28, or at least about 29, or at least about 30, or at least about 31, or at least about 32, or at least about 33, or at least about 34, or at least about 35, or at least about 36, or at least about 37, or at least about 38, or at least about 39 or at least about 40 nucleotides in length.

In the context of a probe, a target identification domain is a polynucleotide that comprises a nucleic acid sequence that identifies the target analyte bound to the target binding domain of that probe. That is to say, the target identification domain comprises a specific nucleic acid sequence that is *a priori* assigned to the specific target analyte bound to the target binding to which the target identification domain is attached. In a non-limiting example, a probe designated as "probe *X"* designed to spatially detect "target analyte *X"* comprises a target binding domain designated "target binding domain *X"* linked to an target identification domain "target identification domain *X*". Target binding domain *X* binds to target analyte *X* and target identification domain *X* comprises a nucleic acid sequence, designated as "nucleic acid sequence *X*", which corresponds to target analyte *X.* Thus, if a skilled artisan practicing the methods of the present disclosure were to collect probes a region of interest in sample and obtain nucleic acid sequence *X* after sequencing, the skilled artisan would understand that to indicate that target analyte *X* was present in that region of interest. The amount, or number of sequencing reads, of nucleic acid sequence *X* can be used to determine the quantify, in absolute or relative terms, the amount of target analyte *X* within the region of interest.

In some aspects, a probe can comprise a unique molecular identifier (UMI).

In some aspects, a unique molecular identifier can be at least about 9 nucleotides to at least about 19 nucleotides in length. In some aspects, a unique molecular identifier can be at least about 4 nucleotides to at least about 24 nucleotides in length. In some aspects, a unique molecular identifier can be about 14 nucleotides in length. The terms unique molecular identifier and random molecular tags are used interchangeably herein. Using methods known in that art, unique molecular identifiers can be used to correct for biases in amplification prior to sequencing.

In some aspects , a molecular identifier can comprise at least about 5, or at least about 10 nucleotides, or at least about 15, or at least about 20, or at least about 25, or at least about 30, or at least about 35, or at least about 40, or at least about 45, or at least about 50 nucleotides.

In some aspects, a probe can comprise an amplification primer binding site. As used herein, the term "amplification primer binding site" is used in its broadest sense to refer to a nucleic acid sequence that is complementary to, or at least partially complementary to at least one amplification primer, wherein the amplification primer is a short single-stranded or partially single-stranded oligonucleotide that is sufficient to prime DNA and/or RNA synthesis, for example, by PCR.

In some aspects, an amplification primer binding site can comprise at least about 5, at least about 10, at least about 15, at least about 20, at least about 25, at least about 30, at least about 35, at least about 40, at least about 45, at least about 50, at least about 55, at least about 60, at least about 65 or at least about 70 nucleotides.

In some aspects, a probe can comprise a constant region. In some aspects a constant region can comprise a specific nucleic acid sequence that is the same for each location of the tissue sample.

In some aspects, a constant region can be at least about 1, or at least about 2, or at least about 3, or at least about 4, or at least about 5, or at least about 6, or at least about 7, or at least about 8, or at least about 9, or at least about 10, or at least about 11, or at least about 12, or at least about 13, or at least about 14 ,or at least about 15, or at least about 16, or at least about 17, or at least about 18, or at least about 19, or at least about 20, or at least about 21, or at least about 22, or at least about 23, or at least about 24, or at least about 25, or at least about 26, or at least about 27, or at least about 28, or at least about 29, or at least about 30 nucleotides in length.

In some aspects, a constant region can be located between a target identification domain and a free 3'-OH moiety, such that after a spatial barcode is added to the probe *in situ,* the constant region is located between the target identification and the extended spatial barcode. Without wishing to be bound by theory, the constant region can be used during sequence analysis to define the beginning of the spatial barcode domain.

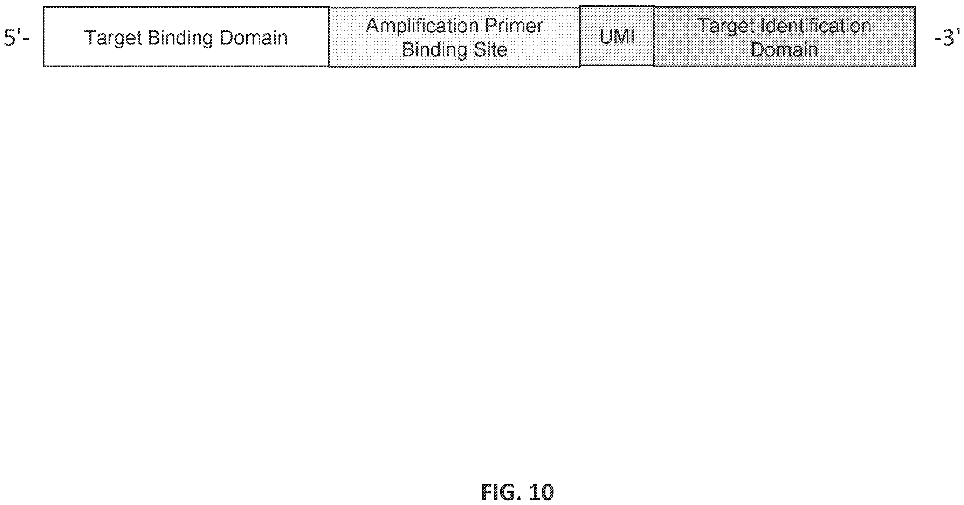

In some aspects, a probe can be a single-stranded polynucleotide comprising, from 5' to 3', a target-binding domain, followed by an amplification primer binding site, followed by a unique molecular identifier, followed by a target identification domain. A schematic of the aforementioned probe is shown in FIG. 10.

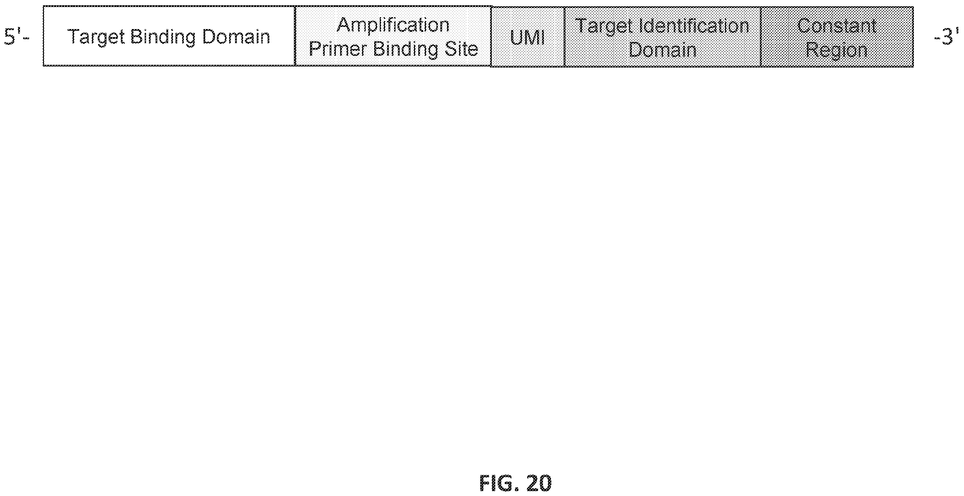

In some aspects, a probe can be a single-stranded polynucleotide comprising, from 5' to 3', a target-binding domain, followed by an amplification primer binding site, followed by a unique molecular identifier, followed by a target identification domain, followed by a constant region. A schematic of the aforementioned probe is shown in FIG. 20.

In some aspects, a probe can be a single-stranded polynucleotide comprising, from 5' to 3', a target binding domain, followed by an amplification primer binding site, followed by a target identification domain, followed by a unique molecular identifier.

In aspects, probes are provided to a sample at concentrations typically less than that used for immunohistochemistry (IHC) or for in situ hybridization (ISH). Alternately, the concentration may be significantly less than that used for IHC or ISH. For example, the probe concentration may be 2 fold less, 5 fold less, 10 fold less, 20 fold less, 25 fold less, 30 fold less, 50 fold less, 60 fold less, 70 fold less, 80 fold less, 90 fold less, 100 fold less, 200 fold less, 300 fold less, 400 fold less, 500 fold less, 600 fold less, 700 fold less, 800 fold less, 900 fold less, 1000 fold less, 2000 fold less, or less and any number in between. In aspects, probes are provided at a concentration of 100 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 0.9 nM, 0.8 nM, 0.7 nM, 0.6 nM, 0.5 nM, 0.4 nM, 0.3 nM, 0.2 nM, 0.1 nM, 0.09 nM, 0.08 nM, 0.07 nM, 0.06 nM, 0.05 nM, 0.04 nM, 0.03 nM, 0.02 nM, 0.01 nM, and less and any concentration in between.

Background noise, during protein detection, can be reduced by performing a negative purification of the intact probe molecule. This can be done by conducting an affinity purification of the probe after collection of eluate from a region of interest.

In some aspects, at least one, or at least two, or at least three, or at least four, or at least five, or at least six, or at least seven, or at least eight, or at least nine, or at least ten, or at least eleven, or at least twelve, or at least thirteen, or at least fourteen, or at least fifteen, or at least sixteen, or at least seventeen, or at least eighteen, or at least nineteen, or at least twenty, or at least thirty, or at least forty, or at least fifty, or at least sixty, or at least seventy, or at least eighty, or at least ninety, or at least one hundred probes can bind to a single target analyte. As used herein, the term "tiling" is used to describe when more than one probe is bound to a target analyte.

A set of probes, a plurality of probes, a solution comprising a plurality of probes can include at least one species of probes, *i.e.,* directed to one target. A set of probes preferably includes at least two, e.g., 3, 4, 5, 6, 7, 8, 9, 10, 11, 12, 13, 14, 15, 16, 17, 18, 19 20, 30, 40, 50, 60, 70, 80, 90, 100, 200, 300, 400, 500, 600, 700, 800, 900, 1000 or more species of probes. A probe set may include one or multiple copies of each species of probe.

A first set of, a first plurality of or a first solution comprising probes only may be applied to a sample. Alternately, a second set (or higher number) of probes may be later applied to the sample. The first set and second (or higher number) may target only nucleic acids, only proteins, or a combination thereof.

Two or more targets (*i.e.,* proteins, nucleic acids, or a combination thereof) are detected; 3, 4, 5, 6, 7, 8, 9, 10, 11, 12, 13, 14, 15, 16, 17, 18, 19 20, 30, 40, 50, 60, 70, 80, 90, 100, 200, 300, 400, 500, 600, 700, 800, 900, 1000 or more targets, and any number there between, are detected.

A set of probes may be pre-defined based upon the cell type or tissue type to be targeted. For example, if the tissue is a breast cancer, then the set of probes will include probes directed to proteins relevant to breast cancer cells (e.g., Her2, EGFR, and PR) and/or probes directed to proteins relevant to normal breast tissues. Additionally, the set of probes may be pre-defined based upon developmental status of a cell or tissue to be targeted. Alternately, the set of probes may be pre-defined based upon subcellular localizations of interest, e.g., nucleus, cytoplasm, and membrane. For example, antibodies directed to Foxp3, Histone H3, or P-S6 label the nucleus, antibodies directed to CD3, CD4, PD-1, or CD45RO label the cytoplasm, and antibodies directed to PD-L1 label membranes.

### Spatial Barcode Domains

In some aspects of the methods of the invention and related methods provided for illustrative purposes, a spatial barcode domain can comprise a spatial identifier sequence, wherein the spatial identifier sequence comprises a nucleic acid sequence that is unique to a specific location/region of interest within a tissue sample. In this way, when a probe on which a spatial barcode has been extended is collected and sequenced, the user can use the spatial identifier sequence of the spatial barcode domain to determine in what location/region of interest of the tissue sample the probe in when it was bound to its corresponding target analyte.

In some aspects, a spatial identifier sequence can comprise at least about 1, or at least about 2, or at least about 3, or at least about 4, or at least about 5, or at least about 6, or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20 nucleotides in length, or at least about 21, or at least about 22, or at least about 23, or at least about 24, or at least about 25, or at least about 26, or at least about 27, or at least about 28, or at least about 29, or at least about 30, or at least about 31, or at least about 32, or at least about 33, or at least about 34, or at least about 35, or at least about 36, or at least about 37, or at least about 38, or at least about 39 or at least about 40 nucleotides. In some aspects, a spatial identifier sequence can comprise at least about 20 nucleotides.

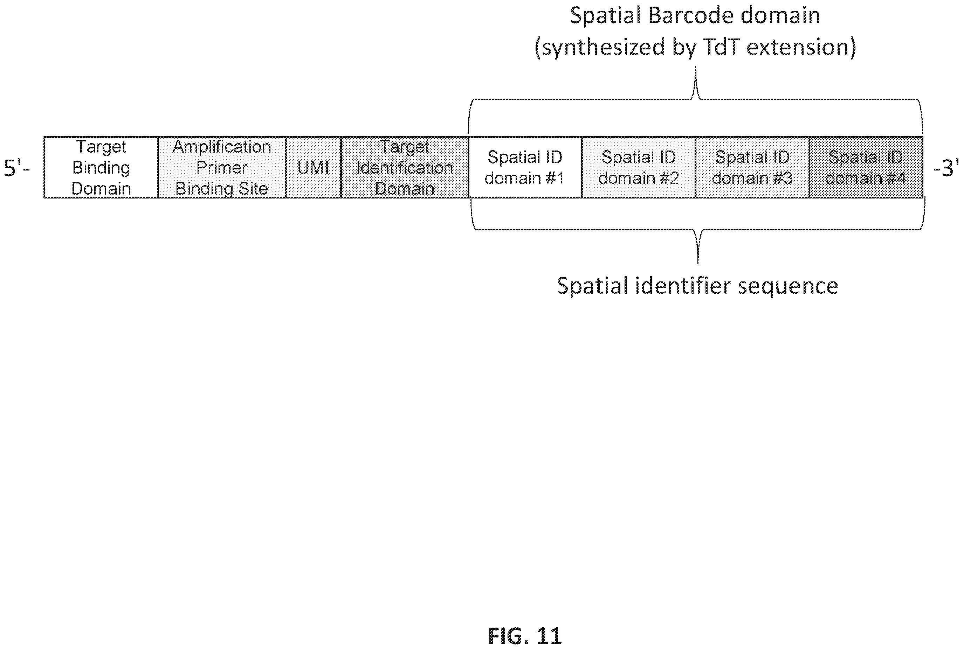

In some aspects, a spatial identifier sequence can comprise at least one, at least two, at least three, at least four, at least five, at least six, at least seven, at least eight, at least nine, at least 10, at least 11, at least 12, at least 13, at least 14, at least 15, at least 16, at least 17, at least 18, at least 19 or at least 20 spatial identification domains. FIG. 11 is schematic of a probe comprising a target binding domain, an amplification primer binding site, a unique molecular identifier and a target identification domain, along with a spatial barcode domain that has been synthesized by TdT extension in the methods of the present disclosure. In this non-limiting example, the spatial barcode domain comprises a spatial identifier sequence, wherein the spatial identifier sequence comprises four (#1, #2, #3 and #4) spatial identification domains.

A spatial identification domain can comprise at least about one, or at least about two, or at least about three, or at least about four, or at least about five, or at least about six, or at least about seven, or at least about eight, or at least about nine, or at least about ten, or at least about 11, or at least about 12, or at least about 13, or at least about 14, or at least about 15, or at least about 16 or at least about 17, or at least about 18, or at least about 19, or at least about 20 nucleotides. In some aspects, and spatial identification domain can comprise

In some aspects, an individual spatial identification domain within a single spatial barcode domain can comprise the same number or a different number of nucleotides as compared to another individual spatial identification domain within that same single spatial barcode domain. In a non-limiting example, a spatial barcode domain may comprise four spatial identification domains, a first spatial identification domain, a second spatial identification domain, a third spatial identification domain and a fourth spatial identification domain. The first spatial identification domain may comprise two nucleotides, a second spatial identification domain may comprise three nucleotides, the third spatial identification domain may comprise two nucleotides and the fourth spatial identification domain may comprise four nucleotides. Thus, in this non-limiting example, the first spatial identification domain comprises the same number of nucleotides as the third spatial identification domain, but a different number of nucleotides as compared to the second spatial identification domain and the fourth spatial identification domain.

In some aspects, each spatial identification domain within a single spatial barcode may comprise the same nucleotide at the 3'-terminus. In a non-limiting example, a spatial barcode domain may comprise four spatial identification domains, a first spatial identification domain, a second spatial identification domain, a third spatial identification domain, and fourth spatial identification domain. In this non-limiting example, each spatial identification domain comprises a "C" nucleotide at the 3' terminus. Thus, the first spatial identification domain may comprise the nucleotide sequence 5'-ATC-3', the second spatial identification domain may comprise the nucleotide sequence 5'-TGC-3', the third spatial identification domain may comprise the nucleotide sequence 5'-TAC-3' and the fourth spatial identification domain may comprise the nucleotide sequence 5'-AAC-3'. Note in this non-limiting example each spatial identification domain in the spatial barcode comprises the same number of nucleotides.

In some aspects, each spatial identification domain within any spatial barcode present within a sample may comprise the same nucleotide at the 3'-terminus.

In another non limiting example, a spatial barcode domain may comprise four spatial identification domains, a first spatial identification domain, a second spatial identification domain, a third spatial identification domain, and fourth spatial identification domain. In this non-limiting example, each spatial identification domain comprises a "C" nucleotide at the 3' terminus. Thus, the first spatial identification domain may comprise the nucleotide sequence 5'-AC-3', the second spatial identification domain may comprise the nucleotide sequence 5'-GTC-3', the third spatial identification domain may comprise the nucleotide sequence 5'-TATC-3' and the fourth spatial identification domain may comprise the nucleotide sequence 5'-AAC-3'. Note in this non-limiting example the spatial identification domains in the spatial barcode comprises differing number of nucleotides (the first spatial identification domain has two nucleotides, the second spatial identification domain has three nucleotides, the third spatial identification domain has four nucleotides and the fourth spatial identification domain has three nucleotides) and each spatial identification domain has a "C" nucleotide at the 3' terminus.

Without wishing to be bound by theory, whether each spatial identification domain within a spatial barcode has the same number of nucleotides or a differing number of nucleotides can determine the number of extension cycles that are needed and the number of different spatial barcodes that can be constructed, and consequently, the number of regions of interest that can be analyzed on a single tissue sample. For example, a spatial barcode domain may comprise 10 or 11 spatial identification domains, wherein each spatial identification domain comprises 1 nucleotide. This would allow for the creation of 4¹⁰ to 4¹¹ unique spatial barcode sequences, meaning that 4¹⁰ to 4¹¹ different regions of interest could be analyzed on a single tissue sample. In this configuration, 40-44 extensions cycles would be needed in order to construct all of the barcodes on the tissue sample. In another example, approximately 2.1 million unique spatial barcodes could be constructed if spatial identification domains could have differing numbers of nucleotides ranging from 1 nucleotide to 4 nucleotides. In this configuration, the spatial barcodes would be 7 to 28 nucleotides in total length, and only 28 extension cycles would be needed to construct all of the barcodes.

The spatial barcode domain corresponding a first location in a tissue sample may have more than, less than, or the same number of spatial identification domains as a spatial barcode domain corresponding to a second location in a tissue sample.

A spatial barcode domain can comprise a nucleic acid sequence that is specific and unique to a single location/region of interest in a tissue sample. For example, the combined nucleic acid sequence of all of the spatial identification domains within a spatial barcode domain can be specific and unique to a single location/region of interest in a tissue sample. In this way, when a probe on which a spatial barcode has been extended is collected and sequenced, the user can use the sequence of the spatial barcode domain to determine in what location/region of interest of the tissue sample the probe in when it was bound to its corresponding target analyte.

A spatial identification domain can comprise at least about one, or at least about two, or at least about three, or at least about four, or at least about five, or at least about six, or at least about seven, or at least about eight, or at least about nine, or at least about ten, or at least about 11, or at least about 12, or at least about 13, or at least about 14, or at least about 15, or at least about 16 or at least about 17, or at least about 18, or at least about 19, or at least about 20 nucleotides. In some aspects, and spatial identification domain can comprise

In some aspects of the methods of the invention, the methods can further comprise, after the spatial barcode domains are synthesized in each location of the tissue sample (*e.g.* step (e)), repeating steps (c) and (d) to extend the spatial barcode domain in each location of the tissue sample such that the spatial barcode domain comprises, at the 3' end, a delimiting domain.

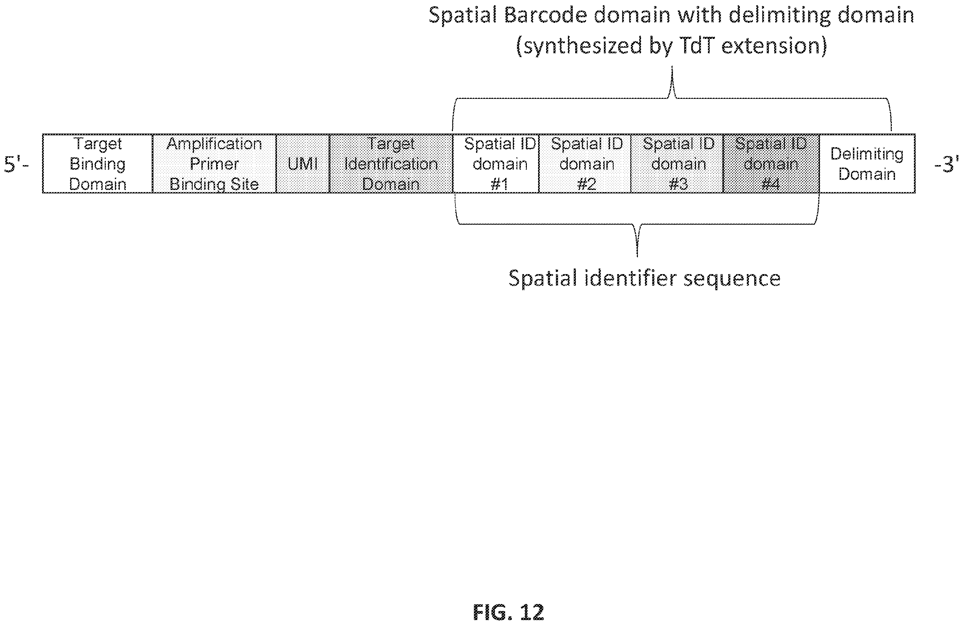

A delimiting domain can comprise a specific nucleic acid sequence that is the same for each location of the tissue sample. A delimiting domain can be at least 1, or at least 2, or at least 3, or at least 4, or at least 5, or at least 6, or at least 7, or at least 8, or at least 9, or at least 10, or at least 11 or at least 12, or at least 13, or at least 14, or at least 15, or at least 16, or at least 17, or at least 18, or at least 19, or at least 20 nucleotides in length. A delimiting domain can comprise any nucleic acid sequence. In a non-limiting example, the delimiting domain can comprise the nucleic acid sequence "TCTC". FIG. 12 is a schematic of the probe of FIG. 11, wherein the spatial barcode domain has been further extended by TdT synthesis to include a delimiting domain.

Without wishing to be bound by theory, the sequence of a delimiting domain can be used during sequencing analysis to define the end of the spatial barcode domain, thereby aiding in the quantification via sequencing of the collected probes.

In some aspects of the methods of the invention, the methods can further comprise, after the spatial barcode domains are synthesized in each location of the tissue sample (*e.g.* step (e)), extending the spatial barcode domain in each location of the tissue sample such that the spatial barcode domain comprises a polyT domain or other amplification primer binding site.

In some aspects, the methods can further comprise after the spatial barcode domains are synthesized in each location of the tissue sample (*e.g.* step (e)): (i) repeating steps (c) and (d) to extend the spatial barcode domain in each location of the tissue sample such that the spatial barcode domain comprises, a delimiting domain: and (ii) extending the spatial barcode domain in each location of the tissue sample such that the spatial barcode domain comprises a polyT domain.

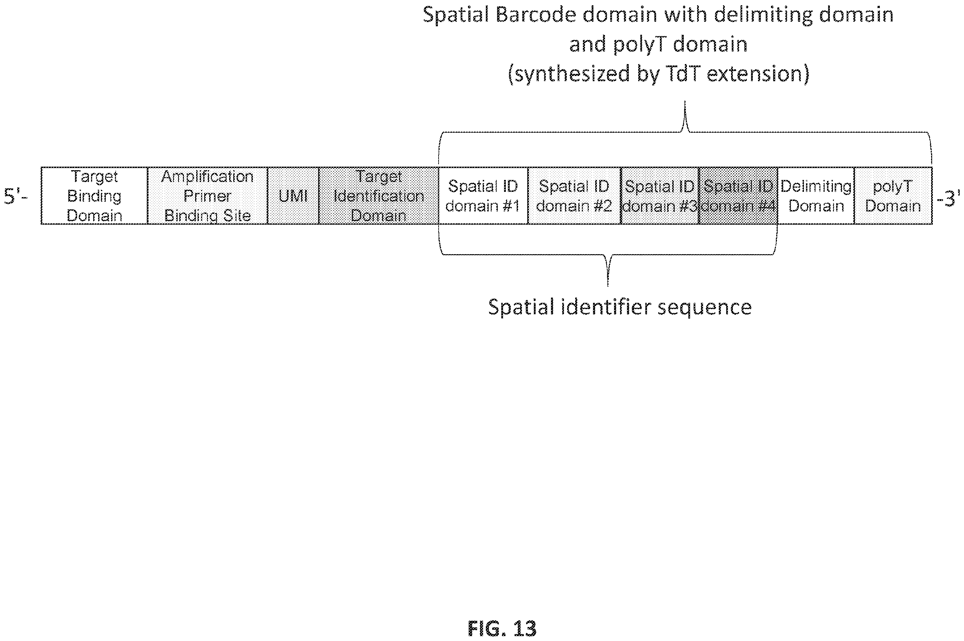

FIG. 13 is a schematic of the probe of FIG. 11, wherein the spatial barcode domain has been further extended by TdT synthesis to include a delimiting domain and then further extended by TdT synthesis to include a polyT domain.

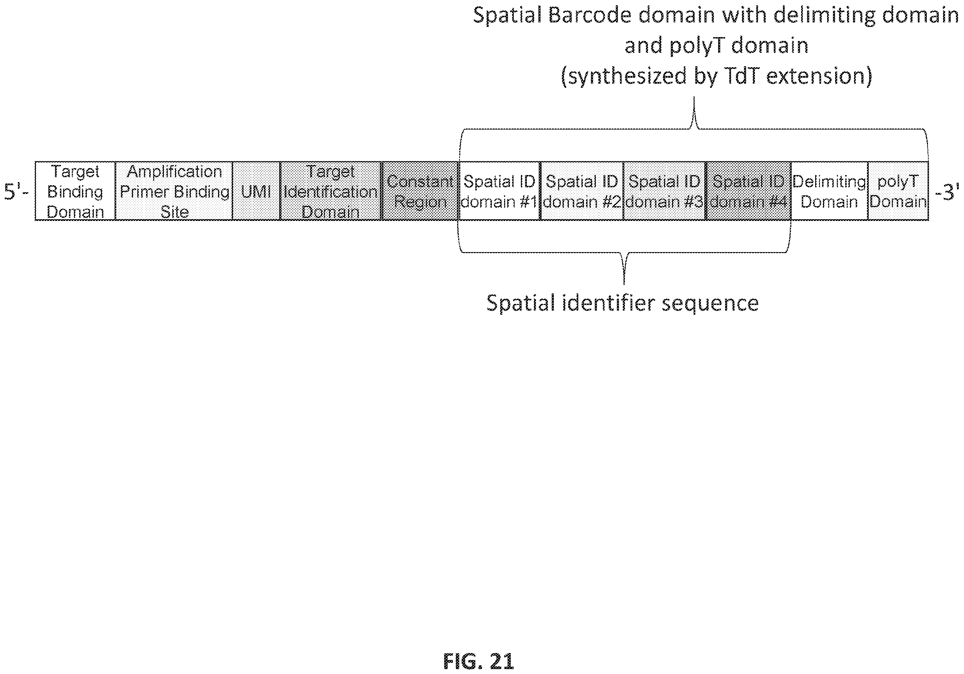

FIG. 21 is a schematic of the probe of FIG. 20, wherein the probe has been extended by TdT synthesis in the methods of the present disclosure to include a spatial barcode domain, wherein the spatial barcode domain comprises four spatial identification domains, a delimiting domain and a polyT domain.

In some aspects, a polyT domain is a polynucleotide that is comprised of only T nucleotides. In some aspects, the polyT domain can comprise at least about 1, or at least about 2, or at least about 3, or at least about 4, or at least about 5, or at least about 6, or at least about 7, or at least about 8, or at least about 9, or at least about 10, or at least about 11 or at least about 12, or at least about 13, or at least about 14, or at least about 15, or at least about 16, or at least about 17, or at least about 18, or at least about 19, or at least about 20 nucleotides, or at least about 25, or at least about 30, or at least about 35, or at least about 40, or at least about 50, or at least about 55, or at least about 60, or at least about 65, or at least about 70, or at least about 75, or at least about 80, or at least about 85, or at least about 90, or at least about 95, or at least about 100, or at least about 125, or at least about 150, or at least about 175, or at least about 200, or at least about 225, or at least about 250, or at least about 275, or at least about 300, or at least about 350, or at least about 400, or at least about 450, or at least about 500, or at least about 2000 T nucleotides. In some aspects, a polyT domain comprises more than 20 but less than 500 T nucleotides. In some aspects, a polyT domain comprises more than 20 but less than 2000 T nucleotides.

In some aspects, a polyT domain of a spatial barcode domain of a first bound probe can the same length or a different length than a polyT domain of a spatial barcode domain of a second bound probe.

In some aspects, extending the spatial barcode domain in each location of the tissue sample such that the spatial barcode domain comprises a polyT domain can comprise contacting the sample with a solution comprising dTTP and TdT.

### Amplification Methods

In some aspects of the methods of the invention, quantifying via sequence can comprise amplifying the probes collected from the tissue sample.

In some aspects, amplifying the probes collected from the tissue sample can comprise amplifying the collected probes using a first amplification primer and a second amplification. In some aspects amplifying the probes collected from the tissue sample can comprise contacting the collected probes with a first amplification primer and a second amplification primer, wherein the first amplification primer and the second amplification primer hybridize to the collected probes. In some aspects, amplifying the probes collected from the tissue sample can comprise amplification reactions known in the art, including, but not limited to PCR.

In some aspects, an amplification can comprise at least one NGS index sequence. In some aspects, the NGS index sequence is an i5 index sequence or an i7 index sequence. In some aspects, the NGS index sequence can be any index sequence known in the art.

In some aspects, an NGS index sequence can be at least about 1, or at least about 2, or at least about 3, or at least about 4, or at least about 5, or at least about 6, or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20 nucleotides in length. In some aspects, an NGS index sequence is at least about 8 nucleotides in length. In some aspects, an NGS index sequence is at least about 10 nucleotides in length.

In some aspects, an amplification primer can comprise an i5 index sequence, wherein the i5 sequence comprises the sequence set forth in any one of SEQ ID NOs: 8-13 and 20-10,404, or the reverse complement thereof.

**Table 1. Exemplary i5 index sequences**

| **Sequence** | **SEQ ID NO:** |
|---|---|
| GAATGCACGA | 8 |
| AAGACTATAG | 9 |
| TCGGCAGCAA | 10 |
| CTAATGATGG | 11 |
| GGTTGCCTCT | 12 |
| CGCACATGGC | 13 |

In some aspects, an amplification primer can comprise an i7 index sequence, wherein the i7 sequence comprises the sequence set forth in any one of SEQ ID NO: 14-10,404, or the reverse complement thereof.

**Table 2. Exemplary i7 index sequences**

| **Sequence** | **SEQ ID NO:** |
|---|---|
| CGGCAATGGA | 14 |
| GCCGTAACCG | 15 |
| AACCATTCTC | 16 |
| GGTTGCCTCT | 17 |
| CTAATGATGG | 18 |
| TCGGCCTATC | 19 |

In some aspects, an amplification primer can comprise a sequencing primer binding site. In some aspects, a sequencing primer binding site can be at least about 1, or at least about 2, or at least about 3, or at least about 4, or at least about 5, or at least about 6, or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20 nucleotides in length, or at least about 21, or at least about 22, or at least about 23, or at least about 24, or at least about 25, or at least about 26, or at least about 27, or at least about 28, or at least about 29, or at least about 30, or at least about 31, or at least about 32, or at least about 33, or at least about 34, or at least about 35, or at least about 36, or at least about 37, or at least about 38, or at least about 39 or at least about 40 nucleotides in length.

In some aspects, a sequencing primer binding site is at least about 9 nucleotides in length. In some aspects, a sequencing primer binding site is at least about 34 nucleotides in length.

In some aspects, an amplification primer can comprise a flow cell adapter sequence, wherein the flow cell adapter sequence is suitable for sequencing. At least one amplification primer can comprise a P5 flow cell adapter sequence, wherein the P5 flow cell adapter sequence comprises the sequence set forth in SEQ ID NO: 10,405, or the reverse complement thereof. At least one amplification primer can comprises a P7 flow cell adapter sequence, wherein the P7 flow cell adapter sequence comprises the sequence set forth in SEQ ID NO: 10,406, or the reverse complement thereof.

In some aspects, a flow cell adapter sequence can comprise between about 15 to about 40 nucleotides. A flow cell adapter sequence can comprise about 29 nucleotides. A flow cell adapter sequence can comprise about 24 nucleotides.

In some aspects , a flow cell adapter sequence suitable for sequencing can comprise at least about 5, at least about 10, at least about 15, at least about 20, at least about 25, at least about 30, at least about 35 at least about 40, at least about 45, at least about 50, at least about 55, at least about 60, at least about 65, at least about 70, at least about 75, at least about 80, at least about 85, at least about 90, at least about 95 or at least about 100 nucleotides.

In some aspects, an amplification primer can comprise a polyA domain. In some aspects, a polyA domain is a polynucleotide that is comprised of only A nucleotides. In some aspects, the polyA domain can comprise at least about 1, or at least about 2, or at least about 3, or at least about 4, or at least about 5, or at least about 6, or at least about 7, or at least about 8, or at least about 9, or at least about 10, or at least about 11 or at least about 12, or at least about 13, or at least about 14, or at least about 15, or at least about 16, or at least about 17, or at least about 18, or at least about 19, or at least about 20 nucleotides, or at least about 25, or at least about 30, or at least about 35, or at least about 40 A nucleotides. In some aspects, a polyA domain can comprise at last about 32 nucleotides. In some aspects, a polyA domain can be used to hybridize a sequence probe to an extended spatial barcode domain by hybridizing the polyA domain to a polyT domain.

In some aspects, an amplification primer can comprise a degenerate base. In some aspects, an amplification primer can comprise at least about two degenerate bases. In some aspects, an amplification primer can comprise at least about three, or at least about four, or at least about five, or at least about six, or at least about seven, or at least about eight, or at least about nine, or at least about ten degenerate bases. In some aspects, the degenerate base(s) can be located at the 3' end of an amplification primer. In some aspects, in the context of a plurality of amplification primers, a degenerate base position may have an A, a T, a C or a G nucleotide within the plurality. In some aspects, in the context of a plurality of amplification primers, a degenerate base position may have an A, a C or a G nucleotide.

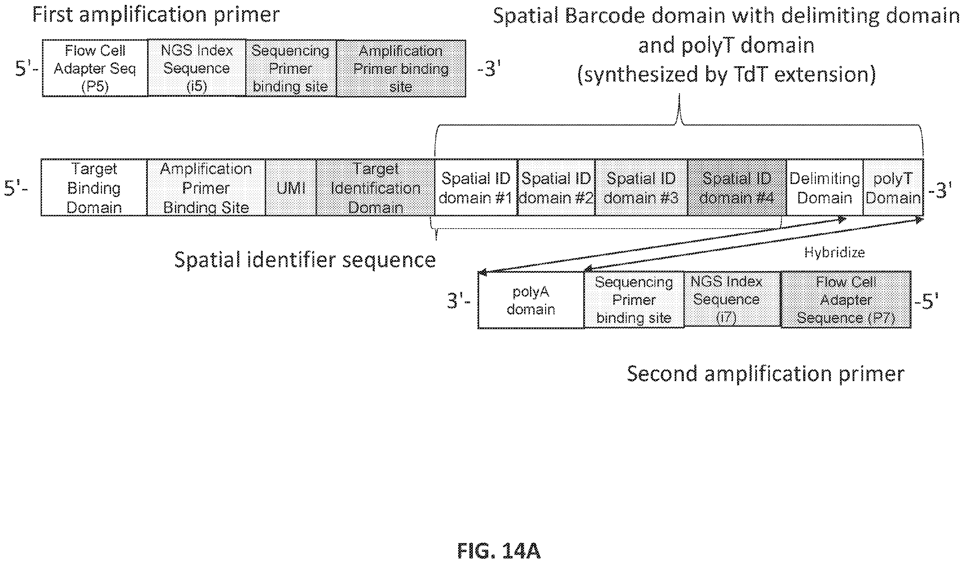

FIG. 14A is an exemplary schematic of a method of amplifying the probes collected from the tissue sample as part of quantifying the probes via sequencing. In this non-limiting example, the probe has a target binding domain, followed by an amplification primer binding site, followed by a unique molecular identifier, followed by a target identification domain. In this non-limiting example, a spatial barcode has been synthesized and extended on the 3' end of the probe. The spatial barcode domain has been synthesized by TdT extension and comprises a spatial identifier sequence comprising four (#1, #2, #3 and #4) spatial identification domains, a delimiting domain and a polyT domain. In this non-limiting example, a first amplification primer has a flow cell adapter sequence (P5), followed by an NGS index sequence (i5), followed by a sequencing primer binding site, followed by a sequence that matches the amplification primer bindings site on the probe. In this non-limiting example, a second amplification primer has a polyA domain, followed by a sequencing primer binding site, followed by an NGS index sequence (i7), followed by a Flow Cell Adapter sequence (P7). The amplification primers can be hybridized to the probes as shown and used to amplify the probe using standard techniques, including, but not limited to, PCR, as would be appreciated by the skilled artisan.

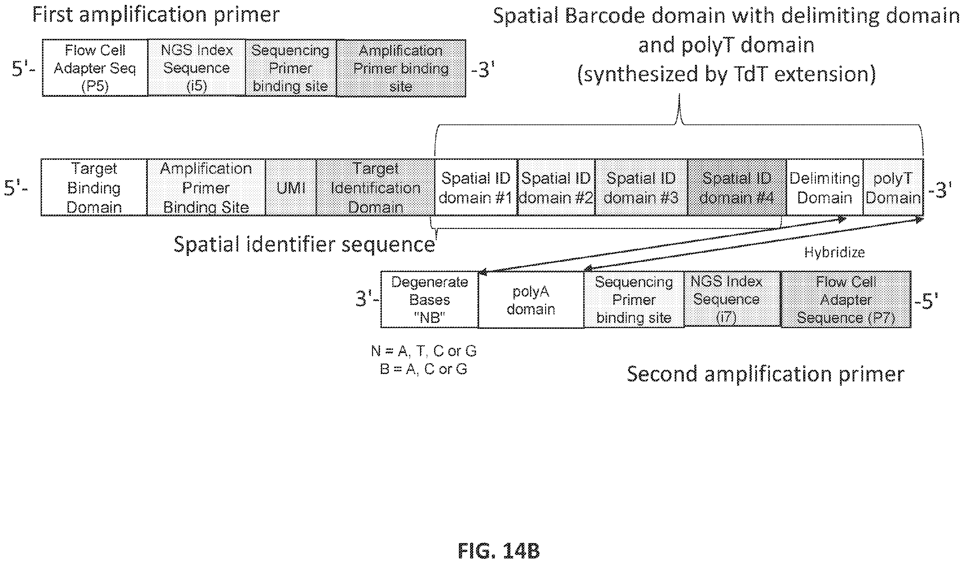

FIG. 14B is an exemplary schematic of a method of amplifying the probes collected from the tissue sample as part of quantifying the probes via sequencing. In this non-limiting example, the probe has a target binding domain, followed by an amplification primer binding site, followed by a unique molecular identifier, followed by a target identification domain. In this non-limiting example, a spatial barcode has been synthesized and extended on the 3' end of the probe. The spatial barcode domain has been synthesized by TdT extension and comprises a spatial identifier sequence comprising four (#1, #2, #3 and #4) spatial identification domains, a delimiting domain and a polyT domain. In this non-limiting example, a first amplification primer has a flow cell adapter sequence (P5), followed by an NGS index sequence (i5), followed by a sequencing primer binding site, followed by a sequence that matches the amplification primer bindings site on the probe. In this non-limiting example, a second amplification primer has two degenerate bases (3'-NB-5', wherein N is A, T, C or G and B is A, C or G), followed by a polyA domain, followed by a sequencing primer binding site, followed by an NGS index sequence (i7), followed by a Flow Cell Adapter sequence (P7). The degenerate bases are located at the 3'-terminus of the second amplification primer. The amplification primers can be hybridized to the probes as shown and used to amplify the probe using standard techniques, including, but not limited to, PCR, as would be appreciated by the skilled artisan.

In some aspects, an amplification primer can comprise an affinity moiety. An affinity moiety can include, but is not limited to, biotin. In aspects wherein an amplification primer comprises an affinity moiety, the affinity moiety can be used to further purify the amplified probes using a reagent that specifically binds to the affinity moiety. In some aspects wherein the affinity moiety is biotin, streptavidin-coated beads can be used to purify the amplified probes.

### Illumination Methods

In some aspects of the methods of the invention and related methods provided for illustrative purposes, illuminating a location of a tissue sample can comprise illuminating the location using a two-photon excitation method. In some aspects, the two-photon excitation method is a two-photon excitation method using light that has a near-infrared (NIR) wavelength. In some aspects, a NIR wavelength can be about 700 to about 1000 nm. Without wishing to be bound by theory, two-photo excitation allows for improved spatial confinement and minimized excitation crosstalk as compared with one-photon excitation methods, including one-photon excitation methods at UV (300-405 nm) wavelengths. Thus, spatial barcodes can be synthesized in smaller, more defined regions of the tissue sample, increasing the spatial resolution and accuracy of the methods provided herein.

In some aspects, a two-photon excitation method can be a patterned two-photon illumination method. A patterned two-photon illumination method can comprise raster scanning a point of excitation light over a sample. This raster scanning can be achieved through the use of galvanometric mirrors, micro-electro-mechanical systems (MEMS) mirrors, acousto-optic deflectors or any combination thereof. A patterned two-photon illumination method can comprise rapidly modulating a laser on and off on a per-pixel basis. Such modulating can be achieved through the use of an acousto-optic modulator, a Pockels cell or any combination thereof. In some aspects, a patterned two-photon illumination method can comprise raster scanning a point of excitation light over a sample and rapidly modulating a laser on and off on a per-pixel basis.

Exemplary optical schematics of a two-photon excitation method is shown in FIGS. 5A and 5B.

In some aspects, a two-photon excitation method can comprise the use of erbium fiber laser. In some aspects, an erbium fiber laser can emit light at a wavelength of about 1550 nm. In some aspects, the light from an erbium fiber laser can be frequency doubled to about 775 nm with pulse widths less than 100 femtoseconds.

In some aspects, a two-photon excitation method can comprise the use of a 780 nm fiber-based femtosecond laser. In some aspects, the repetition rate of the laser can be specified from 10 to 80 MHz with clean pulse widths of less than 90 femtoseconds. In some aspects, the beam quality, M² factor, can be less than 1.2. In some aspects, the beam diameter can be about 1.57 mm. In some aspects, the beam circularity can be at least 94%.

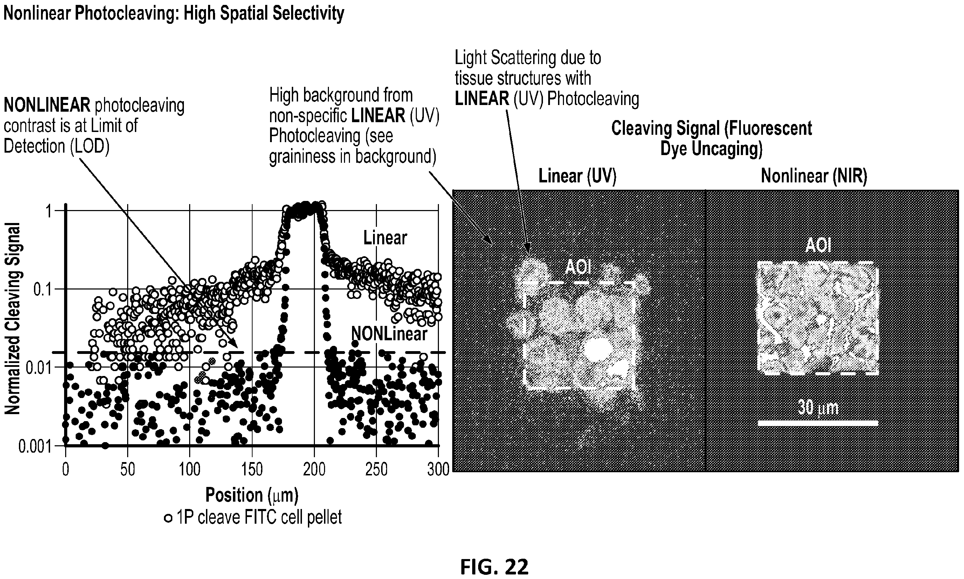

The application of two photon illumination in the present invention resulted in three surprising improvements in the ability to detect and quantify protein and/or nucleic acid expression in a user-defined region of a tissue, user-defined cell, and/or user-defined subcellular structure within a cell. First, two photon illumination as used in the present invention provides dramatic improvements in Z and modest improvements in X-Y resolution. Second, with two photon illumination of the background in the whole field of view away from the features is also lower (see FIG. 22). Third and perhaps most significant, with one-photon UV illumination, there are unexpected diffraction, scattering and/or light piping phenomena caused by cellular structures. These artifacts can significantly degrade the ability to focus UV light below diffraction expectations. These tissue artifacts are not present with two photon illumination as used in the present invention.

### Locations or Regions of Interest

As used herein, the terms "location of a tissue sample" and a "region of interest" are used interchangeably to refer to a specific and defined area of the tissue sample within which the abundance of one or more target analytes will be quantified.

In some aspects of the methods of the invention, a region of interest can be no less than about 10 nm in the x and/or y direction, no less than about 100 nm in the x and/or y direction, or no less than about 400 nm in the x and/or y direction. In some aspects, an illuminated location of a tissue sample can be no less than about 10 nm in the x and/or y direction, no less than about 100 nm in the x and/or y direction, or no less than about 400 nm in the x and/or y direction.

In some aspects, a region of interest can be no less than about 10 nm in the z direction, no less than about 500 nm in the z direction, or no less than about 1500 nm in the z direction. In some aspects, an illuminated location of a tissue sample can be no more than about 1500 nm in the z direction.

A region of interest may be a tissue type present in a sample, a cell type, a cell, or a subcellular structure within a cell.

Together, a comparison of the identity and abundance of the target proteins and/or target nucleic acids present in a first region of interest (*e.g.,* tissue type, a cell type (including normal and abnormal cells), and a subcellular structure within a cell) and the identity and abundance of the target proteins and/or target nucleic acids present in second region of interest or more regions of interest can be made using the methods of the invention.

In some aspects, a region of interest may comprise a single cell. In some aspects, a region of interest may include a plurality of cells, such as, but not limited to, no more than two cells, no more than three cells, no more than four cells, no more than five cells, no more than six cells, no more than seven cells, no more than eight cells, no more than nine cells or no more than ten cells.

In some aspects, a region of interest may comprise a subcellular structure within a single cell.

In some aspects, an area of the tissue sample may be manually selected by a user and each cell within the area would be automatically identified as a region of interest and encoded. Cells can be identified by specific staining, and software used to locate the boundaries of the cell. Thus, the methods and systems described herein can encode each cell differently with minimal user interaction.

### Probe Collection

In some aspects of the methods of the invention and related methods provided for illustrative purposes, the step of collecting the probes bound to target analytes in the tissue sample can comprise scraping the tissue sample, digesting the tissue sample with proteinase K or a combination thereof. In some aspects, a proteinase K digestion can be performed at about 56°C for at least about one hour, followed by a further digestion at about 90°C for at least about 15 minutes.

In some aspects, the step of collecting the probes bound to target analytes in the tissue sample can comprise an Ampure SPRI purification, as would be appreciated by the skilled artisan.

In some aspects, the step of collecting the probes bound to target analytes in the tissue sample can comprise an xGen hybridization capture-based purification, as would be appreciated by the skilled artisan.

In some aspects, an xGen hybridization capture-based purification comprises hybridizing at least one at least one capture probe to the probes bound to target analytes in the tissue sample.

In some aspects, a capture probe can comprise and affinity moiety. An affinity moiety can be biotin.

In some aspects, a capture probe can comprise at least one copy of a nucleic acid sequence that is complementary to a probe of the present disclosure. In some aspects, a capture probe can comprise at least about two copies of a nucleic acid sequence that is complementary to a probe of the present disclosure. In some aspects, a capture probe can comprise at least about three, or at least about four, or at least about five, or at least about six, or at least about seven, or at least about eight, or at least about nine, or at least about ten copies of a nucleic acid sequence that is complementary to a probe of the present disclosure. The nucleic acid sequence that is complementary to a probe of the present disclosure can be complementary to an amplification primer binding site located on the probe.

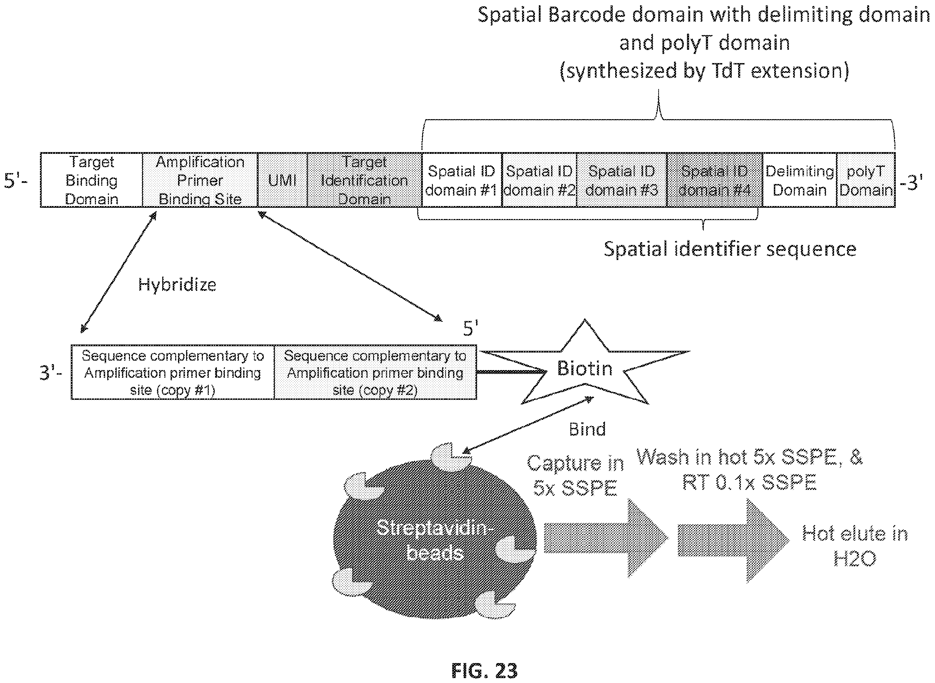

FIG. 23 is an exemplary schematic of a method of purifying the probes collected from the tissue sample as part of quantifying the probes via sequencing. In this non-limiting example, the probe has a target binding domain, followed by an amplification primer binding site, followed by a unique molecular identifier, followed by a target identification domain. In this non-limiting example, a spatial barcode has been synthesized and extended on the 3' end of the probe. The spatial barcode domain has been synthesized by TdT extension and comprises a spatial identifier sequence comprising four (#1, #2, #3 and #4) spatial identification domains, a delimiting domain and a polyT domain. In this non-limiting example, a capture probe is used, wherein the capture probe comprises two copies of a sequence that is complementary to the amplification primer binding site on the probe. The capture probe further comprises a biotin moiety. The capture probe is hybridized to the probe, and then the biotin moiety is bound to streptavidin-beads in 5x SSPE buffer. The beads can then be washed in hot 5x SSPE and room temperature (RT) 0.1x SSPE. The probes can then be eluted from the streptavidin beads in hot H₂O.

Example 4 describes non-limiting examples of xGen hybridization capture-based purifications that can be used in the methods of the present disclosure. As would be appreciated by the skilled artisan, the volumes of reagents, the amounts of reagents, the concentration of reagents, the length of certain steps and the temperature at which certain steps are performed described in Example 4 can be modified and optimized for particular sample types and/or sizes.

### Tissue Sample Pre-treatment

In some aspects of the methods of the invention and related methods provided for illustrative purposes, the step of contacting a tissue sample with at least one probe or a plurality of probes can comprise incubating the tissue sample with the at least one probe or the plurality of probes for at least about one hour, or at least about two hours, or at least about three hours, or at least about four hours, or at least about five hours, or at least about six hours, or at least about seven hours, or at least about eight hours, or at least about nine hours, or at least about 10 hours, or at least about 11 hours, or at least about 12 hours, or at least about 24 hours, or at least about 36 hours, or at least about 48 hours.

In some aspects, the step of contacting a tissue sample with at least one probe or a plurality of probes can comprise contacting the tissue sample with the at least one probe or a plurality of probes in combination with blocking DNA that has been terminated with ddTTP.

In some aspects, prior to contacting a tissue sample with at least one probe or a plurality of probes, the tissue sample can be subjected to ddTTP (dideoxthymidine-triphosphate) termination. ddTTP termination can comprise contacting the tissue sample with ddTTP and TdT. Without wishing to be bound by theory, ddTTP termination is performed to remove block free 3'-OH moieties of endogenous nucleic acids within the tissue sample, preventing the endogenous nucleic acids from being extended during the extension of the spatial barcodes.

In some aspects, the step of collecting the probes bound to target analytes in the tissue sample can comprise ExoIII digestion. Without wishing to be bound by theory, ExoIII digestion allows for the removal of dsDNA and the enrichment of ssDNA or dsDNA with a protruding 3' overhang.

### Landmarking Probes and methods

In some aspects, the methods of the invention and related methods provided for illustrative purposes can further comprise incubating a sample with at least one general landmarking probe.

In some aspects, the methods can further comprise detecting at least one general landmarking probe bound within a sample.

In some aspects, the detection of at least one general landmarking probe bound within the sample can be used to define the boundaries of individual cells within a sample. By detecting the boundaries of individual cells within a sample, spatial patterns can be determined for downstream synthesis of spatial barcodes within the sample. In some aspects, the detection of one or more general landmarking probes in a sample can be used to generate a fluorescent cell image that subsequently collected gene expression data can then be mapped onto to create a spatially-resolved profile of gene expression.

In some aspects, a general landmarking probe can comprise a detectable label. In some aspects, the detectable label in be a fluorescent label.

In some aspects, a general landmarking probe can comprise at least one antibody. The antibody can specifically bind to at least one cellular marker. In some aspects, the at least one antibody can be linked, directly or indirectly, to at least one detectable label.

A cellular marker can be a cytoplasmic marker. A cellular marker can be any cytoplasmic marker known in the art. Examples of cytoplasmic markers include, but are not limited to, specific proteins that are located exclusively or primarily within the cytoplasm of a cell. Specific proteins that are located exclusively or primarily within the cytoplasm of a cell can include, but are not limited to, microtubule proteins, vimentin, desmin and cytokeratin.

A cellular marker can be a membrane marker. A cellular marker can be any membrane marker known in the art. Examples of membrane markers include, but are not limited to, specific proteins that are exclusively or primarily on, associated with, or embedded in a specific membrane within a cell. Examples of such proteins include, but are not limited to, sodium-potassium ATPase, plasma membrane calcium ATPase (PMCA), proteins of the cadherin family, CD98, proteins associated with caveolae (*e.g.* caveolin), any integral membrane protein known in the art and any membrane-associated protein known in the art.

A cellular marker can be an organelle marker. An organelle marker can be any organelle marker known in the art. Examples of organelle markers include, but are not limited to, specific proteins that are exclusively or primarily associated with a specific organelle (*e.g.* Endoplasmic reticulum, golgi apparatus, mitochondria, ribosome, lysosome, endosomes, peroxisome, autophagosome, or any other organelle known in the art). Examples of such proteins include, but are not limited to, calreticulin (endoplasmic reticulum), GM130 (golgi apparatus), ATP5A (mitochondria), TOMM20 (mitochondria), RPS3 (ribosome), M6PR (lysosome), EEA1 (endosome), RAB7 (endosome), catalase (peroxisome), SQSTM1/p62 (autophagosome) and LC3B (autophagosome).

A cellular marker can be a nuclear marker. A nuclear marker can be any nuclear marker known in the art. Examples of nuclear markers include, but are not limited to, specific proteins that are exclusively or primarily associated with the nucleus of a cell, or a specific sub-structure within the nucleus of the cell. Examples of such proteins include, but are not limited to KDM1, NUP98, Lamin A, Lamin C, Lamin, SC35, Fibrillarin, HP1 alpha, CENPA or any other nuclear protein known in the art. Nuclear markers can also include, but are not limited to, nucleic acid stains such as DAPI or SYTO9 to stain cellular nucleic acids.

In some aspects, a general landmarking probe of the present disclosure can comprise a dye that targets one or more specific membrane(s) in a cell such as the plasma membrane, the nuclear membrane, the endoplasmic reticulum membrane, the lysosome membrane or any other membrane known in the art.

In some aspects, a general landmarking probe of the present disclosure can be detected in order to define the boundaries of individual cells within a sample. By detecting the boundaries of individual cells within a sample, spatial patterns can be determined for downstream synthesis of spatial barcodes within the sample. In some aspects, the detection of one or more general landmarking probes in a sample can be used to generate a fluorescent cell image that subsequently collected gene expression can then be mapped onto to create a spatially-resolved profile of gene expression.

### Samples

In some aspects of the invention and related disclosure provided for illustrative purposes, samples may comprise any number of things, including, but not limited to: cells (including both primary cells and cultured cell lines) and tissues (including cultured or explanted). In aspects, a tissue sample (fixed or unfixed) is embedded, serially sectioned, and immobilized onto a microscope slide. As is well known, a pair of serial sections will include at least one cell that is present in both serial sections. Structures and cell types, located on a first serial section will have a similar location on an adjacent serial section. The sample can be cultured cells or dissociated cells (fixed or unfixed) that have been immobilized onto a slide. A sample can be a formalin-fixed paraffin-embedded (FFPE) tissue sample.

In aspects, a tissue sample is a biopsied tumor or a portion thereof, i.e., a clinically-relevant tissue sample. For example, the tumor may be from a breast cancer. The sample may be an excised lymph node.

The sample can be obtained from virtually any organism including multicellular organisms, e.g., of the plant, fungus, and animal kingdoms; preferably, the sample is obtained from an animal, *e.g.,* a mammal. Human samples are particularly preferred.

In some aspects, the probes, compositions, methods, and kits described herein are used in the diagnosis of a condition. As used herein the term diagnose or diagnosis of a condition includes predicting or diagnosing the condition, determining predisposition to the condition, monitoring treatment of the condition, diagnosing a therapeutic response of the disease, and prognosis of the condition, condition progression, and response to particular treatment of the condition. For example, a tissue sample can be assayed according to any of the probes, methods, or kits described herein to determine the presence and/or quantity of markers of a disease or malignant cell type in the sample (relative to the non-diseased condition), thereby diagnosing or staging a disease or a cancer.

In general, samples attached to a slide can be first imaged using fluorescence (e.g., fluorescent antibodies or fluorescent stains (e.g., DAPI)) to identify morphology, regions of interest, cell types of interest, and single cells and then expression of proteins and/or nucleic acids can be digitally counted from the sample on the same slide.

### Sequencing

In some aspects of the methods of the invention and related methods provided for illustrative purposes, quantifying via sequencing can comprise using any known sequencing method in the art to determine the number of probes that were collected that a) correspond to a specific target analyte (*i.e.* comprise a specific target identification domain) and that b) correspond bound to a specific location/region of interest (*i.e.* comprise the same identifier domains within a spatial barcode domain.

Sequencing can be performed by any known sequencing method, including, but not limited to next-generation sequencing methods, sequencing by synthesis, massively parallel sequencing, or any other sequencing method known and practice by the skilled artisan.

In a preferred aspect, nucleic acid amplification can be solid-phase nucleic acid amplification. Thus, in further aspects the invention provides a method of solid-phase nucleic acid amplification of template polynucleotide molecules which comprises: preparing a library of template polynucleotide molecules which have common sequences at their 5' and 3' ends using the methods of the present disclosure and carrying out a solid-phase nucleic acid amplification reaction wherein said template polynucleotide molecules are amplified. Compositions and methods for nucleic acid amplification and sequencing have been described in, e.g., US9376678.

The term "solid-phase amplification" as used herein refers to any nucleic acid amplification reaction carried out on or in association with a solid support such that all or a portion of the amplified products are immobilized on the solid support as they are formed. In particular, the term encompasses solid-phase polymerase chain reaction (solid-phase PCR), which is a reaction analogous to standard solution phase PCR, except that one or both of the forward and reverse amplification primers is/are immobilized on the solid support.

Although the invention encompasses "solid-phase" amplification methods in which only one amplification primer is immobilized (the other primer usually being present in free solution), it is preferred for the solid support to be provided with both the forward and the reverse primers immobilized. In practice, there will be a "plurality" of identical forward primers and/or a "plurality" of identical reverse primers immobilized on the solid support, since the PCR process requires an excess of primers to sustain amplification. References herein to forward and reverse primers are to be interpreted accordingly as encompassing a "plurality" of such primers unless the context indicates otherwise.

As will be appreciated by the skilled reader, any given PCR reaction requires at least one type of forward primer and at least one type of reverse primer specific for the template to be amplified. However, in certain aspects the forward and reverse primers may comprise template-specific portions of identical sequence, and may have entirely identical nucleotide sequence and structure (including any non-nucleotide modifications). In other words, it is possible to carry out solid-phase amplification using only one type of primer, and such single-primer methods are encompassed within the scope of the invention. Other aspects may use forward and reverse primers which contain identical template-specific sequences but which differ in some other structural features. For example one type of primer may contain a non-nucleotide modification which is not present in the other.

In other aspects of the invention the forward and reverse primers may contain template-specific portions of different sequence.

Amplification primers for solid-phase PCR are preferably immobilized by covalent attachment to the solid support at or near the 5' end of the primer, leaving the template-specific portion of the primer free for annealing to its cognate template and the 3' hydroxyl group free for primer extension. Any suitable covalent attachment means known in the art may be used for this purpose. The chosen attachment chemistry will depend on the nature of the solid support, and any derivatization or functionalization applied to it. The primer itself may include a moiety, which may be a non-nucleotide chemical modification, to facilitate attachment. In one particularly preferred aspect the primer may include a sulphur-containing nucleophile, such as phosphorothioate or thiophosphate, at the 5' end. In the case of solid-supported polyacrylamide hydrogels (as described below), this nucleophile will bind to a "C" group present in the hydrogel. The most preferred means of attaching primers and templates to a solid support is via 5' phosphorothioate attachment to a hydrogel comprised of polymerized acrylamide and N-(5-bromoacetamidylpentyl)acrylamide (BRAPA).

The terms "cluster" and "colony" are used interchangeably herein to refer to a discrete site on a solid support comprised of a plurality of identical immobilized nucleic acid strands and a plurality of identical immobilized complementary nucleic acid strands. The term "clustered array" refers to an array formed from such clusters or colonies. In this context the term "array" is not to be understood as requiring an ordered arrangement of clusters.

The invention also encompasses methods of sequencing the amplified nucleic acids generated by solid-phase amplification. Thus, the invention provides a method of nucleic acid sequencing comprising amplifying a library of nucleic acid templates by the methods of the present disclosure described above, using solid-phase amplification as described above to amplify this library on a solid support, and carrying out a nucleic acid sequencing reaction to determine the sequence of the whole or a part of at least one amplified nucleic acid strand produced in the solid-phase amplification reaction.

Sequencing, as referred to herein, can be carried out using any suitable "sequencing-by-synthesis" technique, wherein nucleotides are added successively to a free 3' hydroxyl group, resulting in synthesis of a polynucleotide chain in the 5' to 3' direction. The nature of the nucleotide added is preferably determined after each nucleotide addition.

The initiation point for the sequencing reaction may be provided by annealing of a sequencing primer to a product of the whole genome or solid-phase amplification reaction. In this connection, one or both of the adapters added during formation of the template library may include a nucleotide sequence which permits annealing of a sequencing primer to amplified products derived by whole genome or solid-phase amplification of the template library.

The products of solid-phase amplification reactions wherein both forward and reverse amplification primers are covalently immobilized on the solid surface are so-called "bridged" structures formed by annealing of pairs of immobilized polynucleotide strands and immobilized complementary strands, both strands being attached to the solid support at the 5' end. Arrays comprised of such bridged structures provide inefficient templates for nucleic acid sequencing, since hybridization of a conventional sequencing primer to one of the immobilized strands is not favored compared to annealing of this strand to its immobilized complementary strand under standard conditions for hybridization.

In order to provide more suitable templates for nucleic acid sequencing it is preferred to remove substantially all or at least a portion of one of the immobilized strands in the "bridged" structure in order to generate a template which is at least partially single-stranded. The portion of the template which is single-stranded will thus be available for hybridization to a sequencing primer. The process of removing all or a portion of one immobilized strand in a "bridged" double-stranded nucleic acid structure may be referred to herein as "linearization".

Bridged template structures may be linearized by cleavage of one or both strands with a restriction endonuclease or by cleavage of one strand with a nicking endonuclease. Other methods of cleavage can be used as an alternative to restriction enzymes or nicking enzymes, including inter alfa chemical cleavage (e.g. cleavage of a diol linkage with periodate), cleavage of abasic sites by cleavage with endonuclease, or by exposure to heat or alkali, cleavage of ribonucleotides incorporated into amplification products otherwise comprised of deoxyribonucleotides, photochemical cleavage or cleavage of a peptide linker.

It will be appreciated that a linearization step may not be essential if the solid-phase amplification reaction is performed with only one primer covalently immobilized and the other in free solution.

In order to generate a linearized template suitable for sequencing it is necessary to remove "unequal" amounts of the complementary strands in the bridged structure formed by amplification so as to leave behind a linearized template for sequencing which is fully or partially single stranded. Most preferably one strand of the bridged structure is substantially or completely removed.

Following the cleavage step, regardless of the method used for cleavage, the product of the cleavage reaction may be subjected to denaturing conditions in order to remove the portion(s) of the cleaved strand(s) that are not attached to the solid support. Suitable denaturing conditions will be apparent to the skilled reader with reference to standard molecular biology protocols.

Denaturation (and subsequent re-annealing of the cleaved strands) results in the production of a sequencing template which is partially or substantially single-stranded. A sequencing reaction may then be initiated by hybridization of a sequencing primer to the single-stranded portion of the template.

Thus, the nucleic acid sequencing reaction may comprise hybridizing a sequencing primer to a single-stranded region of a linearized amplification product, sequentially incorporating one or more nucleotides into a polynucleotide strand complementary to the region of amplified template strand to be sequenced, identifying the base present in one or more of the incorporated nucleotide(s) and thereby determining the sequence of a region of the template strand.

One preferred sequencing method which can be used in accordance with the invention relies on the use of modified nucleotides that can act as chain terminators. Once the modified nucleotide has been incorporated into the growing polynucleotide chain complementary to the region of the template being sequenced there is no free 3'-OH group available to direct further sequence extension and therefore the polymerase cannot add further nucleotides. Once the nature of the base incorporated into the growing chain has been determined, the 3' block may be removed to allow addition of the next successive nucleotide. By ordering the products derived using these modified nucleotides it is possible to deduce the DNA sequence of the DNA template. Such reactions can be done in a single experiment if each of the modified nucleotides has attached a different label, known to correspond to the particular base, to facilitate discrimination between the bases added at each incorporation step. Alternatively, a separate reaction may be carried out containing each of the modified nucleotides separately.

The modified nucleotides may carry a label to facilitate their detection. Preferably this is a fluorescent label. Each nucleotide type may carry a different fluorescent label. However, the detectable label need not be a fluorescent label. Any label can be used which allows the detection of an incorporated nucleotide.

One method for detecting fluorescently labelled nucleotides comprises using laser light of a wavelength specific for the labelled nucleotides, or the use of other suitable sources of illumination. The fluorescence from the label on the nucleotide may be detected by a CCD camera or other suitable detection means.

Suitable alternative sequence techniques for use in the methods of the invention and disclosure include, for example, Pyrosequencing, FISSEQ (fluorescent in situ sequencing), MPSS (massively parallel signature sequencing) and sequencing by ligation-based methods.

### Hybridization

As used herein, the term "hybridize" is used in its broadest sense to mean the formation of a stable nucleic acid duplex. In one aspect, "stable duplex" means that a duplex structure is not destroyed by a stringent wash under conditions such as, for example, a temperature of either about 5 °C below or about 5 °C above the Tm of a strand of the duplex and low monovalent salt concentration, *e.g.,* less than 0.2 M, or less than 0.1 M or salt concentrations known to those of skill in the art. A duplex can be "perfectly matched", such that the polynucleotide and/or oligonucleotide strands making up the duplex form a double stranded structure with one another such that every nucleotide in each strand undergoes Watson-Crick base pairing with a nucleotide in the other strand. The term "duplex" comprises, but is not limited to, the pairing of nucleoside analogs, such as deoxyinosine, nucleosides with 2-aminopurine bases, PNAs, and the like, that can be employed. A duplex can comprise at least one mismatch, wherein the term "mismatch" means that a pair of nucleotides in the duplex fail to undergo Watson-Crick bonding.

As used herein, the term "hybridization conditions," will typically include salt concentrations of less than about 1 M, more usually less than about 500 mM and even more usually less than about 200 mM. Hybridization temperatures can be as low as 5 °C, but are typically greater than 22 °C, more typically greater than about 30 °C, and often in excess of about 37 °C. Hybridizations are usually performed under stringent conditions, *e.g.,* conditions under which a probe will specifically hybridize to its target analyte. Stringent conditions are sequence-dependent and are different in different circumstances. Longer fragments can require higher hybridization temperatures for specific hybridization. As other factors can affect the stringency of hybridization, including base composition and length of the complementary strands, presence of organic solvents and extent of base mismatching, the combination of parameters is more important than the absolute measure of any one alone. Certain hybridization conditions will promote the formation of a duplex between the entire length of a target binding domain and the target analyte. Other hybridization conditions will promote the formation of a duplex only along certain portions of the target binding domain.

### Compositions of the Present Disclosure (compositions not claimed)

In a related instance provided for illustrative purposes, the present disclosure provides a composition comprising any of the probes, extended probes or probe-intermediates described herein.

### Examples

### Example 1-amplification of the probes of the present disclosure

The following are a set of experiment to test the amplification of the probes of the present disclosure.

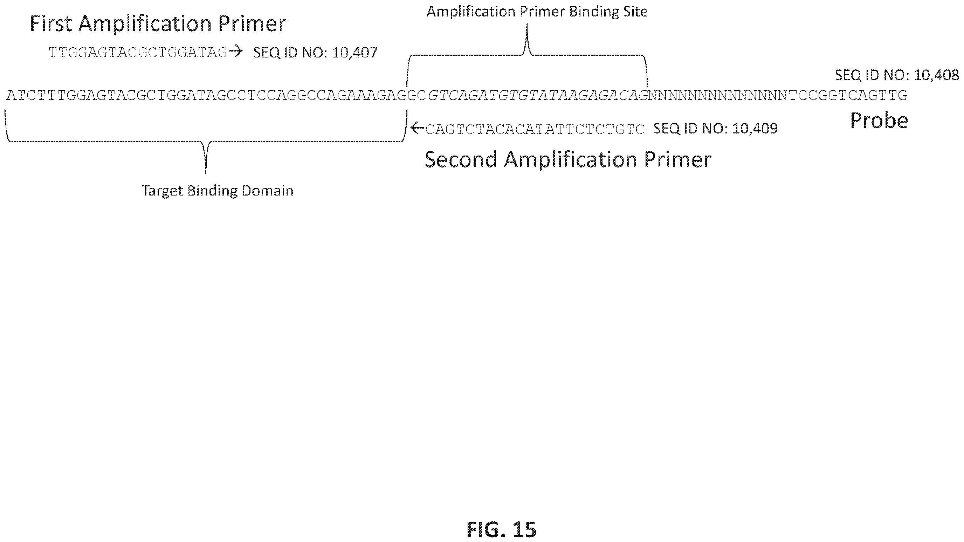

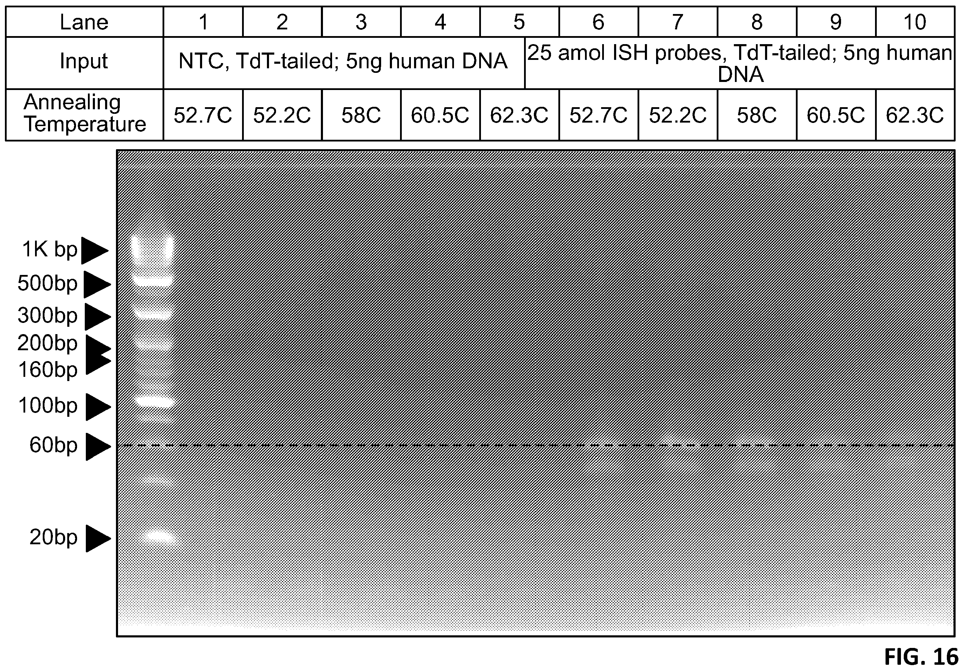

In a first experiment, the probe to be amplified was comprised of a target-binding domain and an amplification primer binding site, as shown in FIG. 15. The first amplification primer comprised a sequence of the target-binding domain and the second amplification primer comprised a sequence complementary to the amplification primer binding site on the probe, as shown in FIG. 15. These two amplification primers were then used to amplify the probe, with an expected amplicon of 61 bp. FIG. 16 shows the results of the amplification experiment as analyzed using gel electrophoresis. In the first five lanes were control amplification reactions that contained no probes (NTC, non-template control) that had been incubated with TdT and dTTP and then added to 5 ng of human DNA. In lanes 6-10 were amplification reactions that contained 25 attomols of probes that had been incubated with TdT and dTTP and then added to 5 ng of human DNA. The control reactions yielded no amplification products, while the amplification reactions with the probes yielded amplification products at the expected molecular weight.

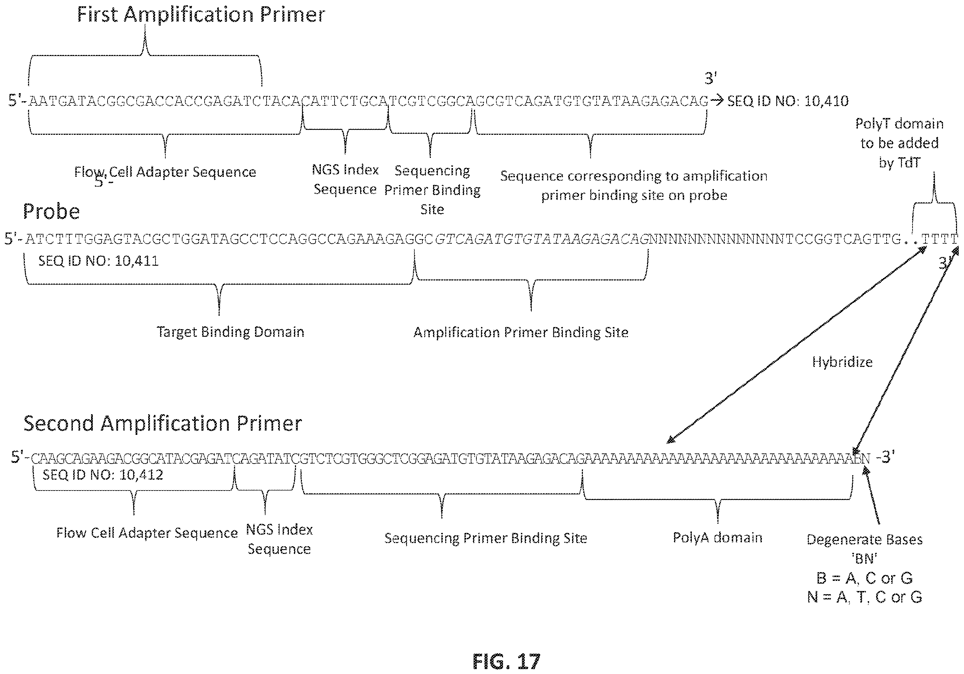

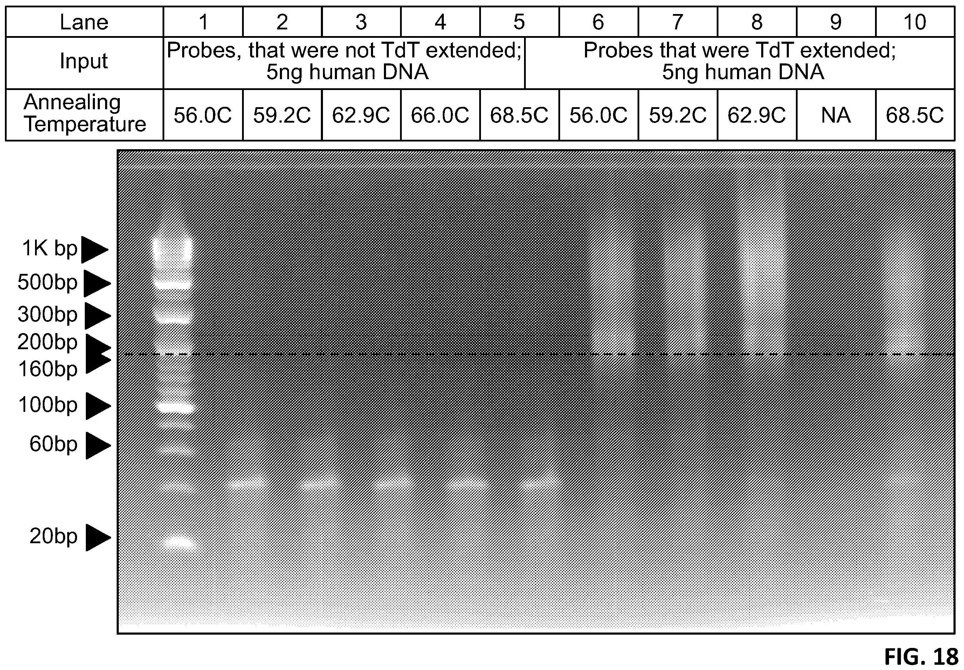

In a second experiment, the probe to be amplified was comprised of a target-binding domain and an amplification primer binding site, as shown in FIG. 17. The first amplification primer comprised a flow cell adapter sequence, an NGS index sequence, a sequencing primer binding site and a sequence of the amplification primer binding site of the probe, as shown in FIG. 17. The second amplification primer comprised a flow cell adapter sequence, an NGS index sequence, a sequencing primer binding site and a polyA domain that was able to hybridize to the polyT domain added to the end of the probe by TdT extension. These two amplification primers were then used to amplify the probe, with an expected amplicon of 194 bp. FIG. 18 shows the results of the amplification experiment as analyzed using gel electrophoresis. 25 attomols of the probes were first incubated with dTTP in the presence or absence of TdT to perform TdT mediated, polyT extension. These reactions were then added separately to 5 ng of 300 bp human gDNA and amplified using the amplification primers. Lanes 1-5 of the gel contain the amplification reactions using the probes that were not treated with TdT, and thus should have no polyT domains. As expected, these probes were not amplified as the second amplification primer was unable to bind to the lack of a polyT domain. Lanes 6-8 and 10 of the gel contain the amplification reactions using the probes treated with TdT, and thus should have a polyT domain. These amplification reactions yielded products at the expected molecular weight, demonstrating that the polyT domain can be used to specifically amplify probes of the present disclosure that had been extended by TdT.

Taken together, these results indicate that the probes of the present disclosure can be amplified using the amplification primers and methods described herein

### Example 2-in situ TdT synthesis and probe/DNA recover

The following is a set of experiments to test the recovery of DNA from tissue samples that have been incubated with TdT and dTTP.

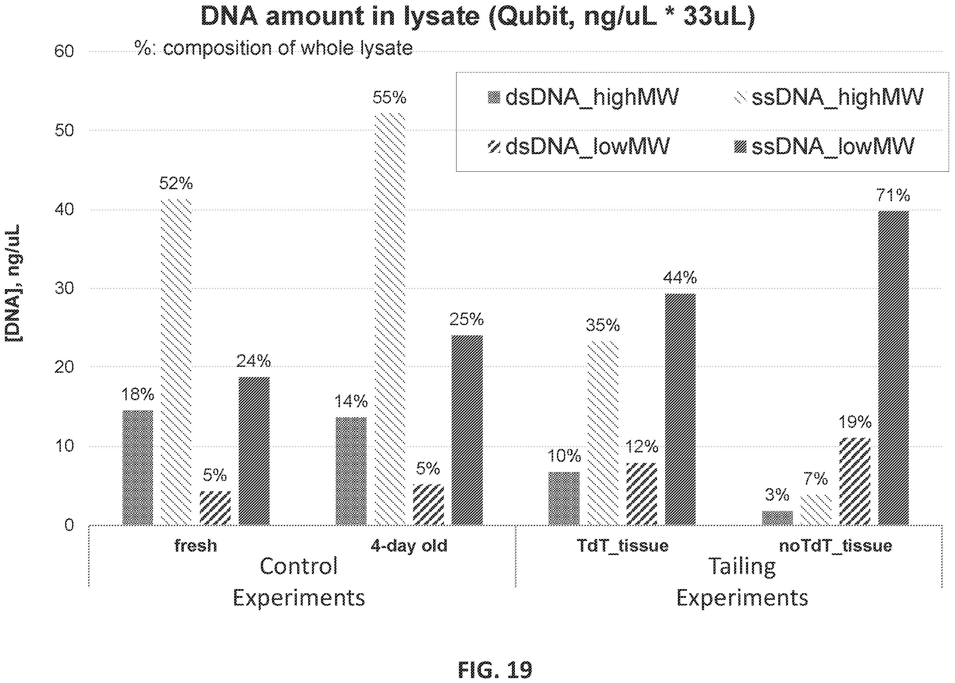

In a first set of control experiments, fresh and four-day-old tissue samples were subjected to proteinase K digestion at 56°C for 1 hour followed by further digestion at 90°C for 15 minutes. DNA extracted from the tissue samples and subjected to Qubit analysis to determine the amount of double stranded DNA of high molecular weight (dsDNA _highMW), double stranded DNA of low molecular weight (dsDNA_lowMW), single stranded DNA of high molecular weight (ssDNA_highMW) and single-stranded DNA of low molecular weight (ssDNA_lowMW). The results of this analysis are shown on the left side of the bar graph in FIG. 19 (labeled control experiments). As show in FIG. 19, high molecular weight fractions (>500bp) were observed for both dsDNA and ssDNA, and ssDNA accounted for about 80% of the DNA recovered.

In a second set of experiments, tissue samples were incubated with probes of the present disclosure in combination with ddTTP-terminated blocking DNA overnight. The tissue samples were then washed and dehydrated. The tissue samples were then incubated with dTTP in the presence (TdT_tissue) or the absence (noTdT_tissue) of TdT to allow for polyT tailing by TdT. After the tailing reaction, the tissue samples were subjected to proteinase K digestion at 56°C for 1 hour followed by further digestion at 90°C for 15 minutes. DNA extracted from the tissue samples and subjected to Qubit analysis to determine the amount of double stranded DNA of high molecular weight (dsDNA_highMW), double stranded DNA of low molecular weight (dsDNA_lowMW), single stranded DNA of high molecular weight (ssDNA_highMW) and single-stranded DNA of low molecular weight (ssDNA_lowMW). The results of this analysis are shown on the right side of the bar graph in FIG. 19 (tailing experiments). As shown in FIG. 19, the tissue sample that had been incubated TdT yielded increased amounts of high molecular weight DNA, indicating the polyT tailing reaction was successful. In fact, the on-tissue tailing resulted in >35% of DNA having a polyT tail with an average polyT length of > 500 nucleotides. The low and high molecular weight DNA fractions were then further analyzed to determine if they contained the probes that were contacted to the tissue sample. Amplification using amplification primers specific to the probes indicated that the probes were present in the low molecular weight fractions of DNA. Moreover, more probes were recovered in the tissue sample that was subjected to TdT tailing.

Taken together, these results indicate that TdT can be used to extend polynucleotides *in situ* within a tissue sample, and that these extended polynucleotides can be subsequently collected. The results also indicate that the addition of polyT tails on bound probes can increase the recovery of the probes.

### Example 3 - in situ DNA synthesis using TdT

The following example describes a set of experiments that demonstrate that the TdT enzyme can be used to perform *in situ* DNA synthesis within a tissue sample.

FFPE tissue samples (TMA tonsil or 3-cell-pellet array, 5 µm thickness on silane coated cover glass) were prepared using the following two-day protocol:
Day 1:
   1) Samples were baked at 60°C and then subjected to deparaffinization via xylene and EtOH;
   2) Samples were heated at 100°C and treated with protease;
   3) Fiducial markers were deposited onto the samples;
   4) Samples were fixed/cross-linked using NBF, Tris-Glycine and Sulfo-NHS acetate (to block silane); and
   5) Samples were incubated overnight in the presence or absence of probes along with 100 µg/mL carrier DNA (<2k bp).
Day 2:
   6) samples were washed at 37°C or room temperature using 2x saline-sodium citrate, 50% formamide, with or without 0.1% Tween20;
   7) samples were incubated with 75 µl of TdT tailing mix (25 nmol dTTP with or without 150 units of TdT) for 1.5 hours at 37°C, after which the reaction was quenched by the addition of 0.2M EDTA;
   8) samples were then washed three times with EtOH, air dried and stored at 4°C.

To determine whether the TdT enzyme added poly-T tails onto the probes bound within the samples, the samples as prepared above were stained with 100 nM of Alexa546-labeled, polyA oligonucleotides comprising 15 "A" nucleotides. These labeled polyA oligonucleotides should bind to any polyT tails added by the TdT enzyme and be detectable under a fluorescent microscope. After initial detection of the polyA oligonucleotides, the samples were also treated with a 0.0033x Sodium Chloride-Sodium Phosphate-EDTA (SSPE) wash to strip the polyA oligonucleotides from the samples. Cells were stained with the poly A15 oligonucleotide regardless of whether the cells had been treated with the probes. Without wishing to be bound by theory, this most likely indicates that the TdT enzyme added polyT tails on the blocking DNA and endogenous nuclear DNA, in addition to the ISH probes. Moreover, the SSPE successfully striped a majority of the labeled polyA oligonucleotides from the cells.

In a similar experiment as described above, cells were stained with 20 nM of the labeled polyA oligonucleotide and three different sample types were tested (CCRF-CEM cells, DAUDI cells and MDA-MB-468 cells). All three cell types that were treated with the probes and TdT were stained with the polyA oligonucleotide, indicating that the TdT treatment resulted in the addition of polyT tails to DNA molecules within the cells. Control experiments were also performed wherein the same three types of samples were treated with the probes ("ISH probes") but were not treated with TdT. These control samples exhibited low to no staining with the labeled polyA oligonucleotide, indicating that the signal observed in the experiments described above is due to polyT tailing by TdT.

Taken together, these results indicate that TdT can perform *in situ* DNA synthesis in a tissue sample.

### Example 4 - xGen hybridization capture-based purification

The following are non-limiting examples of xGen hybridization capture-based purifications that can be used in the methods of the present disclosure.

An xGen hybridization capture-based purification can comprise:
a) incubating tissue sample lysate (crude or Ampure purified) with capture probes comprising at least one sequence complementary to probes of the present disclosure and at least one biotin moiety at 95°C for 30 seconds in 5x SSPE, followed by an incubation at 43°C for one hour in 5x SSPE;
b) incubating the solution from step (a) three times with streptavidin-coated beads at 38°C for 10 minutes in 5x SSPE;
c) washing the beads with 5x SSPE twice at 43°C to remove unbound molecules;
d) further washing the beads with 0.1x SSPE three times at room temperature to reduce non-specific binding; and
e) eluting the bound probes from the beads by incubating the beads in H₂O at 45°C for ten minutes.

An xGen hybridization capture-based purification can comprise:
a) incubating 25 µl of tissue lysate (about 75 to 125 ng/µl total DNA) or mock lysate (200 ng huDNA +/- 25amol probes) with 200pmol of capture probes in a total of 35 µl of 5xSSPE buffer at room temperature for 5 minutes;
b) further incubating the solution from step (a) at 95°C for 30 seconds;
c) further incubating the solution from step (b) at 43°C for 1 hour;
d) mixing the solution from step (c) with 110 µl (1.1 mg) of washed, magnetic streptavidin-coated beads, wherein the streptavidin-coated beads were washed with twice with 1mL of 50°C 5x SSPE;
e) pipetting the solution from step (d) up and down 10 times;
f) incubating the solution from step (e) at 38°C for 10 minutes three times, wherein between each 10 minute incubation, the solution is gently vortexed to resuspend the beads;
g) harvesting the beads on a magnet;
h) washing the beads twice with 100 µl of 5x SSPE incubated at 43°C for five minutes each wash;
i) resuspending the washed beads in 100 µl of 0.1x SSPE;
j) further washing the beads three times with 1 mL of 0.1x SSPE;
k) resuspending the washed beads in 100 µl of 0.1x SSPE;
l) harvesting the beads on a magnet;
m) resuspending the beads in 25 µl of H₂O and incubate at 45°C for 10 minutes to elute the bound probes from the beads
n) optionally, adding carrier DNA to each purified elute to prevent loss of probes due to non-specific absorption onto the tube.

## Claims

1. A method of producing a spatially-resolved profile of the abundance of at least two target analytes in a first and an at least second location of a tissue sample comprising:
a) contacting the tissue sample with a solution comprising at least two species of probes, the probes comprising a target-binding domain and a target identification domain,
wherein each species of probe comprises a unique target-binding domain that binds to one of the at least two target analytes and a unique target identification domain specific for the target analyte, and a free 3'-OH moiety;
b) ligating a nucleotide to the free 3'-OH moiety of at least one bound probe by contacting the tissue sample with a first plurality of nucleotide-polymerase complexes, thereby extending the barcode domain of the at least one bound probe and forming a spatial barcode domain,
wherein at least one nucleotide-polymerase complex in the first plurality comprises the nucleotide operably linked to a polymerase via a photocleavable linker;
c) illuminating at least one location of the tissue sample with light sufficient to cleave the photocleavable linker of the nucleotide-polymerase complexes, thereby releasing the polymerases and exposing a free 3'-OH moiety on the spatial barcode domain at the at least one location of the tissue sample;
d) ligating a nucleotide to the free 3'-OH moiety of the spatial barcode domain of at least one bound probe at the at least one location illuminated in step (c) by contacting the tissue sample with an additional plurality of nucleotide-polymerase complexes, thereby extending the spatial barcode domain of the at least one bound probe,
wherein at least one nucleotide-polymerase complex in the additional plurality comprises the nucleotide operably linked to a polymerase via a photocleavable linker;
e) repeating steps (c) and (d) until
the spatial barcode domain of at least one probe bound to a target analyte in the first location of the tissue sample has been extended such that the spatial barcode domain comprises a unique nucleic acid sequence specific to the first location of the tissue sample, and
the spatial barcode domain of at least one probe bound to a target analyte in the at least second location of the tissue sample has been extended such that the spatial barcode domain comprises a unique nucleic acid sequence specific to the at least second location of the tissue sample;
f) collecting the probes bound to target analytes in the tissue sample; and
g) quantifying via sequencing the probes collected in step (f), thereby determining the abundance of the at least two target analytes in the first and the at least second location of the tissue sample, thereby producing a spatially-resolved profile of the abundance of the at least two target analytes.

2. A method of producing a spatially-resolved profile of the abundance of at least two target analytes in a first and an at least second location of a tissue sample comprising:
a) contacting the tissue sample with a solution comprising at least two species of probes, the probes comprising a target-binding domain and a target identification domain,
wherein each species of probe comprises a unique target-binding domain that binds to one of the at least two target analytes and a unique target identification domain specific for the target analyte, and a free 3'-OH moiety;
b) ligating a nucleotide to the free 3'-OH moiety of the barcode domain of at least one bound probe by contacting the tissue sample with a first plurality of reversible terminator nucleotides and a first plurality of polymerases, thereby extending the at least one bound probe and forming a spatial barcode domain,
wherein at least one reversible terminator nucleotide in the first plurality comprises the nucleotide operably linked to a 3' terminator moiety via a photocleavable linker;
c) illuminating at least one location of the tissue sample with light sufficient to cleave the photocleavable linker of the reversible terminator nucleotides, thereby releasing the reversible 3' terminator moieties and exposing a free 3'-OH moiety on the spatial barcode domain at the at least one location of the tissue sample;
d) ligating a nucleotide to the free 3'-OH moiety of the spatial barcode domain of at least one bound probe at the at least one location illuminated in step (c) by contacting the tissue sample with an additional plurality of reversible terminator nucleotides and an additional plurality of polymerases, thereby extending the spatial barcode domain of the at least one bound probe,
wherein at least one reversible terminator nucleotide in the additional plurality comprises the nucleotide operably linked to a reversible 3' terminator moiety via a photocleavable linker;
e) repeating steps (c) and (d) until
the spatial barcode domain of at least one probe bound to a target analyte in the first location of the tissue sample has been extended such that the spatial barcode domain comprises a unique nucleic acid sequence specific to the first location of the tissue sample,
the spatial barcode domain of at least one probe bound to a target analyte in the at least second location of the tissue sample has been extended such that the spatial barcode domain comprises a unique nucleic acid sequence specific to the at least second location of the tissue sample;
f) collecting the probes bound to target analytes in the tissue sample; and
g) quantifying via sequencing the probes collected in step (f), thereby determining the abundance of the at least two target analytes in the first and the at least second location of the tissue sample, thereby producing a spatially-resolved profile of the abundance of the at least two target analytes.

3. The method of claim 1 or claim 2, further comprising comparing the abundance of the at least two target analytes in the first location of the tissue sample and the at least two target analytes in the at least second location of the tissue sample.

4. The method of any of the preceding claims, wherein the polymerase is terminal deoxynucleotidyl transferase or a biologically-active portion thereof.

5. The method of any one of claims 2-4, wherein
(a) the at least one reversible terminator nucleotide comprises 3'-O-(2-nitrobenzyl)-dATP, 3'-O-(2-nitrobenzyl)-dCTP, 3'-O-(2-nitrobenzyl)-dGTP or 3'-O-(2-nitrobenzyl)-dTTP; or
(b) the 3' terminator moiety comprises 2-nitrobenzyl.

6. The method of any one of the preceding claims, wherein the probes further comprise:
i) a unique molecular identifier, preferably wherein the unique molecular identifier is at least about 14 nucleotides in length;
ii) an amplification primer binding site, preferably wherein the amplification primer binding site is at least about 24 nucleotides in length; and/or
iii) a constant region, preferably wherein the constant region is at least about 12 to at least about 20 nucleotides in length,
preferably wherein the probes comprise, from 5' to 3', the target binding domain, followed by the amplification primer binding site, followed by the unique molecular identifier, followed by the target identification domain, followed by the constant region.

7. The method of any one of the preceding claims, wherein the spatial barcode domain of at least one probe bound to a target analyte in the first location of the tissue sample comprises a unique spatial identifier sequence specific to the first location of the tissue sample, and the spatial barcode domain of at least one probe bound to a target analyte in the at least second location of the tissue sample comprises a unique spatial identifier sequence specific to the at least second location of the tissue sample, preferably wherein spatial identifier sequence comprises at least about 20 nucleotides.

8. The method of any one of the preceding claims, wherein the spatial identifier sequence comprises at least four spatial identification domains, preferably wherein:
(a) each of the at least four spatial identification domains comprise the same number of nucleotides, preferably wherein each spatial identification domain comprises about 1 to about 4 nucleotides, preferably wherein each spatial identification domain comprises about 4 nucleotides; or
(b) at least one of the at least four spatial identifications domains comprise a different number of nucleotides as compared to another spatial identification domain within the same spatial barcode.

9. The method of any one of the preceding claims, wherein the method further comprises:
i) after step (e) and prior to step (f), repeating steps (c) and (d) to extend the spatial barcode domain in each location of the tissue sample such that the spatial barcode domain comprises, at the 3' end, a delimiting domain, preferably wherein
(a) the delimiting domain is at least about 4 to at least about 6 nucleotides in length; and/or
(b) the sequence of the delimiting domain is the same for every spatial barcode in the sample.;
and/or
ii) after step (e) and prior to step (f), extending the spatial barcode domain in each location of the tissue sample such that the spatial barcode domain comprises a polyT domain.

10. The method of any one of the preceding claims, wherein the polyT domain comprises at least about 14 nucleotides.

11. The method of any one of the preceding claims, wherein the illumination in step (c) is:
i) provided by a light source selected from the group consisting of an arc-lamp, a laser, a focused UV light source, and light emitting diode, preferably wherein the laser is an infrared laser; or
ii) provided by a two-photon excitation microscope.

12. The method of any one of the preceding claims, wherein the first location of the tissue sample and the second location of the tissue sample are no more than about 500 nm in the x and/or y direction and no more than about 1500 nm in the z direction, preferably wherein the first location of the tissue sample and the at least second location of the tissue sample
(a) are subcellular;
(b) each comprise no more than one cell; or
(c) each comprise no more than ten cells..

13. The method of any of the preceding claims, the method further comprising prior to step (a), subjecting the tissue sample to ddTTP (dideoxthymidine-triphosphate) termination, preferably wherein subjecting the tissue sample to ddTTP termination comprises contacting the tissue sample with ddTTP and TdT.

14. The method of any one of the preceding claims, the method further comprising after step (f) and prior to step (g), amplifying the collected probes, preferably wherein amplifying the collected probes comprises the use of a first amplification primer and a second amplification primer, wherein
the first amplification primer comprises a first flow cell adapter sequence, a first NGS index sequence and a first sequencing primer binding site, and
the second amplification primer comprises a second flow cell adapter sequence, a second NGS index sequence and second sequencing primer binding site.

15. The method of any one of the preceding claims, wherein at least one of the first and the second amplification primers comprises a nucleic acid sequence that is complementary to the delimiting sequence and/or the polyT domain.

## Patentansprüche

1. Verfahren zur Erstellung eines räumlich-aufgelösten Profils der Häufigkeit von mindestens zwei Zielanalyten an einer ersten und einer zumindest zweiten Stelle einer Gewebeprobe, umfassend:
a) Inkontaktbringen der Gewebeprobe mit einer mindestens zwei Sondenspezies umfassenden Lösung, wobei die Sonden eine Zielbindungsdomäne und eine Zielidentifikationsdomäne umfassen,
wobei jede Sondenspezies eine eindeutige Zielbindungsdomäne, die an einen von den mindestens zwei Zielanalyten bindet, und eine für den Zielanalyten spezifische eindeutige Zielidentifikationsdomäne und eine freie 3'-OH-Gruppe umfasst;
b) Ligieren eines Nukleotids an die freie 3'-OH-Gruppe von mindestens einer gebundenen Sonde durch Inkontaktbringen der Gewebeprobe mit einer ersten Vielzahl von Nukleotid-Polymerase-Komplexen, wodurch die Barcodedomäne der mindestens einen gebundenen Sonde verlängert und eine räumliche Barcodedomäne gebildet wird,
wobei mindestens ein Nukleotid-Polymerase-Komplex in der ersten Vielzahl das Nukleotid, operativ verknüpft an eine Polymerase vermittels eines photospaltbaren Linkers, umfasst;
c) Beleuchten mindestens einer Stelle der Gewebeprobe mit ausreichend Licht zum Spalten des photospaltbaren Linkers der Nukleotid-Polymerase-Komplexe, wodurch die Polymerasen freigesetzt und eine freie 3'-OH-Gruppe auf der räumlichen Barcodedomäne an der mindestens einen Stelle der Gewebeprobe freigelegt wird;
d) Ligieren eines Nukleotids an die freie 3'-OH-Gruppe der räumlichen Barcodedomäne von mindestens einer gebundenen Sonde an der mindestens einen in Schritt (c) beleuchteten Stelle durch Inkontaktbringen der Gewebeprobe mit einer weiteren Vielzahl von Nukleotid-Polymerase-Komplexen, wodurch die räumliche Barcodedomäne der mindestens einen gebundenen Sonde verlängert wird,
wobei mindestens ein Nukleotid-Polymerase-Komplex in der weiteren Vielzahl das Nukleotid, operativ verknüpft an eine Polymerase vermittels eines photospaltbaren Linkers, umfasst;
e) Wiederholen der Schritte (c) und (d) bis
die räumliche Barcodedomäne von mindestens einer an einen Zielanalyten in der ersten Stelle der Gewebeprobe gebundenen Sonde so verlängert worden ist, dass die räumliche Barcodedomäne eine für die erste Stelle der Gewebeprobe spezifische eindeutige Nukleinsäuresequenz umfasst, und
die räumliche Barcodedomäne von mindestens einer an einen Zielanalyten in der zumindest zweiten Stelle der Gewebeprobe gebundenen Sonde so verlängert worden ist,
dass die räumliche Barcodedomäne eine für die zumindest zweite Stelle der Gewebeprobe spezifische eindeutige Nukleinsäuresequenz umfasst;
f) Sammeln der an Zielanalyten in der Gewebeprobe gebundenen Sonden; und
g) Quantifizieren durch Sequenzierung der in Schritt (f) gesammelten Sonden, wodurch die Häufigkeit der mindestens zwei Zielanalyten an der ersten und der zumindest zweiten Stelle der Gewebeprobe bestimmt wird, wodurch ein räumlich-aufgelöstes Profil der Häufigkeit der mindestens zwei Zielanalyten erstellt wird.

2. Verfahren zur Erstellung eines räumlich-aufgelösten Profils der Häufigkeit von mindestens zwei Zielanalyten an einer ersten und einer zumindest zweiten Stelle einer Gewebeprobe, umfassend:
a) Inkontaktbringen der Gewebeprobe mit einer mindestens zwei Sondenspezies umfassenden Lösung, wobei die Sonden eine Zielbindungsdomäne und eine Zielidentifikationsdomäne umfassen,
wobei jede Sondenspezies eine eindeutige Zielbindungsdomäne, die an einen von den mindestens zwei Zielanalyten bindet, und eine für den Zielanalyten spezifische eindeutige Zielidentifikationsdomäne und eine freie 3'-OH-Gruppe umfasst;
b) Ligieren eines Nukleotids an die freie 3'-OH-Gruppe der Barcodedomäne von mindestens einer gebundenen Sonde durch Inkontaktbringen der Gewebeprobe mit einer ersten Vielzahl von reversiblen Terminatornukleotiden und einer ersten Vielzahl von Polymerasen, wodurch die mindestens eine gebundene Sonde verlängert und eine räumliche Barcodedomäne gebildet wird,
wobei mindestens ein reversibles Terminatornukleotid in der ersten Vielzahl das Nukleotid, operativ verknüpft an eine 3'-Terminatorgruppe vermittels eines photospaltbaren Linkers, umfasst;
c) Beleuchten mindestens einer Stelle der Gewebeprobe mit ausreichend Licht zum Spalten des photospaltbaren Linkers der reversiblen Terminatornukleotide, wodurch die reversiblen 3'-Terminatorgruppen freigesetzt und eine freie 3'-OH-Gruppe auf der räumlichen Barcodedomäne an der mindestens einen Stelle der Gewebeprobe freigelegt wird;
d) Ligieren eines Nukleotids an die freie 3'-OH-Gruppe der räumlichen Barcodedomäne von mindestens einer gebundenen Sonde an der mindestens einen in Schritt (c) beleuchteten Stelle durch Inkontaktbringen der Gewebeprobe mit einer weiteren Vielzahl von reversiblen Terminatornukleotiden und einer weiteren Vielzahl von Polymerasen, wodurch die räumliche Barcodedomäne der mindestens einen gebundenen Sonde verlängert wird, wobei mindestens ein reversibles Terminatornukleotid in der weiteren Vielzahl das Nukleotid, operativ verknüpft an eine reversible 3'-Terminatorgruppe vermittels eines photospaltbaren Linkers, umfasst;
e) Wiederholen der Schritte (c) und (d) bis
die räumliche Barcodedomäne von mindestens einer an einen Zielanalyten in der ersten Stelle der Gewebeprobe gebundenen Sonde so verlängert worden ist, dass die räumliche Barcodedomäne eine für die erste Stelle der Gewebeprobe spezifische eindeutige Nukleinsäuresequenz umfasst,
die räumliche Barcodedomäne von mindestens einer an einen Zielanalyten in der zumindest zweiten Stelle der Gewebeprobe gebundenen Sonde so verlängert worden ist, dass die räumliche Barcodedomäne eine für die zumindest zweite Stelle der Gewebeprobe spezifische eindeutige Nukleinsäuresequenz umfasst;
f) Sammeln der an Zielanalyten in der Gewebeprobe gebundenen Sonden; und
g) Quantifizieren durch Sequenzierung der in Schritt (f) gesammelten Sonden, wodurch die Häufigkeit der mindestens zwei Zielanalyten an der ersten und der zumindest zweiten Stelle der Gewebeprobe bestimmt wird, wodurch ein räumlich-aufgelöstes Profil der Häufigkeit der mindestens zwei Zielanalyten erstellt wird.

3. Verfahren nach Anspruch 1 oder Anspruch 2, ferner umfassend das Vergleichen der Häufigkeit der mindestens zwei Zielanalyten an der ersten Stelle der Gewebeprobe und der mindestens zwei Zielanalyten an der zumindest zweiten Stelle der Gewebeprobe.

4. Verfahren nach beliebigen der vorhergehenden Ansprüche, wobei die Polymerase terminale Desoxynukleotidyltransferase oder ein biologisch aktiver Teil davon ist.

5. Verfahren nach einem der Ansprüche 2-4, wobei
(a) das mindestens eine reversible Terminatornukleotid 3'-O-(2-Nitrobenzyl)-dATP, 3'-*O*-(2-Nitrobenzyl)-dCTP, 3'-O-(2-Nitrobenzyl)-dGTP oder 3'-O-(2-Nitrobenzyl)-dTTP umfasst; oder
(b) die 3'-Terminatorgruppe 2-Nitrobenzyl umfasst.

6. Verfahren nach einem der vorhergehenden Ansprüche, wobei die Sonden ferner umfassen:
i) einen eindeutigen molekularen Identifikator, wobei der eindeutige molekulare Identifikator vorzugsweise mindestens etwa 14 Nukleotide lang ist;
ii) eine Amplifikationsprimer-Bindungsstelle, wobei die Amplifikationsprimer-Bindungsstelle vorzugsweise mindestens etwa 24 Nukleotide lang ist; und/oder
iii) eine konstante Region, wobei die konstante Region vorzugsweise mindestens etwa 12 bis mindestens etwa 20 Nukleotide lang ist,
wobei die Sonden vorzugsweise, von 5' nach 3', die Zielbindungsdomäne, gefolgt von der Amplifikationsprimer-Bindungsstelle, gefolgt von dem eindeutigen molekularen Identifikator, gefolgt von der Zielidentifikationsdomäne, gefolgt von der konstanten Region, umfassen.

7. Verfahren nach einem der vorhergehenden Ansprüche, wobei die räumliche Barcodedomäne von mindestens einer an einen Zielanalyten in der ersten Stelle der Gewebeprobe gebundenen Sonde eine für die erste Stelle der Gewebeprobe spezifische eindeutige räumliche Identifikatorsequenz umfasst, und die räumliche Barcodedomäne von mindestens einer an einen Zielanalyten in der zumindest zweiten Stelle der Gewebeprobe gebundenen Sonde eine für die zumindest zweite Stelle der Gewebeprobe spezifische eindeutige räumliche Identifikatorsequenz umfasst, vorzugsweise wobei die räumliche Identifikatorsequenz mindestens etwa 20 Nukleotide umfasst.

8. Verfahren nach einem der vorhergehenden Ansprüche, wobei die räumliche Identifikatorsequenz mindestens vier räumliche Identifikationsdomänen umfasst, vorzugsweise wobei:
(a) jede der mindestens vier räumlichen Identifikationsdomänen dieselbe Anzahl an Nukleotiden umfasst, vorzugsweise wobei jede räumliche Identifikationsdomäne etwa 1 bis etwa 4 Nukleotide umfasst, vorzugsweise wobei jede räumliche Identifikationsdomäne etwa 4 Nukleotide umfasst; oder
(b) mindestens eine der mindestens vier räumlichen Identifikationsdomänen eine unterschiedliche Anzahl an Nukleotiden verglichen mit einer anderen räumlichen Identifikationsdomäne innerhalb desselben räumlichen Barcodes umfasst.

9. Verfahren nach einem der vorhergehenden Ansprüche, wobei das Verfahren ferner umfasst:
i) nach Schritt (e) und vor Schritt (f) Wiederholen der Schritte (c) und (d), um die räumliche Barcodedomäne an jeder Stelle der Gewebeprobe zu verlängern, so dass die räumliche Barcodedomäne, am 3'-Ende, eine Begrenzungsdomäne umfasst, vorzugsweise wobei
(a) die Begrenzungsdomäne mindestens etwa 4 bis mindestens etwa 6 Nukleotide lang ist; und/oder
(b) die Sequenz der Begrenzungsdomäne für jeden räumlichen Barcode in der Probe gleich ist;
und/oder
ii) nach Schritt (e) und vor Schritt (f) Verlängern der räumlichen Barcodedomäne an jeder Stelle der Gewebeprobe, so dass die räumliche Barcodedomäne eine PolyT-Domäne umfasst.

10. Verfahren nach einem der vorhergehenden Ansprüche, wobei die PolyT-Domäne mindestens etwa 14 Nukleotide umfasst.

11. Verfahren nach einem der vorhergehenden Ansprüche, wobei das Beleuchten in Schritt (c):
i) bereitgestellt wird durch eine Lichtquelle, ausgewählt aus der Gruppe bestehend aus einer Bogenlampe, einem Laser, einer fokussierten UV-Lichtquelle und einer Licht-emittierenden Diode, vorzugsweise wobei der Laser ein Infrarotlaser ist; oder
ii) bereitgestellt wird durch ein Mikroskop mit Zwei-Photonen-Anregung.

12. Verfahren nach einem der vorhergehenden Ansprüche, wobei die erste Stelle der Gewebeprobe und die zweite Stelle der Gewebeprobe nicht mehr als etwa 500 nm in der x- und/oder y-Richtung und nicht mehr als etwa 1500 nm in der z-Richtung voneinander entfernt sind, vorzugsweise wobei die erste Stelle der Gewebeprobe und die zumindest zweite Stelle der Gewebeprobe
(a) subzellulär sind;
(b) jeweils nicht mehr als eine Zelle umfassen; oder
(b) jeweils nicht mehr als zehn Zellen umfassen.

13. Verfahren nach einem der vorhergehenden Ansprüche, wobei das Verfahren ferner, vor dem Schritt (a), das Unterziehen der Gewebeprobe unter eine ddTTP (Didesoxthymidintriphosphat)-Terminierung umfasst, vorzugsweise wobei das Unterziehen der Gewebeprobe unter die ddTTP-Terminierung das Inkontaktbringen der Gewebeprobe mit ddTTP und TdT umfasst.

14. Verfahren nach einem der vorhergehenden Ansprüche, wobei das Verfahren ferner, nach Schritt (f) und vor Schritt (g), das Amplifizieren der gesammelten Sonden umfasst, vorzugsweise wobei das Amplifizieren der gesammelten Sonden die Verwendung eines ersten Amplifikationsprimer und eines zweiten Amplifikationsprimers umfasst, wobei
der erste Amplifikationsprimer eine erste Durchflusszellen-Adaptersequenz, eine erste NGS-Indexsequenz und eine erste Sequenzierungsprimer-Bindungsstelle umfasst, und
der zweite Amplifikationsprimer eine zweite Durchflusszellen-Adaptersequenz, eine zweite NGS-Indexsequenz und eine zweite Sequenzierungsprimer-Bindungsstelle umfasst.

15. Verfahren nach einem der vorhergehenden Ansprüche, wobei mindestens einer von dem ersten und dem zweiten Amplifikationsprimer eine Nukleinsäuresequenz umfasst, die komplementär zu der Begrenzungssequenz und/oder der PolyT-Domäne ist.

## Revendications

1. Procédé de production d'un profil résolu spatialement de l'abondance d'au moins deux analytes cibles à un premier et au moins un deuxième emplacement d'un échantillon de tissu comprenant :
a) la mise en contact de l'échantillon de tissu avec une solution comprenant au moins deux espèces de sonde, les sondes comprenant un domaine de liaison à la cible et un domaine d'identification de la cible,
chaque espèce de sonde comprenant un domaine de liaison à la cible unique qui se lie à l'un des au moins deux analytes cibles et un domaine d'identification de la cible unique spécifique de l'analyte cible, et un groupement 3'-OH libre ;
b) la ligature d'un nucléotide au groupement 3'-OH libre d'au moins une sonde liée par mise en contact de l'échantillon de tissu avec une première pluralité de complexes nucléotide-polymérase, et ainsi l'extension du domaine de code à barres de l'au moins une sonde liée et la formation d'un domaine de code à barres spatial,
au moins un complexe nucléotide-polymérase dans la première pluralité comprenant le nucléotide fonctionnellement lié à une polymérase par le biais d'un lieur photoclivable ;
c) l'éclairage d'au moins un emplacement de l'échantillon de tissu avec suffisamment de lumière pour cliver le lieur photoclivable des complexes nucléotide-polymérase, et ainsi la libération des polymérases et l'exposition d'un groupement 3'-OH libre sur le domaine de code à barres spatial à l'au moins un emplacement de l'échantillon de tissu ;
d) la ligature d'un nucléotide au groupement 3'-OH libre du domaine de code à barres spatial d'au moins une sonde liée à l'au moins un emplacement éclairé à l'étape (c) par mise en contact de l'échantillon de tissu avec une pluralité supplémentaire de complexes nucléotide-polymérase, et ainsi l'extension du domaine de code à barres spatial de l'au moins une sonde liée,
au moins un complexe nucléotide-polymérase dans la pluralité supplémentaire comprenant le nucléotide fonctionnellement lié à une polymérase par le biais d'un lieur photoclivable ;
e) la répétition des étapes (c) et (d) jusqu'à ce que le domaine de code à barres spatial d'au moins une sonde liée à un analyte cible au premier emplacement de l'échantillon de tissu ait été étendu de telle sorte que le domaine de code à barres spatial comprend une séquence d'acide nucléique unique spécifique du premier emplacement de l'échantillon de tissu, et
le domaine de code à barres spatial d'au moins une sonde liée à un analyte cible à l'au moins un deuxième emplacement de l'échantillon de tissu ait été étendu de telle sorte que le domaine de code à barres spatial comprend une séquence d'acide nucléique unique spécifique de l'au moins un deuxième emplacement de l'échantillon de tissu ;
f) la collecte des sondes liées à des analytes cibles dans l'échantillon de tissu ; et
g) la quantification par séquençage des sondes collectées à l'étape (f), et ainsi la détermination de l'abondance des au moins deux analytes cibles au premier et à l'au moins un deuxième emplacement de l'échantillon de tissu, et ainsi la production d'un profil résolu spatialement de l'abondance des au moins deux analytes cibles.

2. Procédé de production d'un profil résolu spatialement de l'abondance d'au moins deux analytes cibles à un premier et au moins un deuxième emplacement d'un échantillon de tissu comprenant :
a) la mise en contact de l'échantillon de tissu avec une solution comprenant au moins deux espèces de sonde, les sondes comprenant un domaine de liaison à la cible et un domaine d'identification de la cible,
chaque espèce de sonde comprenant un domaine de liaison à la cible unique qui se lie à l'un des au moins deux analytes cibles et un domaine d'identification de la cible unique spécifique de l'analyte cible, et un groupement 3'-OH libre ;
b) la ligature d'un nucléotide au groupement 3'-OH libre du domaine de code à barres d'au moins une sonde liée par mise en contact de l'échantillon de tissu avec une première pluralité de nucléotides terminateurs réversibles et une première pluralité de polymérases, et ainsi l'extension de l'au moins une sonde liée et la formation d'un domaine de code à barres spatial,
au moins un nucléotide terminateur réversible dans la première pluralité comprenant le nucléotide fonctionnellement lié à un groupement terminateur en 3' par le biais d'un lieur photoclivable ;
c) l'éclairage d'au moins un emplacement de l'échantillon de tissu avec suffisamment de lumière pour cliver le lieur photoclivable des nucléotides terminateurs réversibles, et ainsi la libération des groupements terminateurs en 3' réversibles et l'exposition d'un groupement 3'-OH libre sur le domaine de code à barres spatial à l'au moins un emplacement de l'échantillon de tissu ;
d) la ligature d'un nucléotide au groupement 3'-OH libre du domaine de code à barres spatial d'au moins une sonde liée à l'au moins un emplacement éclairé à l'étape (c) par mise en contact de l'échantillon de tissu avec une pluralité supplémentaire de nucléotides terminateurs réversibles et une pluralité supplémentaire de polymérases, et ainsi l'extension du domaine de code à barres spatial de l'au moins une sonde liée,
au moins un nucléotide terminateur réversible dans la pluralité supplémentaire comprenant le nucléotide fonctionnellement lié à un groupement terminateur en 3' réversible par le biais d'un lieur photoclivable ;
e) la répétition des étapes (c) et (d) jusqu'à ce que le domaine de code à barres spatial d'au moins une sonde liée à un analyte cible au premier emplacement de l'échantillon de tissu ait été étendu de telle sorte que le domaine de code à barres spatial comprend une séquence d'acide nucléique unique spécifique du premier emplacement de l'échantillon de tissu,
le domaine de code à barres spatial d'au moins une sonde liée à un analyte cible à l'au moins un deuxième emplacement de l'échantillon de tissu ait été étendu de telle sorte que le domaine de code à barres spatial comprend une séquence d'acide nucléique unique spécifique de l'au moins un deuxième emplacement de l'échantillon de tissu ;
f) la collecte des sondes liées à des analytes cibles dans l'échantillon de tissu ; et
g) la quantification par séquençage des sondes collectées à l'étape (f), et ainsi la détermination de l'abondance des au moins deux analytes cibles au premier et à l'au moins un deuxième emplacement de l'échantillon de tissu, et ainsi la production d'un profil résolu spatialement de l'abondance des au moins deux analytes cibles.

3. Procédé selon la revendication 1 ou la revendication 2, comprenant en outre la comparaison de l'abondance des au moins deux analytes cibles au premier emplacement de l'échantillon de tissu et des au moins deux analytes cibles à l'au moins un deuxième emplacement de l'échantillon de tissu.

4. Procédé selon l'une quelconque des revendications précédentes, dans lequel la polymérase est une désoxynucléotidyltransférase terminale ou une partie biologiquement active de celle-ci.

5. Procédé selon l'une quelconque des revendications 2 à 4, dans lequel
(a) l'au moins un nucléotide terminateur réversible comprend du 3'-O-(2-nitrobenzyl)-dATP, du 3'-O-(2-nitrobenzyl)-dCTP, du 3'-O-(2-nitrobenzyl)-dGTP ou du 3'-O-(2-nitrobenzyl)-dTTP ; ou
(b) le groupement terminateur en 3' comprend du 2-nitrobenzyle.

6. Procédé selon l'une quelconque des revendications précédentes, dans lequel les sondes comprennent en outre :
i) un identifiant moléculaire unique, l'identifiant moléculaire unique étant de préférence d'une longueur d'au moins environ 14 nucléotides ;
ii) un site de liaison d'amorce d'amplification, le site de liaison d'amorce d'amplification étant de préférence d'une longueur d'au moins environ 24 nucléotides ; et/ou
iii) une région constante, la région constante étant de préférence d'une longueur d'au moins environ 12 à au moins environ 20 nucléotides,
de préférence dans lequel les sondes comprennent, de 5' à 3', le domaine de liaison à la cible, suivi du site de liaison d'amorce d'amplification, suivi de l'identifiant moléculaire unique, suivi du domaine d'identification de la cible, suivi de la région constante.

7. Procédé selon l'une quelconque des revendications précédentes, dans lequel le domaine de code à barres spatial d'au moins une sonde liée à un analyte cible au premier emplacement de l'échantillon de tissu comprend une séquence d'identifiant spatial unique spécifique du premier emplacement de l'échantillon de tissu, et le domaine de code à barres spatial d'au moins une sonde liée à un analyte cible à l'au moins un deuxième emplacement de l'échantillon de tissu comprend une séquence d'identifiant spatial unique spécifique de l'au moins un deuxième emplacement de l'échantillon de tissu, de préférence dans lequel la séquence d'identifiant spatial comprend au moins environ 20 nucléotides.

8. Procédé selon l'une quelconque des revendications précédentes, dans lequel la séquence d'identifiant spatial comprend au moins quatre domaines d'identification spatiale, de préférence dans lequel :
(a) chacun des au moins quatre domaines d'identification spatiale comprend le même nombre de nucléotides, de préférence dans lequel chaque domaine d'identification spatiale comprend environ 1 à environ 4 nucléotides, de préférence dans lequel chaque domaine d'identification spatiale comprend environ 4 nucléotides ; ou
(b) au moins un des au moins quatre domaines d'identification spatiale comprend un nombre différent de nucléotides par rapport à un autre domaine d'identification spatiale à l'intérieur du même code à barres spatial.

9. Procédé selon l'une quelconque des revendications précédentes, le procédé comprenant en outre :
i) après l'étape (e) et avant l'étape (f), la répétition des étapes (c) et (d) pour étendre le domaine de code à barres spatial à chaque emplacement de l'échantillon de tissu de telle sorte que le domaine de code à barres spatial comprenne, à l'extrémité 3', un domaine de délimitation, de préférence dans lequel
(a) le domaine de délimitation est d'une longueur d'au moins environ 4 à au moins environ 6 nucléotides ; et/ou
(b) la séquence du domaine de délimitation est la même pour chaque code à barres spatial dans l'échantillon ; et/ou
ii) après l'étape (e) et avant l'étape (f), l'extension du domaine de code à barres spatial à chaque emplacement de l'échantillon de tissu de telle sorte que le domaine de code à barres spatial comprenne un domaine polyT.

10. Procédé selon l'une quelconque des revendications précédentes, dans lequel le domaine polyT comprend au moins environ 14 nucléotides.

11. Procédé selon l'une quelconque des revendications précédentes, dans lequel l'éclairage à l'étape (c) est :
i) assuré par une source de lumière choisie dans le groupe constitué par une lampe à arc, un laser, une source de rayonnement UV focalisé et une diode électroluminescente, de préférence dans lequel le laser est un laser infrarouge ; ou
ii) assuré par un microscope à excitation biphotonique.

12. Procédé selon l'une quelconque des revendications précédentes, dans lequel le premier emplacement de l'échantillon de tissu et le deuxième emplacement de l'échantillon de tissu ne sont pas à plus d'environ 500 nm dans la direction x et/ou y et pas à plus d'environ 1500 nm dans la direction z, de préférence dans lequel le premier emplacement de l'échantillon de tissu et l'au moins un deuxième emplacement de l'échantillon de tissu
(a) sont intracellulaires ;
(b) ne comprennent chacun pas plus d'une cellule ; ou
(c) ne comprennent chacun pas plus de dix cellules.

13. Procédé selon l'une quelconque des revendications précédentes, le procédé comprenant en outre, avant l'étape (a), la soumission de l'échantillon de tissu à une terminaison ddTTP (didésoxythymidine-triphosphate), de préférence dans lequel la soumission de l'échantillon de tissu à une terminaison ddTTP comprend la mise en contact de l'échantillon de tissu avec du ddTTP et de la TdT.

14. Procédé selon l'une quelconque des revendications précédentes, le procédé comprenant en outre, après l'étape (f) et avant l'étape (g), l'amplification des sondes collectées, de préférence dans lequel l'amplification des sondes collectées comprend l'utilisation d'une première amorce d'amplification et d'une deuxième amorce d'amplification, dans lequel
la première amorce d'amplification comprend une première séquence d'adaptation à une cellule d'écoulement, une première séquence d'indexation NGS et un premier site de liaison d'amorce de séquençage, et
la deuxième amorce d'amplification comprend une deuxième séquence d'adaptation à une cellule d'écoulement, une deuxième séquence d'indexation NGS et un deuxième site de liaison d'amorce de séquençage.

15. Procédé selon l'une quelconque des revendications précédentes, dans lequel au moins une des première et deuxième amorces d'amplification comprend une séquence d'acide nucléique qui est complémentaire de la séquence de délimitation et/ou du domaine polyT.
